(19) Europäisches Patentamt
European Patent Office
Office européen des brevets

(11) EP 3 733 206 A1

(12) **EUROPEAN PATENT APPLICATION**
published in accordance with Art. 153(4) EPC

(43) Date of publication:
**04.11.2020 Bulletin 2020/45**

(21) Application number: **18895190.9**

(22) Date of filing: **26.12.2018**

(51) Int Cl.:
***A61K 45/06*** (2006.01) ***A61K 31/337*** (2006.01)
***A61K 31/357*** (2006.01) ***A61K 31/475*** (2006.01)
***A61K 31/517*** (2006.01) ***A61P 35/00*** (2006.01)
***A61P 43/00*** (2006.01)

(86) International application number:
**PCT/JP2018/047937**

(87) International publication number:
**WO 2019/131794 (04.07.2019 Gazette 2019/27)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **27.12.2017 JP 2017252178**

(71) Applicants:
- **Japanese Foundation For Cancer Research Tokyo 135-8550 (JP)**
- **RIKEN Wako-shi Saitama 351-0198 (JP)**
- **Kabushiki Kaisha Yakult Honsha Tokyo 105-8660 (JP)**

(72) Inventors:
- **SEIMIYA Hiroyuki Tokyo 135-8550 (JP)**
- **YOSHIDA Minoru Wako-shi, Saitama 351-0198 (JP)**
- **YASHIRODA Yoko Wako-shi, Saitama 351-0198 (JP)**
- **MURAMATSU Yukiko Tokyo 135-8550 (JP)**
- **SHIRAI, Fumiyuki Wako-shi, Saitama 351-0198 (JP)**
- **WASHIZUKA, Kenichi Wako-shi, Saitama 351-0198 (JP)**

(74) Representative: **Blodig, Wolfgang**
**Wächtershäuser & Hartz**
**Patentanwaltspartnerschaft mbB**
**Weinstrasse 8**
**80333 München (DE)**

(54) **ANTICANCER AGENT**

(57) To provide an anticancer agent for preventing and/or treating cancer, comprising a tankyrase inhibitor containing a tankyrase inhibitory compound and a microtubule inhibitor containing a microtubule inhibitory compound as active ingredients.

[FIG. 1A]

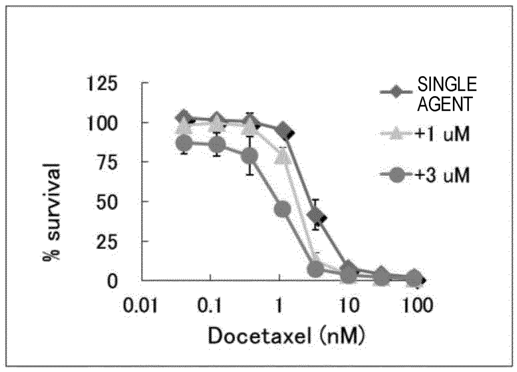


EP 3 733 206 A1

[FIG. 1B]

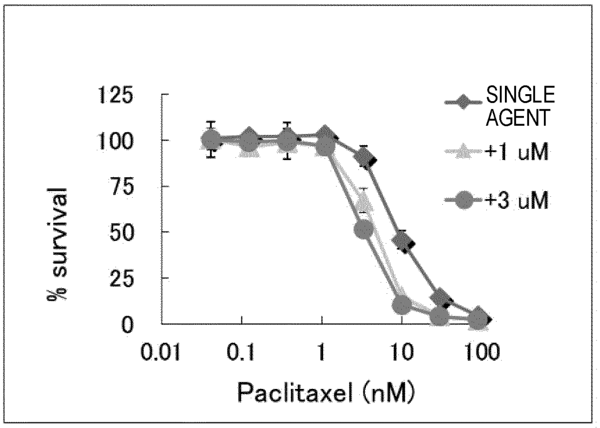


[FIG. 1C]

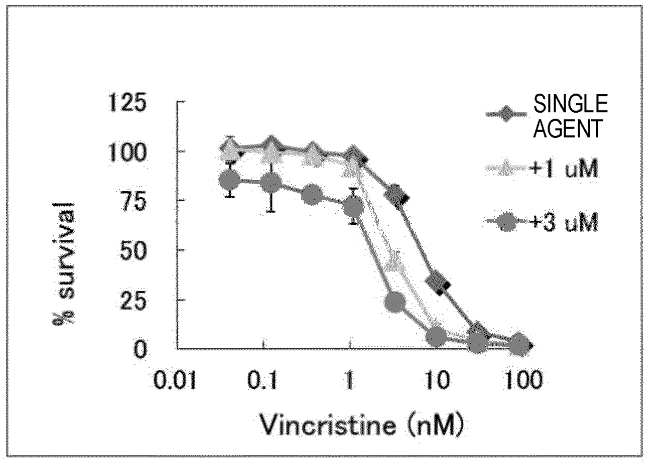


[FIG. 1D]

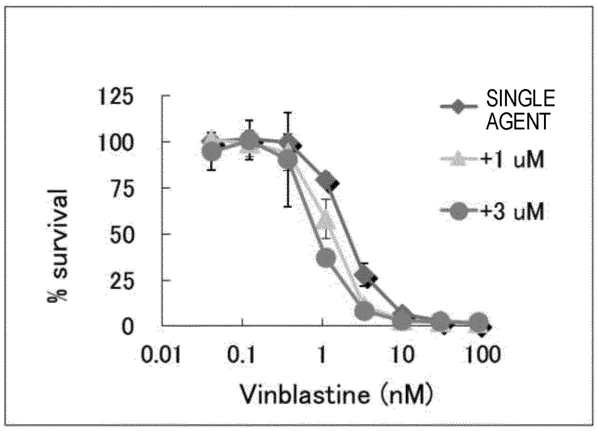

## Description

Technical Field

The present invention relates to an anticancer agent and a kit for preventing and/or treating a cancer.

Background Art

Poly(ADP-ribosyl)ation is a biochemical reaction which is a chain addition of ADP-ribose to glutamic acid or aspartic acid residues of a protein, with nicotinamide adenine dinucleotide as a substrate. A produced poly(ADP-ribose) chain is composed of about 200 ADP-riboses at longest. A poly(ADP-ribose) polymerase (PARP) family is known as an enzyme which catalyzes the poly(ADP-ribosyl)ation reaction.

Of the PARP family, PARP-5a and PARP-5b are called tankyrase-1 and tankyrase-2, respectively. Normally, in many cases, both enzymes are simply called tankyrase collectively. The tankyrase is composed of an ankyrin region which recognizes a protein which is to be poly(ADP-ribosyl)ated; a sterile alpha motif (SAM) region which is involved in self-multimerization; and a PARP catalytic domain which controls the poly(ADP-ribosyl)ation reaction.

The tankyrase binds to various proteins through the ankyrin region in the molecule to poly(ADP-ribosyl)ate these proteins. Examples of tankyrase-bound proteins include TRF1, NuMA, Plk1, Miki, Axin, TNKS1BP1, IRAP, Mcl-1 and 3BP2. The tankyrase poly(ADP-ribosyl)ates these proteins to regulate the physiological functions of the proteins. Hence, inhibition of tankyrase is considered to be effective for the control of cell proliferation, cell differentiation, tissue formation and the like which are the physiological functions of the proteins.

Examples of known tankyrase inhibitory compounds having tankyrase inhibitory activity include the compound XAV939 described in Non Patent Literature 1 (Huang SM. et al., Nature, Vol. 461, pp. 614-620, 2009), the compounds described in Patent Literature 1 (WO 2013/117288) and Patent Literature 2 (WO 2013/182580), NVP-TNKS656 described in Non Patent Literature 2 (Michael D. Shultz et al., Journal of Medicinal Chemistry, 56, pp. 6495-6511, 2013), and G007-LK described in Non Patent Literature 3 (Andrew Voronkov. et al., Journal of Medicinal Chemistry, 56, pp. 3012-3023, 2013) and Patent Literature 3 (WO 2012/076898).

A microtubule is a protein forming a cytoskeleton, and is involved in formation of a spindle in the phase of cell division period, formation and maintenance of cellular morphology, arrangement of intracellular organelles and transport of substances to the organs, axonal transport in nerve cells, and the like. The microtubule is composed of tubulin dimers each composed of an $\alpha$-subunit and a $\beta$-subunit. A process in which the dimers aggregate to form a microtubule is referred to as polymerization, and a process in which a microtubule reverts to a tubulin is referred to as depolymerization.

As microtubule inhibitors which inhibit microtubules, microtubule depolymerization inhibitors for promoting the polymerization to stabilize and excessively form microtubules, and microtubule polymerization inhibitors for inhibiting the polymerization are known, and these microtubule inhibitors each suppress cell proliferation by disrupting the state of dynamic equilibrium of microtubule polymerization to arrest the cell cycle in the M phase. As such microtubule inhibitors, paclitaxel, vinblastine, vincristine, vindesine, vinorelbine, docetaxel, cabazitaxel, eribulin and the like are known. These microtubule inhibitors are commercially available as agents having an antitumor effect on leukemia, malignant lymphoma and malignant tumors.

Such tankyrase inhibitors and microtubule inhibitors are considered to have an effect against fibrosarcoma, ovary cancer, glioblastoma, pancreatic cancer, breast cancer, astrocytoma, lung cancer, gastric cancer, hepatocyte cancer, multiple myeloma, colorectal intestine cancer, bladder cancer, leukemia, infections with a Herpes simplex virus, an Epstein-Barr virus and the like, fibroses such as pulmonary fibrosis, cherubism, multiple sclerosis, amyotrophic lateral sclerosis, skin and cartilage injuries, metabolic diseases and the like, and a suppressive effect on cancer metastasis. Development of a new pharmaceutical product (anticancer agent) for preventing and/or treating the above-mentioned diseases, particularly proliferative diseases such as a cancer, is desired.

For example, Non Patent Literature 4 (Oriol Arques et al., Clinical Cancer Research, 22 (3), pp. 644-656, 2016), Non Patent Literature 5 (Kevin S. Quackenbush et al., Oncotarget, Vol. 7, No. 19, pp. 28273-28285, 2016) and Non Patent Literature 6 (Hannah A. Scarborough et al., Clinical Cancer Research, 23 (6), pp. 1531-1541, 2017) disclose use of a tankyrase inhibitory compound along with an inhibitor against PI3 kinase or AKT which is a survival signal kinase (Non Patent Literature 4), irinotecan which is a topoisomerase inhibitor (Non Patent Literature 5) and EGFR tyrosine kinase inhibitor (Non Patent Literature 6).

Citation List

Patent Literature

Patent Literature 1: WO 2013/117288
Patent Literature 2: WO 2013/182580
Patent Literature 3: WO 2012/076898

Non Patent Literature

Non Patent Literature 1: Huang SM. et al., Nature, Vol. 461, pp. 614-620, 2009
Non Patent Literature 2: Michael D. Shultz et al., Journal of Medicinal Chemistry, 56, pp. 6495-6511, 2013
Non Patent Literature 3: Andrew Voronkov. et al., Journal of Medicinal Chemistry, 56, pp. 3012-3023, 2013
Non Patent Literature 4: Oriol Arques et al., Clinical Cancer Research, 22 (3), pp. 644-656, 2016
Non Patent Literature 5: Kevin S. Quackenbush et al., Oncotarget, Vol. 7, No. 19, pp. 28273-28285, 2016
Non Patent Literature 6: Hannah A. Scarborough et al., Clinical Cancer Research, 23 (6), pp. 1531-1541, 2017

Problem to Solve

The present invention was made in view of the above problems of the conventional technology. The present invention aims to provide an anticancer agent and a kit, useful for treating and/or preventing proliferative diseases such as a cancer.

Solution

The present inventors earnestly studied to solve the above problems and found that use of a combination of a tankyrase inhibitory compound and a microtubule inhibitory compound is useful for treating and/or preventing proliferative diseases such as a cancer, thereby completed the present invention.
That is, the present invention encompasses the following:

[1] An anticancer agent for preventing and/or treating a cancer, comprising a combination of a tankyrase inhibitor containing a tankyrase inhibitory compound as an active ingredient and a microtubule inhibitor containing a microtubule inhibitory compound as an active ingredient.
[2] An anticancer agent for preventing and/or treating a cancer, which is used to be administered in combination with a microtubule inhibitor containing a microtubule inhibitory compound as an active ingredient and which contains a tankyrase inhibitory compound as an active ingredient.
[3] An anticancer agent for preventing and/or treating a cancer, which is used to be administered in combination with a tankyrase inhibitor containing a tankyrase inhibitory compound as an active ingredient and which contains a microtubule inhibitory compound as an active ingredient.
[4] An anticancer agent for preventing and/or treating a cancer, comprising a tankyrase inhibitory compound and a microtubule inhibitory compound as active ingredients.
[5] The anticancer agent according to any one of claims 1 to 4, where the tankyrase inhibitory compound acts on at least one of a nicotinamide binding pocket of tankyrase and an adenosine binding pocket of tankyrase.
[6] The anticancer agent according to any one of claims 1 to 5, where the tankyrase inhibitory compound is at least one compound selected from the group consisting of:

a compound of the formula (1):

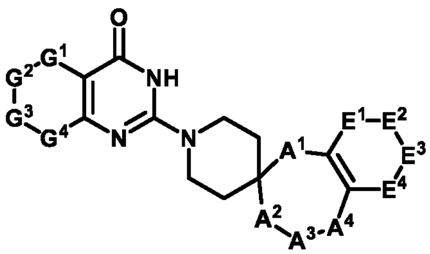
(1)

where $A^1$, $A^2$, $A^3$ and $A^4$ form a structure in which $A^1$ and $A^2$ each represent a single bond, one of $A^1$ and $A^2$ represents a single bond and the other represents $CH_2$, or $A^1$ represents a single bond and $A^4$ represents

$CH_2$,

with the proviso that one of $A^3$ and $A^4$ is $CH_2$ or CO and the other is O or $NR^1$ when $A^1$ and $A^2$ each represent a single bond or $A^1$ represents $CH_2$ and $A^2$ represents a single bond, one of $A^3$ and $A^4$ is $NR^1$ and the other is $CH_2$ or CO when $A^1$ represents a single bond and $A^2$ represents $CH_2$, and one of $A^2$ and $A^3$ is $NR^1$ and the other is $CH_2$ or CO when $A^1$ represents a single bond and $A^4$ represents $CH_2$,

where $R^1$ represents a hydrogen atom, an optionally substituted $C_{1-6}$ alkyl group, an optionally substituted heteroaryl group, an optionally substituted $C_{3-8}$ cycloalkyl $C_{1-3}$ alkyl group, an optionally substituted aryl $C_{1-3}$ alkyl group, an optionally substituted heteroaryl $C_{1-3}$ alkyl group, an optionally substituted three- to seven-membered ring heterocycloalkyl $C_{1-3}$ alkyl group, a group represented by the formula: $-(CH_2)_m-C(=O)-L$, or a group represented by the formula: $-S(=O)_2-R^{13}$,

m is 0, 1, 2 or 3, L is $R^{11}$ when m is 0, and L is $R^{12}$ when m is 1, 2 or 3,

$R^{11}$ is a hydrogen atom, an optionally substituted $C_{1-6}$ alkyl group, $OR^{51}$, a group represented by the formula: $-C(=O)-OR^{52}$, or a group represented by the formula: $-N(R^{53a})-R^{53b}$,

$R^{51}$ is an optionally substituted aryl $C_{1-3}$ alkyl group,

$R^{52}$ is a hydrogen atom or an optionally substituted $C_{1-6}$ alkyl group,

$R^{53a}$ and $R^{53b}$ are each independently a hydrogen atom or an optionally substituted $C_{1-6}$ alkyl group, or $R^{53a}$ and $R^{53b}$ are linked together to form a three- to seven-membered ring heterocycloalkyl group optionally containing at least one atom or group selected from the group consisting of an oxygen atom, a sulfur atom and NR81,

$R^{81}$ is a hydrogen atom or an optionally substituted $C_{1-6}$ alkyl group,

$R^{12}$ is an optionally substituted aryl group, $OR^{54}$, or a group represented by the formula: $-N(R^{55a})-R^{55b}$,

$R^{54}$ is a hydrogen atom, an optionally substituted $C_{1-6}$ alkyl group, an optionally substituted aryl $C_{1-3}$ alkyl group, or an optionally substituted heteroaryl $C_{1-3}$ alkyl group,

$R^{55a}$ and $R^{55b}$ are each independently a hydrogen atom, an optionally substituted $C_{1-6}$ alkyl group, an optionally substituted aryl $C_{1-3}$ alkyl group, an optionally substituted heteroaryl $C_{1-3}$ alkyl group, or a group represented by the formula: $-(C=O)-R^{82}$, or $R^{55a}$ and $R^{55b}$ are linked together to form a three- to seven-membered ring heterocycloalkyl group optionally containing at least one atom or group selected from the group consisting of an oxygen atom, a sulfur atom and $NR^{83}$, or form an optionally substituted 6,8-dihydro-5H-imidazolo[1,2-a]pyrazin-7-yl group,

$R^{82}$ is a hydrogen atom, an optionally substituted $C_{1-6}$ alkyl group, or an optionally substituted aryl $C_{1-3}$ alkyl group,

$R^{83}$ is a hydrogen atom or an optionally substituted $C_{1-6}$ alkyl group, and

$R^{13}$ is an optionally substituted $C_{1-6}$ alkyl group;

the structure formed by $E^1$, $E^2$, $E^3$ and $E^4$ is a group represented by the formula: $-E^1-E^2-E^3-E^4-$ (where the bonds between $E^1$, $E^2$, $E^3$ and $E^4$ are single bonds or double bonds) in which $E^1$ is N or $CR^2$, $E^2$ is N or $CR^3$, $E^3$ is N or $CR^4$ and $E^4$ is N or $CR^5$, a group represented by the formula: $-E^2-E^3=E^4-$ in which $E^2$ is O or S and each of $E^3$ and $E^4$ is CH with $E^1$ representing a single bond, or a group represented by the formula: $-E^2=E^3-E^4-$ in which each of $E^2$ and $E^3$ is CH and $E^4$ is O or S with $E^1$ representing a single bond,

where $R^2$, $R^3$, $R^4$ and $R^5$ are each independently a hydrogen atom, a fluorine atom, a chlorine atom, a bromine atom, an iodine atom, a cyano group, an optionally substituted $C_{1-6}$ alkyl group, an optionally substituted aryl group, an optionally substituted heteroaryl group, an optionally substituted three- to seven-membered ring heterocycloalkyl group, or a group represented by the formula: $-Q-(CH_2)_n-R^{14}$,

n is 0, 1, 2 or 3,

Q is a group represented by the formula: -CH=CH-, O, CO, a group represented by the formula: -C(=O)-O-, a group represented by the formula: $-C(=O)-N(R^{56})-$, $NR^{56}$, a group represented by the formula: $-N(R^{56})-C(=O)-$, or a group represented by the formula: $-N(R^{56})-C(=O)-O-$,

$R^{56}$ is a hydrogen atom, an optionally substituted $C_{1-3}$ alkyl group, or a group represented by the formula: $-C(=O)-R^{84}$,

$R^{84}$ is a hydrogen atom, an optionally substituted $C_{1-6}$ alkyl group, or an optionally substituted aryl group, an optionally substituted $C_{1-6}$ alkyloxy group, or an optionally substituted aryloxy group, and

$R^{14}$ is a hydrogen atom, an optionally substituted $C_{1-6}$ alkyl group, an optionally substituted aryl group, an optionally substituted heteroaryl group, an optionally substituted $C_{3-8}$ cycloalkyl group, or an optionally substituted three- to seven-membered ring heterocycloalkyl group; and

the structure formed by $G^1$, $G^2$, $G^3$ and $G^4$ is a group represented by the formula: $-G^1-G^2-G^3-G^4-$ (where the bonds between $G^1$, $G^2$, $G^3$ and $G^4$ are single bonds or double bonds) which is represented by the formula: $-CH=CH-CH=CR^6-$ (with the exception of cases where $A^1$ and $A^2$ each represent a single bond, with $A^3$ being O and $A^4$ being CO), the formula: -CH=CH-CH=N- (with the exception of cases where $A^1$ and $A^2$ each represent a single bond, with $A^3$ being O and $A^4$ being CO), the formula: $-CH_2-CH_2-CH_2-CH_2-$,

the formula: $-CO-CH_2-CH_2-N(R^7)-$, the formula: $-CH_2-CF_2-CH_2-CH_2-$, the formula: $-CH_2-O-CH_2-CH_2-$, the formula: $-CH_2-S-CH_2-CH_2-$, the formula: $-CH_2-CH_2-N(R^7)-CH_2-$, the formula: $-CH_2-CH_2-CH_2-O-$, the formula: $-CH_2-CH_2-CH_2-N(R^7)-$or the formula: $-O-CH_2-CH_2-N(R^7)-$, or a group represented by the formula: $-G^2-G^3-G^4-$ (where the bonds between $G^2$, $G^3$ and $G^4$ are single bonds or double bonds) which is represented by the formula: $-CH=CH-N(R^7)-$, the formula: $-CH_2-CH_2-N(R^7)-$, the formula: $-N=CH-N(R^7)-$ or the formula: $-N(R^7)-CH=N-$, with $G^1$ representing a single bond,
where $R^6$ is a hydrogen atom, a fluorine atom, a chlorine atom, a bromine atom, an iodine atom, an optionally substituted $C_{1-6}$ alkyl group, or an optionally substituted $C_{1-6}$ alkyloxy group,
$R^7$ is a hydrogen atom, an optionally substituted $C_{1-6}$ alkyl group, an optionally substituted $C_{3-8}$ cycloalkyl group, an optionally substituted $C_{3-8}$ cycloalkyl $C_{1-3}$ alkyl group, an optionally substituted three- to seven-membered ring heterocycloalkyl group, an optionally substituted three- to seven-membered ring heterocycloalkyl $C_{1-3}$ alkyl group, a group represented by the formula:$-C(=O)-R^{15}$, or a group represented by the formula $-(CH_2)_p-C(=O)-OR^{16}$,
p is 0, 1, 2 or 3,
$R^{15}$ is a hydrogen atom, an optionally substituted $C_{1-6}$ alkyl group or $OR^{57}$,
$R^{57}$ is an optionally substituted $C_{1-6}$ alkyl group, or an optionally substituted aryl $C_{1-3}$ alkyl group, and
$R^{16}$ is a hydrogen atom or an optionally substituted $C_{1-6}$ alkyl group;

a compound of the formula (11):

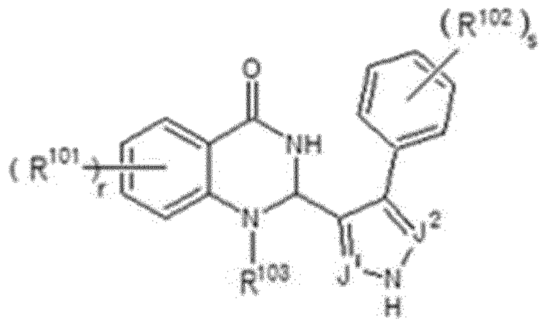

(11)

where $J_1$ and $J_2$ each represent CH or N, with the proviso that both $J_1$ and $J_2$ do not represent CH;
r represents 0 to 4;
each $R^{101}$ is the same or different when r is 2 or more, and represents a halogen atom, a $C_{1-6}$ alkyl group optionally substituted with a halogen atom, $OR^{111}$, or a group represented by the formula: $-N(R^{112a})-R^{112b}$, where $R^{111}$, $R^{112a}$ and $R^{112b}$ are each independently a hydrogen atom or a $C_{1-6}$ alkyl group, and
s represents 0 to 5;
each $R^{102}$ is the same or different when s is 2 or more, and represents a halogen atom, a $C_{1-6}$ alkyl group, $OR^{113}$, a group represented by the formula: $-N(R^{114a})-R^{114b}$, a group represented by the formula: $-NH-C(=O)-R^{115}$, a group represented by the formula: $-C(=O)-R^{116}$, an optionally substituted aryl group, an optionally substituted heteroaryl group, a nitro group, or a cyano group,
where $R^{113}$ is a hydrogen atom, an optionally substituted alkyl group, an optionally substituted aryl group, an optionally substituted arylalkyl group, or an optionally substituted heteroaryl group,
$R^{114a}$ and $R^{114b}$ are each independently a hydrogen atom or a $C_{1-6}$ alkyl group,
$R^{115}$ is an optionally substituted alkyl group, an optionally substituted aryl group, an optionally substituted heteroaryl group, or a group represented by the formula: $-NH-R^{121}$,
$R^{116}$ is an optionally substituted alkyl group, an optionally substituted aryl group, an optionally substituted heteroaryl group, $OR^{122}$, or a group represented by the formula: $-N(R^{123a})-R^{123b}$,
$R^{121}$ is an optionally substituted alkyl group, an optionally substituted aryl group, or an optionally substituted heteroaryl group,
$R^{122}$ is a hydrogen atom, an optionally substituted alkyl group, an optionally substituted aryl group, or an optionally substituted heteroaryl group, and
$R^{123a}$ and $R^{123b}$ are each independently a hydrogen atom, an optionally substituted alkyl group, an optionally substituted aryl group, or an optionally substituted heteroaryl group, or $R^{123a}$ and $R^{123b}$ are linked together to form a cyclic amine;
$R^{103}$ represents a hydrogen atom, a $C_{1-6}$ alkyl group, a $C_{3-6}$ cycloalkyl group, or a $C_{3-6}$ cycloalkyl $C_{1-6}$ alkyl

group; and
$R^{101}$ and $R^{103}$ are optionally linked together to form a five- to seven-membered ring hetero ring when $R^{101}$ is present at the 8-position; and

a pharmacologically acceptable salt thereof.

[7] The anticancer agent according to any one of claims 1 to 6, where the microtubule inhibitory compound is at least one compound selected from the group consisting of paclitaxel, vinblastine, vincristine, vindesine, vinorelbine, docetaxel, cabazitaxel, eribulin and a pharmacologically acceptable salt thereof.
[8] The anticancer agent according to any one of claims 1 to 7, where the cancer is colorectal cancer.
[9] A kit for preventing and/or treating a cancer, comprising a tankyrase inhibitor containing a tankyrase inhibitory compound as an active ingredient and a microtubule inhibitor containing a microtubule inhibitory compound as an active ingredient.
[10] A method for preventing and/or treating a cancer, comprising administering to a patient a combination of a tankyrase inhibitor containing a tankyrase inhibitory compound as an active ingredient and a microtubule inhibitor containing a microtubule inhibitory compound as an active ingredient.
[11] An anticancer agent for preventing and/or treating a cancer, comprising a combination of: a tankyrase inhibitor containing at least one tankyrase inhibitory compound selected from the group consisting of a compound of the formula (1), a compound of the formula (11) and a pharmacologically acceptable salt thereof, as an active ingredient; and at least one agent selected from the group consisting of an alkylating agent, an antimetabolite, a plant alkaloid, a topoisomerase inhibitor, an anticancer antibiotic and a platinating agent.
[12] An anticancer agent for preventing and/or treating a cancer, comprising a tankyrase inhibitor containing at least one tankyrase inhibitory compound selected from the group consisting of a compound of the formula (1), a compound of the formula (11) and a pharmacologically acceptable salt thereof, as an active ingredient, for use in administration in combination with at least one agent selected from the group consisting of an alkylating agent, an antimetabolite, a plant alkaloid, a topoisomerase inhibitor, an anticancer antibiotic and a platinating agent.
[13] An anticancer agent for preventing and/or treating a cancer, comprising at least one agent selected from the group consisting of an alkylating agent, an antimetabolite, a plant alkaloid, a topoisomerase inhibitor, an anticancer antibiotic and a platinating agent as an active ingredient, for use in administration in combination with a tankyrase inhibitor containing at least one tankyrase inhibitory compound selected from the group consisting of a compound of the formula (1), a compound of the formula (11) and a pharmacologically acceptable salt thereof
[14] An anticancer agent for preventing and/or treating a cancer, comprising a combination of: at least one tankyrase inhibitory compound selected from the group consisting of a compound of the formula (1), a compound of the formula (11) and a pharmacologically acceptable salt thereof and at least one agent selected from the group consisting of an alkylating agent, an antimetabolite, a plant alkaloid, a topoisomerase inhibitor, an anticancer antibiotic and a platinating agent, as active ingredients.
[15] A kit for preventing and/or treating a cancer, comprising a combination of: a tankyrase inhibitor containing at least one tankyrase inhibitory compound selected from the group consisting of a compound of the formula (1), a compound of the formula (11) and a pharmacologically acceptable salt thereof, as an active ingredient; and at least one agent selected from the group consisting of an alkylating agent, an antimetabolite, a plant alkaloid, a topoisomerase inhibitor, an anticancer antibiotic and a platinating agent.
[16] A method for preventing and/or treating a cancer, comprising a combination of: a tankyrase inhibitor containing at least one tankyrase inhibitory compound selected from the group consisting of a compound of the formula (1), a compound of the formula (11) and a pharmacologically acceptable salt thereof, as an active ingredient; and at least one agent selected from the group consisting of an alkylating agent, an antimetabolite, a plant alkaloid, a topoisomerase inhibitor, an anticancer antibiotic and a platinating agent.

The reason why the above purpose can be achieved by the present invention is not necessarily clear, but the present inventors infer as follows. That is, in the present invention, a combination of a tankyrase inhibitory compound and a microtubule inhibitory compound is used, but regarding tankyrase, according to Ha et al. (Ha et al., Cell Death Differ., 19:321-332, 2012), tankyrase increases protein stability and poly(ADP-ribosyl)ation activity (PARP activity) by phosphorylation by the kinase Plk1 during mitosis. A mutant tankyrase in which four threonines and one serine are all replaced by alanine, which are assumed to be the phosphorylation site of Plk1, does not undergo phosphorylation by Plk1 and it decrease protein stability and PARP activity. Tankyrase is localized at telomeres at the ends of chromosomes throughout the cell cycle and at spindle poles during cell division stage, but this mutant tankyrase is not localized at either telomeres or spindle poles. On the other hand, it has been reported that at the cell division stage, tankyrases are essential for the resolution of telomeric cohesions between sister chromatids (Dynek and Smith, Science, 304:97-100, 2004). It has also been reported that localization of tankyrases at spindle poles and PARP activity are essential for

normal structure organization and functional expression of the mitotic spindle (Chang et al., Nat Cell Biol. 2005;7:1133-1139). Therefore, the present inventors presume that tankyrase, which is inhibited by the above tankyrase inhibitory compounds, contributes to the progression of cell division by increasing its own stability and PARP activity through selective phosphorylation during cell division stage and exerting intrinsic functions at telomeres and spindle poles.

By the way, the above microtubule inhibitory compounds prevent cell mitosis by inhibiting microtubule polymerization or depolymerization, thereby exerting a cytocidal effect. Because microtubule inhibition keeps the cell cycle in cell division stage, tankyrase phosphorylate, stabilization, and increased PARP activity are observed in cells treated with microtubule inhibitory compounds (Ha et al.,). Therefore, according to the combination of a tankyrase inhibitory compound and a microtubule inhibitory compound of the present invention, the microtubule inhibitory compound may retain the cell cycle in cell division stage to increase the functional requirement of tankyrase at the spindle poles and telomeres, while inhibiting the function of tankyrase by the tankyrase inhibitor compound, so that synergistic cytocidal effects can be achieved compared with the single drug treatment of each of them, and they may be effective in the treatment and/or prevention of proliferative diseases such as cancer.

The present invention can provide a novel anticancer agent and a kit useful for treating and/or preventing proliferative diseases such as a cancer.

[Brief Explanation of Drawings]

Fig. 1A is a graph showing the result by conducting MTT assay (docetaxel) on Example 1.
Fig. 1B is a graph showing the result by conducting MTT assay (paclitaxel) on Example 1.
Fig. 1C is a graph showing the result by conducting MTT assay (vincristine) on Example 1.
Fig. 1D is a graph showing the result by conducting MTT assay (vinblastine) on Example 1.
Fig. 2A is a graph showing the result by conducting MTT assay (docetaxel) on Example 2.
Fig. 2B is a graph showing the result by conducting MTT assay (paclitaxel) on Example 2.
Fig. 2C is a graph showing the result by conducting MTT assay (vincristine) on Example 2.
Fig. 2D is a graph showing the result by conducting MTT assay (vinblastine) on Example 2.
Fig. 3A is a graph showing the result by conducting MTT assay (docetaxel) on Example 3.
Fig. 3B is a graph showing the result by conducting MTT assay (paclitaxel) on Example 3.
Fig. 3C is a graph showing the result by conducting MTT assay (vincristine) on Example 3.
Fig. 3D is a graph showing the result by conducting MTT assay (vinblastine) on Example 3.
Fig. 4A is a graph showing the result by conducting MTT assay (docetaxel) on Example 4.
Fig. 4B is a graph showing the result by conducting MTT assay (paclitaxel) on Example 4.
Fig. 4C is a graph showing the result by conducting MTT assay (vincristine) on Example 4.
Fig. 4D is a graph showing the result by conducting MTT assay (vinblastine) on Example 4.
Fig. 5A is a graph showing the result by conducting MTT assay (docetaxel) on Example 5.
Fig. 5B is a graph showing the result by conducting MTT assay (paclitaxel) on Example 5.
Fig. 5C is a graph showing the result by conducting MTT assay (vincristine) on Example 5.
Fig. 5D is a graph showing the result by conducting MTT assay (vinblastine) on Example 5.
Fig. 6A is a graph showing the result by conducting MTT assay (docetaxel) on Example 6.
Fig. 6B is a graph showing the result by conducting MTT assay (paclitaxel) on Example 6.
Fig. 6C is a graph showing the result by conducting MTT assay (vincristine) on Example 6.
Fig. 6D is a graph showing the result by conducting MTT assay (vinblastine) on Example 6.
Fig. 7A is a graph showing the result by conducting MTT assay (docetaxel) on Example 7.
Fig. 7B is a graph showing the result by conducting MTT assay (paclitaxel) on Example 7.
Fig. 7C is a graph showing the result by conducting MTT assay (vincristine) on Example 7.
Fig. 7D is a graph showing the result by conducting MTT assay (vinblastine) on Example 7.
Fig. 8A is a graph showing the result by conducting MTT assay ((a), (b), (c), (d), (e), and (f)) on Example 8.
Fig. 8B is a graph showing the result by conducting MTT assay ((a), (b), (c), (d), (e), and (f)) on Example 8.
Fig. 8C is a graph showing the result by conducting MTT assay ((a), (b), (c), (d), (e), and (f)) on Example 8.
Fig. 9 is a graph showing the result by conducting MTT assay (irinotecan) on Example 9.
Fig. 10 is a graph showing the result by conducting MTT assay (irinotecan) on Example 10.
Fig. 11 is a graph showing the result by conducting MTT assay (irinotecan) on Example 11.
Fig. 12 is a graph showing the result by conducting MTT assay (irinotecan) on Example 12.
Fig. 13 is a graph showing the result by conducting MTT assay (irinotecan) on Example 13.
Fig. 14 is a graph showing the result by conducting MTT assay (irinotecan) on Example 14

Description of Embodiments

The present invention relates to an anticancer agent for treating and/or preventing cancer, containing a combination of a tankyrase inhibitor containing a tankyrase inhibitory compound as an active ingredient and a microtubule inhibitor containing a microtubule inhibitory compound as an active ingredient;

an anticancer agent for preventing and/or treating a cancer, containing a tankyrase inhibitory compound as an active ingredient, for use in administration in combination with a microtubule inhibitor containing a microtubule inhibitory compound;

an anticancer agent for preventing and/or treating a cancer, containing a microtubule inhibitory compound as an active ingredient, for use in administration in combination with a tankyrase inhibitor containing a tankyrase inhibitory compound;

an anticancer agent for preventing and/or treating a cancer, containing a tankyrase inhibitory compound and a microtubule inhibitory compound as active ingredients; and

a kit for preventing and/or treating a cancer, composed of a tankyrase inhibitor containing a tankyrase inhibitory compound and a microtubule inhibitor containing a microtubule inhibitory compound.

In the present invention, the "treatment and/or prevention of cancer" and the "anticancer" include not only direct treatment by cytotoxic effects or cell proliferation inhibitory effects on cancer cells, but also anticancer actions in a broad sense, such as suppression of invasion and metastasis of cancer cells and suppression of tumor angiogenesis, and the "anticancer agent" refers to an agent which exhibits at least one of these actions. Examples of the proliferative diseases include, but are not limited to, various solid tumors and blood tumors, for example malignant tumors such as fibrosarcoma, ovary cancer, glioblastoma, pancreatic cancer, breast cancer, astrocytoma, lung cancer, gastric cancer, liver cancer, colorectal cancer, bladder cancer and leukemia. Treatment and prevention of the proliferative diseases includes killing of tumor cells of the malignant tumors; and suppression, prevention and retardation of proliferation and metastasis of the tumor cells.

In the present invention, the term "pharmacologically acceptable" means being suitable for pharmacological use, and pharmacologically acceptable salts according to the present invention include, but are not particularly limited to, salts of alkali metals or alkali earth metals such as sodium, potassium and calcium; salts of hydrohalic acids such as hydrofluoric acid, hydrochloric acid, hydrobromic acid and hydroiodic acid; salts of inorganic acids such as sulfuric acid, nitric acid, phosphoric acid, perchloric acid and carbonic acid; salts of organic carboxylic acids such as formic acid, acetic acid, trifluoroacetic acid, trichloroacetic acid, hydroxyacetic acid, propionic acid, lactic acid, citric acid, tartaric acid, oxalic acid, benzoic acid, mandelic acid, butyric acid, fumaric acid, succinic acid, maleic acid and malic acid; salts of acidic amino acids such as aspartic acid and glutamic acid; salts of sulfonic acids such as methanesulfonic acid, ethanesulfonic acid, benzenesulfonic acid and toluenesulfonic acid; adducts such as acetone, methylethylketone, ether, ethyl acetate; and solvates such as hydrates and alcoholates.

In the present invention, the "hydrogen atoms" in the formulae include deuterium atoms (D) unless otherwise specified. The "halogen atoms" in the formulae include fluorine atoms, chlorine atoms, bromine atoms and iodine atoms.

In the formulae according to the present invention, the "alkyl group" refers to a linear or branched saturated hydrocarbon group having 1 to 8 carbon atoms, and the "$C_{1-3}$ alkyl group" and the "$C_{1-6}$ alkyl group" mean that the alkyl groups have 1 to 3 carbon atoms and 1 to 6 carbon atoms, respectively. Examples of the linear or branched saturated hydrocarbon group generally include, but are not particularly limited to, groups such as methyl, ethyl, n-propyl, isopropyl, n-butyl, sec-butyl, isobutyl, tert-butyl, n-pentyl, isopentyl, neopentyl and n-hexyl groups.

In the formulae according to the present invention, the "aryl group" refers to a six-membered ring monocyclic aromatic hydrocarbon group consisting of only carbon atoms, or a fused-ring aromatic hydrocarbon group in which two or more such six-membered ring monocyclic aromatic hydrocarbon groups are fused. Examples of the aryl group generally include, but are not particularly limited to, groups such as phenyl and naphthyl groups.

In the formulae according to the present invention, the "heteroaryl group" refers to a group derived from a five- or six-membered ring monocyclic aromatic heterocycle having 1 to 4 hetero atoms selected from the group consisting of a nitrogen atom, an oxygen atom and a sulfur atom; a group derived from a fused-ring aromatic heterocycle in which a five- or six-membered ring monocyclic aromatic heterocycle having 1 to 4 hetero atoms is fused with a six-membered ring monocyclic aromatic ring consisting of only carbon atoms; or a group derived from a fused-ring aromatic heterocycle in which a five- or six-membered ring monocyclic aromatic heterocycle having 1 to 4 hetero atoms is fused with a five- or six-membered ring monocyclic aromatic heterocycle having 1 to 4 hetero atoms. Examples of the heteroaryl group generally include, but are not particularly limited to, groups such as pyrrolyl, pyrazolyl, furyl, thienyl, oxazolyl, imidazolyl, isoxazolyl, thiazolyl, isothiazolyl, 1,2,3-oxadiazolyl, 1,2,4-oxadiazolyl, 1,2,3-thiadiazolyl, 1,2,4-thiadiazolyl, tetrazolyl, 1,2,4-triazolyl, 1,2,3-triazolyl, pyridyl, pyridazinyl, pyrazyl, pyrimidyl, benzothienyl, benzofuryl, indolyl, isoindolyl, benzoimidazolyl, benzopyrazolyl, benzothiazolyl, benzooxazolyl, benzotriazolyl, quinolyl, isoquinolyl, quinoxalinyl, quinazolinyl, phthalazinyl, imidazo[5,1-b]thiazolyl and 1H-pyrrolo[2,3-b]pyridinyl groups, each of which has a binding site at any possible position.

In the formulae according to the present invention, the "cycloalkyl group" refers to a cyclic saturated hydrocarbon

group (cyclic hydrocarbon group) having 3 to 8 carbon atoms, and the cyclic hydrocarbon group may be a monocyclic ring, or may form a fused ring, a crosslinked ring or a spiro ring. The "$C_{3-8}$ cycloalkyl group" means that the cycloalkyl group has 3 to 8 carbon atoms. Examples of the cyclic saturated hydrocarbon group generally include, but are not particularly limited to, groups such as cyclopropyl, cyclobutyl, cyclopentyl, cyclohexyl, cycloheptyl, cyclooctyl, bicyclo[3.1.0]hexyl, bicyclo[3.2.0]heptyl, bicyclo[4.1.0]heptyl, bicyclo[4.2.0]octyl, bicyclo[3.3.0]octyl, bicyclo[1.1.1]pentyl, bicyclo[2.1.1]hexyl, bicyclo[3.1.1]heptyl, bicyclo[2.2.1]heptyl, bicyclo[3.2.1]octyl, bicyclo[2.2.2]octyl, spiro[2.3]hexyl, spiro[2.4]heptyl, spiro[2.5]octyl, spiro[3.3]heptyl and spiro[3.4]octyl groups, each of which has a binding site at any possible position.

In the formulae according to the present invention, the "heterocycloalkyl group" refers to a 3 to 7-membered ring saturated heterocycle or unsaturated heterocycle other than an aromatic ring, which has 1 to 4 hetero atoms selected from the group consisting of a nitrogen atom, an oxygen atom and a sulfur atom, and the cyclic hydrocarbon group may be a monocyclic ring, or may form a crosslinked ring or a spiro ring, and is optionally substituted with any one or more substituents defined herein. Examples of the heterocycloalkyl group include, but are not particularly limited to, groups such as oxetanyl, tetrahydrofuryl, dihydrofuryl, dihydropyranyl, tetrahydropyranyl, 1,3-dioxanyl, 1,4-dioxanyl, aziridinyl, azetidinyl, pyrrolidinyl, piperidinyl, piperazinyl, 1,1-dioxide thiomorphorinyl, dioxopiperidinyl, diazepanyl, morphorinyl, 1,3-dioxolanyl, imidazolidinyl, imidazolinyl, pyrrolinyl, oxathiolanyl, dithiolanyl, 1,3- dithianyl, 1,4-dithianyl, oxathianyl, thiomorphorinyl, 3,6-diazabicylo[3.1.1]heptyl, 8-oxa-3-azabicyclo[3.2.1]octyl;, 3,8-diazabicyclo[3.2.1]octyl, 3,9-diazabicyclo[3.3.1]nonyl and 2-oxa-7-azaspiro[3.5]nonyl groups, each of which has a binding site at any possible position.

In the formulae according to the present invention, the "hetero ring" refers to an unsaturated heterocycle other than an aromatic ring, which has 1 to 4 hetero atoms selected from the group consisting of a nitrogen atom, an oxygen atom and a sulfur atom, and the hetero ring is preferably five- to seven-membered ring, more preferably five- or six-membered ring. The heterocycle may be a monocyclic ring, or may form a crosslinked ring or a spiro ring. Examples of the hetero ring include, but are not limited to, rings such as 2,3-dihydro-1H-pyrrole, 2,3-dihydroxazole, 2,3-dihydro-1H-imidazole, 1,2,3,4-tetrahydropyridine, 2,3,4,5-tetrahydro-1H-azepine, 3,4-dihydro-2H-1,4-oxazine, 1,2,3,4-tetrahydropyrazine, 3,4-dihydro-2H-1,4-thiazine and 4,5,6,7-tetrahydro-1,4-oxazepine rings, each of which has a binding site at any possible position.

In the formulae according to the present invention, the "arylalkyl group", the "heteroarylalkyl group", the "cycloalkylalkyl group" or the "heterocycloalkylalkyl group" refers to a group (a group represented by the formula: $-Ak^1-Ar^1$) in which an aryl group, a heteroaryl group, a cycloalkyl group or a heterocycloalkyl group (represented by the formula: $-Ar^1$) as defined herein is bonded at a binding site at any possible position to a binding site at any possible position in an alkyl group (represented by the formula: $-Ak^1$) as defined herein. For example, the "$C_{1-3}$ alkyl" in the "aryl $C_{1-3}$ alkyl group", the "heteroaryl $C_{1-3}$ alkyl group", the "$C_{3-8}$ cycloalkyl $C_{1-3}$ alkyl group" and the "heterocycloalkyl $C_{1-3}$ alkyl group" means that the alkyl group has 1 to 3 carbon atoms, and the "$C_{3-8}$ cycloalkyl" in the "$C_{3-8}$ cycloalkyl $C_{1-3}$ alkyl group" means that the cycloalkyl group has 3 to 8 carbon atoms.

In the formulae according to the present invention, the "$C_{1-3}$ alkyloxy group" or the "aryloxy group" refers to a group (a group represented by the formula: $-O-Ak^2$ or a group represented by the formula: $-O-Ar^2$) in which a $C_{1-3}$ alkyl group (represented by the formula: $-Ak^2$) or an aryl group (represented by the formula: $-Ar^2$) as defined herein is bonded to an oxygen atom.

In the formulae according to the present invention, the "di-$C_{1-3}$ alkylamino group" refers to a group (a group represented by the formula: $-N(-Ak^3)-Ak^4$) in which two $C_{1-3}$ alkyl groups (represented by the formulae: $-Ak^3$ and $- Ak^4$) which are the same or different as defined herein are bonded to a nitrogen atom, and the "arylamino group" refers to a group (a group represented by the formula: $- N-Ar^3$) in which an aryl group (represented by the formula: $Ar^3$) as defined herein is bonded to an amino group.

In the formulae according to the present invention, the "cyclic amine" refers to a nitrogen-containing heterocycle having 3 to 8 atoms, and the nitrogen-containing heterocycle may be a monocyclic ring, or may form a fused ring, a crosslinked ring or a spiro ring. Examples of the cyclic amine include, but are not particularly limited to, azetidine, pyrrolidine, piperidine, piperazine, morpholine, thiomorpholine, pyrazole, imidazole, triazole, azepane, azepine and azocane.

In the formulae according to the present invention, the term "optionally substituted" means that unless otherwise specified, any hydrogen atom bonded to a group described as being optionally substituted is substituted with a substituent (atom or group) selected from the group consisting of other atoms or groups, and the number of positions at which the hydrogen atom is substituted may be 1 or more. When the number of positions at which the hydrogen atom is substituted is 2 or more, the substituents are the same or different.

The compound according to the present invention may have one or more asymmetric carbon atoms, and optically active substances, enantiomers, any mixtures thereof, racemates and the like based on the one or more asymmetric carbon atoms are all within the scope of the present invention. The group having an unsaturated double bond can be present in a cis- or trans-form. Further, the compound according to the present invention expresses a form of one of possible isomers (rotational isomers, atropisomers and tautomers) in addition to the above-described isomers,

and these isomers may be present singly, or present as a mixture thereof. Herein, a compound which has any of the above-described isomers and isotopes and whose name is not particularly specified may be one of the isomers and isotopes, or a mixture or a racemate of two or more of the isomers and isotopes.

<Tankyrase Inhibitory compound>

In the present invention, the tankyrase inhibitory compound is a compound having tankyrase inhibitory activity. The tankyrase inhibitory compound is preferably a compound which inhibits tankyrase activity by binding to at least one of a nicotinamide binding pocket and an adenosine binding pocket being a site to which NAD+ as a substrate of tankyrase binds. As the tankyrase inhibitory compound according to the present invention, one that acts on at least one of the nicotinamide binding pocket and the adenosine binding pocket can be used. More specifically, the tankyrase inhibitory compound may be any of a nicotinamide-type compound which acts on the nicotinamide binding pocket; an adenosine-type compound which acts on the adenosine binding pocket; and a dual-type compound which acts on both the nicotinamide binding pocket and the adenosine binding pocket.

In the present invention, whether the tankyrase inhibitory compound is a nicotinamide-type compound or an adenosine-type compound can be determined by X-ray diffraction, more specifically a method in which a complex is crystallized from a mixed liquid of a tankyrase crystal prepared beforehand and the tankyrase inhibitory compound, and subjected to X-ray diffraction (soaking method), or a method in which a complex is crystallized from a mixed liquid having the tankyrase inhibitory compound coexisting with tankyrase in a state before the crystallization, and subjected to X-ray diffraction (co-crystallization). Whether the tankyrase inhibitory compound is a dual-type compound can be determined by preparing a crystal under any one of the above-described conditions, and performing X-ray diffraction.

Examples of the nicotinamide-type tankyrase inhibitory compound include compounds of the following formula (1) and pharmacologically acceptable salts thereof (hereinafter, sometimes referred to as "spiro compounds"), and compounds of the following formula (11) and pharmacologically acceptable salts thereof (hereinafter, sometimes referred to as "dihydroquinazolinone-based compounds"). One of these compounds may be used alone, or two or more thereof may be used in combination.

(Spiro Compound)

The nicotinamide-type tankyrase inhibitory compound according to the present invention is a compound of the following formula (1) or a pharmacologically acceptable salt thereof (spiro compound):

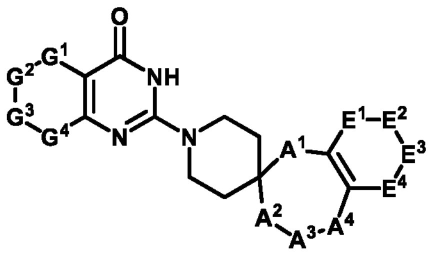

(1)

where $A^1$, $A^2$, $A^3$ and $A^4$ form a structure in which $A^1$ and $A^2$ each represent a single bond, one of $A^1$ and $A^2$ represents a single bond and the other represents $CH_2$, or $A^1$ represents a single bond and $A^4$ represents $CH_2$, with the proviso that one of $A^3$ and $A^4$ is $CH_2$ or CO and the other is O or $NR^1$ when $A^1$ and $A^2$ each represent a single bond or $A^1$ represents $CH_2$ and $A^2$ represents a single bond, one of $A^3$ and $A^4$ is $NR^1$ and the other is $CH_2$ or CO when $A^1$ represents a single bond and $A^2$ represents $CH_2$, and one of $A^2$ and $A^3$ is $NR^1$ and the other is $CH_2$ or CO when $A^1$ represents a single bond and $A^4$ represents $CH_2$,

where $R^1$ represents a hydrogen atom, an optionally substituted $C_{1-6}$ alkyl group, an optionally substituted heteroaryl, an optionally substituted $C_{3-8}$ cycloalkyl $C_{1-3}$ alkyl group, an optionally substituted aryl $C_{1-3}$ alkyl group, an optionally substituted heteroaryl $C_{1-3}$ alkyl group, an optionally substituted three- to seven-membered ring heterocycloalkyl $C_{1-3}$ alkyl group, a group represented by the formula: $-(CH_2)_m-C(=O)-L$, or a group represented by the formula: $-S(=O)_2-R^{13}$,

m is 0, 1, 2 or 3, L is $R^{11}$ when m is 0, and L is $R^{12}$ when m is 1, 2 or 3,

$R^{11}$ is a hydrogen atom, an optionally substituted $C_{1-6}$ alkyl group, $OR^{51}$, a group represented by the formula: $-C(=O)-OR^{52}$, or a group represented by the formula: $-N(R^{53a})-R^{53b}$,

$R^{51}$ is an optionally substituted aryl $C_{1-3}$ alkyl group,

$R^{52}$ is a hydrogen atom or an optionally substituted $C_{1-6}$ alkyl group,

$R^{53a}$ and $R^{53b}$ are each independently a hydrogen atom or an optionally substituted $C_{1-6}$ alkyl group, or $R^{53a}$ and $R^{53b}$ are linked together to form a three- to seven-membered ring heterocycloalkyl group optionally containing at least one atom or group selected from the group consisting of an oxygen atom, a sulfur atom and NR81,
$R^{81}$ is a hydrogen atom or an optionally substituted $C_{1-6}$ alkyl group,
$R^{12}$ is an optionally substituted aryl group, $OR^{54}$, or a group represented by the formula: $-N(R^{55a})-R^{55b}$,
$R^{54}$ is a hydrogen atom, an optionally substituted $C_{1-6}$ alkyl group, an optionally substituted aryl $C_{1-3}$ alkyl group, or an optionally substituted heteroaryl $C_{1-3}$ alkyl group,
$R^{55a}$ and $R^{55b}$ are each independently a hydrogen atom, an optionally substituted $C_{1-6}$ alkyl group, an optionally substituted aryl $C_{1-3}$ alkyl group, an optionally substituted heteroaryl $C_{1-3}$ alkyl group, or a group represented by the formula: $-(C=O)-R^{82}$, or $R^{55a}$ and $R^{55b}$ are linked together to form a three- to seven-membered ring heterocycloalkyl group optionally containing at least one atom or group selected from the group consisting of an oxygen atom, a sulfur atom and $NR^{83}$, or form an optionally substituted 6,8-dihydro-5H-imidazolo[1,2-a]pyrazin-7-yl group,
$R^{82}$ is a hydrogen atom, an optionally substituted $C_{1-6}$ alkyl group, or an optionally substituted aryl $C_{1-3}$ alkyl group,
$R^{83}$ is a hydrogen atom or an optionally substituted $C_{1-6}$ alkyl group, and
$R^{13}$ is an optionally substituted $C_{1-6}$ alkyl group;
the structure formed by $E^1$, $E^2$, $E^3$ and $E^4$ is a group represented by the formula: $-E^1-E^2-E^3-E^4-$ (where the bonds between $E^1$, $E^2$, $E^3$ and $E^4$ are single bonds or double bonds) in which $E^1$ is N or $CR^2$, $E^2$ is N or $CR^3$, $E^3$ is N or $CR^4$ and $E^4$ is N or $CR^5$, a group represented by the formula: $-E^2-E^3=E^4-$ in which $E^2$ is O or S and each of $E^3$ and $E^4$ is CH with $E^1$ representing a single bond, or a group represented by the formula: $-E^2=E^3-E^4-$ in which each of $E^2$ and $E^3$ is CH and $E^4$ is O or S with $E^1$ representing a single bond,
where $R^2$, $R^3$, $R^4$ and $R^5$ are each independently a hydrogen atom, a fluorine atom, a chlorine atom, a bromine atom, an iodine atom, a cyano group, an optionally substituted $C_{1-6}$ alkyl group, an optionally substituted aryl group, an optionally substituted heteroaryl group, an optionally substituted three- to seven-membered ring heterocycloalkyl group, or a group represented by the formula: $-Q-(CH_2)_n-R^{14}$,
n is 0, 1, 2 or 3,
Q is a group of the formula: -CH=CH-, O, CO, a group represented by the formula: -C(=O)-O-, a group represented by the formula: $-C(=O)-N(R^{56})-$, $NR^{56}$, a group represented by the formula: $-N(R^{56})-C(=O)-$, or a group represented by the formula: $-N(R^{56})-C(=O)-O-$,
$R^{56}$ is a hydrogen atom, an optionally substituted $C_{1-3}$ alkyl group, or a group represented by the formula: $-C(=O)-R^{84}$,
$R^{84}$ is a hydrogen atom, an optionally substituted $C_{1-6}$ alkyl group, an optionally substituted aryl group, an optionally substituted $C_{1-6}$ alkyloxy group, or an optionally substituted aryloxy group, and
$R^{14}$ is a hydrogen atom, an optionally substituted $C_{1-6}$ alkyl group, an optionally substituted aryl group, an optionally substituted heteroaryl group, an optionally substituted $C_{3-8}$ cycloalkyl group, or an optionally substituted three- to seven-membered ring heterocycloalkyl group; and
the structure formed by $G^1$, $G^2$, $G^3$ and $G^4$ is a group represented by the formula: $-G^1-G^2-G^3-G^4-$ (where the bonds between $G^1$, $G^2$, $G^3$ and $G^4$ are single bonds or double bonds) which is represented by the formula: $-CH=CH-CH=CR^6-$ (with the exception of cases where $A^1$ and $A^2$ each represent a single bond, with $A^3$ being O and $A^4$ being CO), the formula: -CH=CH-CH=N- (with the exception of cases where $A^1$ and $A^2$ each represent a single bond, with $A^3$ being O and $A^4$ being CO), the formula: $-CH_2-CH_2-CH_2-CH_2-$, the formula: $-CO-CH_2-CH_2-N(R^7)-$, the formula: $-CH_2-CF_2-CH_2-CH_2-$, the formula: $-CH_2-O-CH_2-CH_2-$, the formula: $-CH_2-S-CH_2-CH_2-$, the formula: $-CH_2-CH_2-N(R^7)-CH_2-$, the formula: $-CH_2-CH_2-CH_2-O-$, the formula: $-CH_2-CH_2-CH_2-N(R^7)-$or the formula: $-O-CH_2-CH_2-N(R^7)-$, or a group represented by the formula: $-G^2-G^3-G^4-$ (where the bonds between $G^2$, $G^3$ and $G^4$ are single bonds or double bonds) which is represented by the formula: $-CH=CH-N(R^7)-$, the formula: $-CH_2-CH_2-N(R^7)-$, the formula: $-N=CH-N(R^7)-$ or the formula: $-N(R^7)-CH=N-$, with $G^1$ representing a single bond,
where $R^6$ is a hydrogen atom, a fluorine atom, a chlorine atom, a bromine atom, an iodine atom, an optionally substituted $C_{1-6}$ alkyl group, or an optionally substituted $C_{1-6}$ alkyloxy group,
$R^7$ is a hydrogen atom, an optionally substituted $C_{1-6}$ alkyl group, an optionally substituted $C_{3-8}$ cycloalkyl group, an optionally substituted $C_{3-8}$ cycloalkyl $C_{1-3}$ alkyl group, an optionally substituted three- to seven-membered ring heterocycloalkyl group, optionally substituted three- to seven-membered ring heterocycloalkyl $C_{1-3}$ alkyl group, a group represented by the formula:$-C(=O)-R^{15}$, or a group represented by the formula $-(CH_2)_p-C(=O)-OR^{16}$,
p is 0, 1, 2 or 3,
$R^{15}$ is a hydrogen atom, an optionally substituted $C_{1-6}$ alkyl group or $OR^{57}$,
$R^{57}$ is an optionally substituted $C_{1-6}$ alkyl group, or an optionally substituted aryl $C_{1-3}$ alkyl group, and
$R^{16}$ is a hydrogen atom or an optionally substituted $C_{1-6}$ alkyl group.

When used in combination with any of anticancer agents other than the following microtubule inhibitory compounds, more specifically alkylating agents (e.g., temozolomide and melphalan), antimetabolites (e.g., gemcitabine, cytarabine (Ara-C), fluorouracil (5-FU), pemetrexed, mercaptopurine and methotrexate), plant alkaloids (the "plant alkaloids" in the present invention includes analogues in which some of atoms of the plant alkaloid are substituted with other atoms (e.g., irinotecan (SN-38) and etoposide (VP-16))), topoisomerase inhibitors (which include irinotecan (SN-38) and etoposide (VP-16)), agents serving as both a plant alkaloid and a topoisomerase inhibitor (including agents serving as both an analogue of a plant alkaloid and a topoisomerase inhibitor (e.g., irinotecan (SN-38) and etoposide (VP-16))), anticancer antibiotics (e.g., actinomycin D, daunorubicin, doxorubicin, bleomycin and mitoxantrone) and platinating agents (e.g., oxaliplatin, carboplatin and cisplatin), the spiro compound exhibits an excellent anticancer action without causing inhibition of each other's anticancer action. When used in combination with any of the following microtubule inhibitory compounds, the spiro compound exhibits a further excellent synergistic anticancer action.

In the formula (1), $A^1$, $A^2$, $A^3$ and $A^4$ form a structure in which $A^1$ and $A^2$ each represent a single bond, one of $A^1$ and $A^2$ represents a single bond and the other represents $CH_2$, or $A^1$ represents a single bond and $A^4$ represents $CH_2$. When $A^1$ and $A^2$ each represent a single bond, $A^3$ is $CH_2$ or CO and $A^4$ is O or $NR^1$, or $A^3$ is O or $NR^1$ and $A^4$ is $CH_2$ or CO. When $A^1$ represents $CH_2$ and $A^2$ represents a single bond, $A^3$ is $CH_2$ or CO and $A^4$ is O or $NR^1$, or $A^3$ is O or $NR^1$ and $A^4$ is $CH_2$ or CO. When $A^1$ represents a single bond and $A^2$ represents $CH_2$, $A^3$ is $NR^1$ and $A^4$ is $CH_2$ or CO, or $A^3$ is $CH_2$ or CO and $A^4$ is $NR^1$. Further, when $A^1$ represents a single bond and $A^4$ represents $CH_2$, $A^2$ is $NR^1$ and $A^3$ is $CH_2$ or CO, or $A^2$ is $CH_2$ or CO and $A^3$ is $NR^1$.

In the formula (1), the structure formed by $A^1$, $A^2$, $A^3$ and $A^4$ is preferably a structure in which $A^1$ and $A^2$ each represent a single bond; more preferably a structure in which one of $A^3$ and $A^4$ is $CH_2$ or CO and the other is O or $NR^1$; still more preferably a structure in which $A^3$ is O, $CH_2$ or CO and $A^4$ is CO or $NR^1$ (with the exception of cases where each of $A^3$ and $A^4$ is CO, where $A^3$ is $CH_2$ and $A^4$ is CO, and where $A^3$ is O and $A^4$ is $NR^1$) ; especially preferably a structure in which the combination of $A^3$ and $A^4$ is a combination of O and CO, $CH_2$ and $NR^1$ or CO and $NR^1$.

In the formula (1), the structure formed by $E^1$, $E^2$, $E^3$ and $E^4$ is a group represented by the formula: $-E^1-E^2-E^3-E^4-$ (where the bonds between $E^1$, $E^2$, $E^3$ and $E^4$ are single bonds or double bonds), or a group represented by the formula: $-E^2-E^3=E^4-$ or the formula: $-E^2=E^3-E^4-$ with $E^1$ representing a single bond. Herein, unless otherwise specified, the symbols "-" and "=" which represent bonds between atoms and/or groups in the structural formulae denote a single bond and a double bond, respectively, and the bonds between $E^1$, $E^2$, $E^3$ and $E^4$ in the group represented by the above formula: $-E^1-E^2-E^3-E^4-$ are single bonds or double bonds depending on a combination of atoms or groups which correspond to $E^1$, $E^2$, $E^3$ and $E^4$.

In the formula (1), the structure formed by $E^1$, $E^2$, $E^3$ and $E^4$ is preferably a group represented by the above formula: $-E^1-E^2-E^3-E^4-$ (where the bonds between $E^1$, $E^2$, $E^3$ and $E^4$ are single bonds or double bonds); more preferably a group in which $E^1$ is N or $CR^2$, $E^2$ is N or $CR^3$, $E^3$ is N or $CR^4$ and $E^4$ is N or $CR^5$; still more preferably a group in which $E^1$ is $CR^2$, $E^2$ is $CR^3$, $E^3$ is $CR^4$ and $E^4$ is $CR^5$; especially preferably a group in which $E^1$, $E^2$, $E^3$ and $E^4$ (Cs of $CR^2$, $CR^3$, $CR^4$ and $CR^5$) form a six-membered ring aromatic hydrocarbon group with two adjacent carbon atoms.

Further, in the formula (1), the structure formed by $G^1$, $G^2$, $G^3$ and $G^4$ is a group represented by the formula: $-G^1-G^2-G^3-G^4-$ (where the bonds between $G^1$, $G^2$, $G^3$ and $G^4$ are single bonds or double bonds), or a group represented by the formula: $-G^2-G^3-G^4-$ (where the bonds between $G^2$, $G^3$ and $G^4$ are single bonds or double bonds) with $G^1$ representing a single bond. In both the group represented by the formula: $-G^1-G^2-G^3-G^4-$ and the group represented by the formula: $-G^2-G^3-G^4-$, the bonds between $G^1$, $G^2$, $G^3$ and $G^4$ are single bonds or double bonds depending on a combination of atoms or groups which correspond to $G^1$, $G^2$, $G^3$ and $G^4$.

In the formula (1), the structure formed by $G^1$, $G^2$, $G^3$ and $G^4$ is preferably a group represented by the above formula: $-G^1-G^2-G^3-G^4-$ which is represented by the above formula: $-CH_2-CH_2-CH_2-CH_2-$, the above formula: $-CH_2-CF_2-CH_2-CH_2-$, the above formula: $-CH_2-O-CH_2-CH_2-$, the above formula: $-CH_2-S-CH_2-CH_2-$, the above formula: $-CH_2-CH_2-CH_2-O-$, the above formula: $-CH_2-CH_2-CH_2-N(R^7)$ or the above formula: $-O-CH_2-CH_2-N(R^7)-$, or a group represented by the formula: $-G^2-G^3-G^4-$ (where the bonds between $G^2$, $G^3$ and $G^4$ are single bonds or double bonds) which is represented by the formula: $-CH=CH-N(R^7)-$ or the formula: $-CH_2-CH_2-N(R^7)-$, with $G^1$ representing a single bond; more preferably a group represented by the above formula: $-G^1-G^2-G^3-G^4-$ which is represented by the above formula: $-CH_2-CH_2-CH_2-CH_2-$ or the above formula: $-CH_2-CH_2-CH_2-N(R^7)$

In the formula (1), the "acyl groups in a broad sense" include groups (groups represented by the formula: $-C(=O)-Ar^4$) in which a hydrogen atom, an amino group, or a $C_{1-6}$ alkyl group, an aryl group, a heteroaryl group, a $C_{3-8}$ cycloalkyl group or a heterocycloalkyl group as defined herein (which is represented by the formula: $-Ar^4$) is bonded to a carbonyl group; and groups (groups represented by the formula: $-C(=O)-O-Ar^5$) in which a hydrogen atom, or a $C_{1-6}$ alkyl group, an aryl group, a heteroaryl group, a $C_{3-8}$ cycloalkyl group or a heterocycloalkyl group as defined herein (which is represented by the formula: $-Ar^5$) is bonded to a carbonyl group via an oxygen atom. The acyl group is optionally substituted with any one or more substituents as defined herein. Examples of the acyl groups in a broad sense generally include, but are not limited to, groups such as formyl, acetyl, propionyl, butyroyl, valeroyl, pivaloyl, trifluoroacetyl, chloroacetyl, dichloroacetyl, trichloroacetyl, hydroxyacetyl, phenylacetyl, benzoyl, naphthoyl, furoyl, thenoyl, nicotinoyl, isonicotinoyl, methoxycarbonyl, trichloromethoxycarbonyl, ethoxycarbonyl, tert-butyloxycarbonyl, phenyloxycarbonyl, benzyloxycarbonyl, 4-methoxybenzyloxycarbonyl, 2,4-dimethoxybenzyloxycarbonyl and 4-nitrobenzyloxycarbonyl groups. Examples of the acyl group also include groups represented by the formula: $-C(=O)-N(R^{58a})-R^{58b}$ [where $R^{58a}$ and

$R^{58b}$ each independently a hydrogen atom, a $C_{1-6}$ alkyl group, or a group represented by the formula: $-S(=O)_2-CH_3$], and carboxy groups (-COOH).

In the formula (1), examples of the optional substituents include substituents such as halogen atoms (fluorine atom, chlorine atom, bromine atom and iodine atom), a cyano group, a hydroxyl group, a thiol group, a nitro group, the acyl groups in a broad sense, $C_{1-6}$ alkyl groups, aryl groups, heteroaryl groups, $C_{3-8}$ cycloalkyl groups, heterocycloalkyl groups, aryl $C_{1-3}$ alkyl groups, heteroaryl $C_{1-3}$ alkyl groups, $C_{3-8}$ cycloalkyl $C_{1-3}$ alkyl groups and heterocycloalkyl $C_{1-3}$ alkyl groups; substituents in which the acyl group, a $C_{1-6}$ alkyl group (alkoxy group or alkylthio group), an aryl group, an aryl $C_{1-3}$ alkyl group, a heteroaryl group, a $C_{3-8}$ cycloalkyl group, a heterocycloalkyl group, a heterocycloalkyl $C_{1-3}$ alkyl group or a group represented by the formula: - $SiR^{31a}R^{31b}R^{31c}$ [where $R^{31a}$, $R^{31b}$ and $R^{31c}$ each independently represent a $C_{1-6}$ alkyl group or an aryl group] is bonded via an oxygen atom or a sulfur atom; substituents represented by the formula: $-N(R^{32a})-R^{32b}$ [where $R^{32a}$ and $R^{32b}$ are each independently a hydrogen atom, the acyl group in a broad sense, a $C_{1-6}$ alkyl group, a group represented by the formula: $-S(=O)_2-N(CH_3)_2$, or a group represented by the formula: $-S(=O)_2-CH_3$, or $R^{32a}$ and $R^{32b}$ are linked together to form a three- to seven-membered ring heterocycloalkyl group optionally containing at least one atom or group selected from the group consisting of an oxygen atom, a sulfur atom, SO, $S(=O)_2$ and $NR^{33}$ [$R^{33}$ represents a hydrogen atom or a $C_{1-6}$ alkyl group]; substituents represented by the formula: - $C(NH_2)=NH$; substituents represented by the formula: - $S(=O)_2-N(R^{59a})-R^{59b}$ [where $R^{59a}$ and $R^{59b}$ each independently represent a hydrogen atom, the acyl group in a broad sense or a $C_{1-6}$ alkyl group]; substituents represented by the formula: $-S(=O)_2-CH_3$; and substituents represented by the formula: -P(=O)-OH. Of the substituents and groups involved in formation of the substituents, the acyl groups in a broad sense, alkyl groups, aryl groups, heteroaryl groups, cycloalkyl groups and heterocycloalkyl groups may be further substituted with any substituent as in the definition described above.

In the formula (1), when the group substituted is a $C_{1-6}$ alkyl group, a heteroaryl group, a $C_{3-8}$ cycloalkyl $C_{1-3}$ alkyl group, an aryl $C_{1-3}$ alkyl group, a heteroaryl $C_{1-3}$ alkyl group or a three- to seven-membered ring heterocycloalkyl $C_{1-3}$ alkyl group which is represented by $R^1$, the substituent is especially preferably a methoxy group ($-OCH_3$), a cyano group, a halogen atom or a hydroxyl group.

In the formula (1), when the group substituted is a $C_{1-6}$ alkyl group, an aryl group, a heteroaryl group or a three- to seven-membered ring heterocycloalkyl group which is represented by $R^2$, $R^3$, $R^4$ or $R^5$, the substituent is especially preferably a cyano group; a hydroxyl group; a heterocycloalkyl group; a heterocycloalkyl $C_{1-3}$ alkyl group; an aryl $C_{1-3}$ alkyl group; a carboxy group; a $C_{1-3}$ alkoxy group; a primary amide group; a methoxycarbonyl group; an acetyl group optionally substituted with a hydroxyl group; a $C_{1-6}$ alkyl group optionally substituted with a halogen atom, a hydroxyl group or a $C_{1-3}$ alkoxy group; a substituent represented by the formula: $-C(=O)-R^{60}$ [where $R^{60}$ is a $C_{1-3}$ alkyl group or a heterocycloalkyl group]; or a substituent represented by the formula: - $N(R^{32a})-R^{32b}$ [where $R^{32a}$ and $R^{32b}$ are more preferably each independently a hydrogen atom or a $C_{1-3}$ alkyl group].

In the formula (1), $R^1$ is preferably a hydrogen atom, an optionally substituted $C_{1-6}$ alkyl group, an optionally substituted aryl $C_{1-3}$ alkyl group, an optionally substituted heteroaryl $C_{1-3}$ alkyl group or a three- to seven-membered ring heterocycloalkyl $C_{1-3}$ alkyl group; preferably a hydrogen atom, or a $C_{1-6}$ alkyl group optionally substituted with one to three substituents selected from the group consisting of a hydroxyl group, a $C_{1-6}$ alkoxy group, a fluorine atom, a chlorine atom, a bromine atom, an iodine group, a cyano group, a nitro group, an aryl group, a heteroaryl group, a $C_{3-8}$ cycloalkyl group, a heterocycloalkyl group and the acyl group in a broad sense (when two or more substituents are present, the substituents are the same or different, and the aryl group, the heteroaryl group, the $C_{3-8}$ cycloalkyl group and the heterocycloalkyl group may be further substituted).

More specific examples of $R^1$ include a methyl group, an ethyl group, a propyl group, an isopropyl group, a butyl group, an isobutyl group, a sec-butyl group, a tert-butyl group, a pentyl group, an isopentyl group, a hexyl group, an isohexyl group, a 2-hydroxyethyl group, a 2-hydroxypropyl group, a 3-hydroxypropyl group, a 2,3-dihydroxypropyl group, a 2-hydroxy-2-methylpropyl group, a 2-methoxyethyl group, a 3-methoxypropyl group, a 2,2,2-trifluoroethyl group, a 2-fluoroethyl group, a 2,3-difluoroethyl group, a 2-chloroethyl group, a 3-chloropropyl group, a 2-bromoethyl group, a 2-iodoethyl group, a cyanomethyl group, a 2-cyanoethyl group, a benzyl group, a 2-fluorophenylmethyl group, a 2-pyridylmethyl group, a 3-pyridylmethyl group, a 4-pyridylmethyl group, a 6-chloro-3-pyridyl group, a 2-pyrimidylmethyl group, a 5-pyrimidylmethyl group, a cyclopropylmethyl group, a 2-tetrahydrofurylmethyl group, an amidinomethyl group and a carbamoylmethyl group.

In the formula (1), it is preferable that $R^2$, $R^3$, $R^4$ and $R^5$ satisfy the following condition: each of $R^2$, $R^3$, $R^4$ and $R^5$ is a hydrogen atom, one or two of $R^2$, $R^3$, $R^4$ and $R^5$ are at least one selected from the group consisting of a halogen atom (fluorine atom, chlorine atom, bromine atom or iodine atom) and a cyano group, one of $R^2$, $R^3$, $R^4$ and $R^5$ is an optionally substituted aryl group, an optionally substituted heteroaryl group, an optionally substituted three- to seven-membered ring heterocycloalkyl group or a group represented by the above formula: $-Q-(CH_2)_n-R^{14}$.

The group presented by the above formula: $-Q-(CH_2)_n-R^{14}$ is preferably a group represented by the formula: $-OR^{14}$ or a group represented by the formula: $-C(=O)-O-R^{14}$, more preferably a group represented by the above formula: $-O-R^{14}$. Further, when one of $R^2$, $R^3$, $R^4$ and $R^5$ is a group represented by the above formula: $-Q-(CH_2)_n-R^{14}$, $R^{14}$ is preferably a hydrogen atom (i.e. for example, in the case of a group represented by the above formula: $-O-R^{14}$, one of

$R^2$, $R^3$, $R^4$ and $R^5$ is a hydroxyl group), a $C_{1-2}$ alkyl group optionally substituted with one substituent, or an optionally substituted three- to seven-membered ring heterocycloalkyl group. The substituent of the optionally substituted $C_{1-2}$ alkyl group is preferably a substituent represented by the formula: -C(=O)-$R^{61}$, a substituent represented by the above formula: -S(=O)$_2$-N($R^{59a}$)-$R^{59b}$, a substituent represented by the above formula: -S(=O)$_2$-$CH_3$, or a substituent represented by the above formula: -P(=O)-OH.

More specifically, $R^2$, $R^3$, $R^4$ and $R^5$ are each independently a hydrogen atom, a fluorine atom, a chlorine atom, a bromine atom, an iodine atom, a cyano group, a hydroxyl group, a methoxy group, an ethoxy group, a 2-hydroxyethoxy group, a 2-methoxyethoxy group, a carboxymethoxy group, a 5-tetrazolylmethoxy group, a cyanomethoxy group, a 4-piperidylmethoxy group, a 2-(N,N-dimethylamino)ethoxy group, a 3-oxetanylmethoxy group, a 2-morpholinoethoxy group, a 2-(N-methylpiperazino)ethoxy group, a 2-pyrrolizinoethoxy group, a 2-piperidinoethoxy group, a 3-pyrrolizinopropoxy group, a 3-tetrahydrofuryloxy group, a 4-tetrahydropyranyloxy group, a 4-(N-methylpiperidyl)methoxy group, a 2-hydroxy-2-methylpropoxy group, a carbamoylmethoxy group, a piperidino group, a morpholino group, a piperazino group, a 4-cyanopiperidino group, a 4-methoxycarbonylpiperazino group, a 3,5-dimethylmorpholino group, a 3,5-dimethylpiperazino group, a 4-methoxypiperidino group, a 4-carboxypiperidino group, N-methylsulfonylpiperazino group, a 4-methylsulfonylpiperidino group, a N-2-hydroxy-2-methylpropylpiperazino group, a N-hydroxyacetylpiperazino group, a N-acetylpiperazino group, a N-methylpiperazino group, a N-(3-oxetanyl)piperazino group, a 4-hydroxycyclohexyl group, a 1-methylpyrazol-4-yl group, a 4-(N,N-dimethylamino)phenyl group, a 4-ethoxycarbonyloxazol-2-yl group, a 4-(N-methylpiperazino)phenyl group, an ethoxycarbonyl group, a N-(2-morpholinoethyl)carbamoyl group, a 2-oxazolyl group, a 4-morpholinocarbonyloxazol-2-yl group, a 4-pyrrolizinomethyloxazol-2-yl group or a 4-carboxyoxazol-2-yl group.

In the formula (1), $R^7$ is preferably a hydrogen atom, or a $C_{1-6}$ alkyl group optionally substituted with one to three substituents selected from the group consisting of a hydroxyl group, a $C_{1-6}$ alkoxy group, a fluorine atom, a chlorine atom, a bromine atom, an iodine atom and a cyano group (when two or more substituents are present, the substituents are the same or different). More specifically, $R^7$ is a hydrogen atom, a methyl group, an ethyl group, a propyl group, an isopropyl group, a 2-hydroxyethyl group, a 3-hydroxypropyl group, a 2,2,2-trifluoroethyl group, a 2-cyanoethyl group or a 2-methoxyethyl group.

Examples of preferred aspects of compounds of the formula (1) according to the present invention include aspects in which the compounds of the above formula (1) are represented by the formula (1-1):

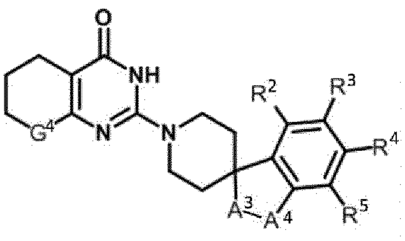

(1-1)

In the formula (1-1), $A^3$ is O, $CH_2$ or CO, and $A^4$ is CO or $NR^1$, with the exception of cases where each of $A^3$ and $A^4$ is CO, where $A^3$ is $CH_2$ and $A^4$ is CO, and where $A^3$ is O and $A^4$ is $NR^1$. $R^1$ is as described for the above formula (1).

In the formula (1-1), $G^4$ is $CH_2$ or $NR^7$, and $R^7$ is a hydrogen atom or an optionally substituted $C_{1-6}$ alkyl group.

Examples of more preferred aspects of compounds of the formula (1) according to the present invention include, but are not particularly limited to, compounds of the formula (1-1) in which $A^3$ is CO or $CH_2$, $A^4$ is $NR^1$ ($R^1$ is more preferably a hydrogen atom or a $C_{1-6}$ alkyl group), $R^2$ is a halogen atom, and $G^4$ is $NR^7$ ($R^7$ is more preferably a $C_{1-6}$ alkyl group).

The compounds of the above formula (1) or pharmacologically acceptable salts thereof (spiro compounds) can be produced through the methods shown below. The method for producing a spiro compound according to the present invention is not limited to the methods shown below, and a range of the spiro compounds according to the present invention is not limited to compounds produced by the following production methods.

The method for producing a spiro compound according to the present invention can be carried out by combining a wide range of various kinds of synthesis methods known to persons skilled in the art, methods obtained by making a modification or the like to the synthesis methods if necessary, and the like while using starting raw materials, precursors, reagents and solvents which are commercially available or can be synthesized through methods known to persons skilled in the art.

In the present invention, a method for introducing, modifying or converting any substituent or the like can be carried out by introducing, modifying or converting an intended substituent itself or a group convertible to the substituent in a raw material stage, an intermediate material stage or a final-form material stage by combining a wide range of various kinds of synthesis methods known to persons skilled in the art, methods obtained by making a modification or

the like to the synthesis methods if necessary, and the like. The method can also be carried out by appropriately changing the order of reaction steps, etc. The method can also be carried out by appropriately employing general means such as protection and deprotection of functional groups which are commonly used in organic synthesis chemistry if necessary for convenience of the reaction (e.g., methods described in, for example, Green Wuts, PROTECTIVE GROUPS in ORGANIC SYNTHESIS THIRD EDITION, John Wiley & Sons, Inc.).

Reaction apparatuses usable in production of the compound include common glass reaction vessels, optionally glass-lined metallic reaction baths, and flow reactors. Examples of cooling or heating at the time of carrying out the reaction include air cooling, water cooling, ice cooling, combination of a cryogen and a cooling medium, and cooling of a reaction vessel or a reaction mixture through a cooling medium cooled by a freezing machine, or heating with hot water or steam, heating of a reaction vessel directly by an electric heater or through a heating medium, and heating by application of an ultrashort electromagnetic wave (i.e. microwave heating). Further, cooling or heating using a Peltier device, etc. can also be performed.

The spiro compound according to the present invention can be prepared through, for example, the following production method 1 or production method 2.

(Production method 1)

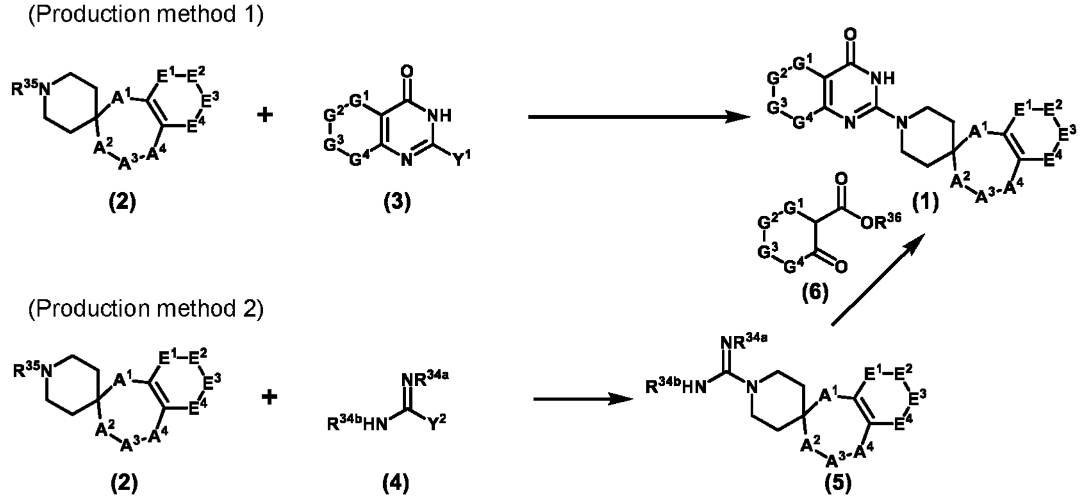

(2) (3) (1) (6)

(Production method 2)

(2) (4) (5)

In the above formulae (2) to (6), $A^1$, $A^2$, $A^3$, $A^4$, $E^1$, $E^2$, $E^3$, $E^4$, $G^1$, $G^2$, $G^3$ and $G^4$ are the same as $A^1$, $A^2$, $A^3$, $A^4$, $E^1$, $E^2$, $E^3$, $E^4$, $G^1$, $G^2$, $G^3$ and $G^4$, respectively, in the above formula (1). In the above formulae (3) and (4), $Y^1$ and $Y^2$ each independently represent a leaving group (the leaving group means a functional group with a leaving ability which is known to persons skilled in the art, and examples thereof include protective groups described in, for example, Green Wuts, PROTECTIVE GROUPS in ORGANIC SYNTHESIS THIRD EDITION, John Wiley & Sons, Inc.). Further, in the above formulae (4) and (5), $R^{34a}$ and $R^{34b}$ each independently represent a hydrogen atom or the protective group. Further, in the above formula (2), $R^{35}$ represents a hydrogen atom or the protective group, and in the above formula (6), $R^{36}$ represents an optionally substituted $C_{1-6}$ alkyl group.

A compound of the formula (2) (hereinafter, referred to as an "intermediate (2)") and a compound of the formula (3) (hereinafter, referred to as a "raw material (3)") in the production method 1 and the intermediate (2) and compounds of the formulae (4) and (6) (hereinafter, referred to as a "raw material (4)" and a "raw material (6)", respectively) in the production method 2 can be synthesized using commercially available reagents, or known methods or methods based on the known methods. The intermediate (2) may have a protective group if necessary, and the protective group can be deprotected if necessary in any stage.

In the production method 1, the intermediate (2) and the raw material (3) are dissolved or suspended in an appropriate solvent, and reacted in the presence or non-presence of a metal catalyst and a ligand thereof and in the presence or non-presence of a base to produce a spiro compound according to the present invention (a compound of the formula (1) or a salt thereof; hereinafter, sometimes referred to as a "compound (1)").

In the production method 1, the intermediate (2) and the raw material (3) are used normally at a molar ratio in the range of 1 : 1 to 3, preferably at a molar ratio in the range of 1 : 1 to 1.5.

Examples of the solvent to be used in the production method 1 include protic solvents such as water and alcohols (e.g., ethanol); hydrocarbon-based solvents such as petroleum ether, nonaromatic hydrocarbons (e.g., n-hexane) and aromatic hydrocarbons (e.g., toluene); halogen-based solvents such as halogenated (e.g., chlorinated or fluorinated) aromatic hydrocarbons (e.g., chlorobenzene) or nonaromatic hydrocarbons (e.g., 1,2-dichloroethane); chain ether-based solvents (e.g., 1,2-dimethoxyethane) or cyclic ether-based solvents (e.g., 1,4-dioxane); ester-based solvents (e.g., ethyl acetate); and aprotic polar solvents (e.g., acetonitrile and N,N-dimethylformamide), and one of these solvents

can be used alone, or two or more thereof can be mixed at an appropriate ratio and used. Of these, at least one of toluene, ethanol, 1,4-dioxane and N,N-dimethylformamide is preferable as the solvent.

Examples of the metal catalyst to be used at the time of carrying out the reaction in the presence of the metal catalyst in the production method 1 include zerovalent palladium catalysts such as tris(dibenzylideneacetone)dipalladium (0); divalent palladium catalysts such as palladium acetate (II) and bis(acetonitrile)dipalladium (II); and palladium phosphine complexes such as tetrakis(triphenylphosphine)palladium (0) and bis(tri-tert-butylphosphine)palladium (0). As a ligand at the time of carrying out the reaction in the presence of the ligand, di-tert-butylphosphine or the like is used. The metal catalyst and the ligand thereof are used at a molar ratio of normally 1 : 0.5 to 2, preferably 1 : 1, and the amount of the metal catalyst and the ligand thereof used is in the range of 0.01 to 1 mol%, preferably in the range of 0.1 to 0.5 mol% based on the amount of the intermediate (2).

Examples of the base to be used at the time of carrying out the reaction in the presence of the base in the production method 1 include salts such as potassium carbonate and potassium phosphate; amines such as triethylamine and diisopropylethylamine; metal hydrides such as lithium hydride and sodium hydride; metal alkoxides such as sodium ethoxide, sodium tert-butoxide and potassium tert-butoxide; and metal amides such as lithium amide and lithium diisopropylamide, and one of these bases can be used alone, or two or more thereof can be mixed at an appropriate ratio and used. Of these, at least one of potassium phosphate, triethylamine, diisopropylethylamine and sodium tert-butoxide is preferably used as the base. The amount of the base used is in the range of 0.01 to 20 equivalents, preferably in the range of 0.1 to 10 equivalents, more preferably in the range of 1 to 5 equivalents based on the amount of the intermediate (2).

In the production method 1, the reaction temperature is in the range of 0 to 250°C, preferably 30 to 200°C, more preferably 60 to 160°C. In the production method 1, the reaction time is in the range of 1 minute to 2 days, preferably 5 minutes to 12 hours, more preferably 10 minutes to 6 hours.

In the production method 1, substituents with which $A^1$, $A^2$, $A^3$, $A^4$, $G^1$, $G^2$, $G^3$, $G^4$, $E^1$, $E^2$, $E^3$ and $E^4$ are optionally substituted may have intended substituents in the stages of the intermediate (2) and the raw material (3). In this case, the resulting compound (1) has intended substituents. By introducing, modifying or converting precursor groups for intended substituents in the intermediate (2) and/or the raw material (3) by combining a wide range of various kinds of synthesis methods known to persons skilled in the art, the compound (1) having intended substituents can be produced. These synthesis methods can be arbitrarily combined, and protection and deprotection may be appropriately performed if necessary.

In the production method 2, the intermediate (2) and the raw material (4) are dissolved or suspended in an appropriate solvent, and reacted in the presence of a base to synthesize a compound of the formula (5) (hereinafter, referred to as an "intermediate (5)"), and the intermediate (5) and the raw material (6) are then dissolved or suspended in an appropriate solvent, and reacted in the presence of a base to produce a spiro compound according to the present invention (compound (1)). While the reaction can be used in the next step after the intermediate (5) is purified and isolated, the reaction can be continuously carried out as a one-pot reaction. Steps of protection, deprotection and the like can be appropriately added if necessary.

In the production method 2, the intermediate (2) and the raw material (4), and the intermediate (5) and the raw material (6) are used generally at a molar ratio in the range of 1 : 1 to 3, preferably at a molar ratio in the range of 1 : 1 to 2.

In the production method 2, examples of the solvent include solvents which are the same as listed as solvents for the production method 1, and of these, at least one of water, ethanol, acetonitrile and N,N-dimethylformamide is preferably used as the solvent.

In the production method 2, examples of the base include bases which are the same as listed as bases for the production method 1, of these, at least one of sodium ethoxide, triethylamine and diisopropylethylamine is preferably used as the base. The amount of the base used is in the range of 0.01 to 20 equivalents, preferably in the range of 0.1 to 10 equivalents, more preferably in the range of 1 to 5 equivalents based on the amount of the intermediates (2) and (5).

In the production method 2, the reaction temperature is in the range of -30 to 200°C, preferably 0 to 150°C, more preferably 20 to 120°C. In the production method 2, the reaction time is in the range of 1 minute to 2 days, preferably 5 minutes to 12 hours, more preferably 30 minutes to 6 hours.

In the production method 2, substituents with which $A^1$, $A^2$, $A^3$, $A^4$, $G^1$, $G^2$, $G^3$, $G^4$, $E^1$, $E^2$, $E^3$ and $E^4$ are optionally substituted may have intended substituents in the stages of the intermediate (2) and the raw material (6). In this case, the resulting compound (1) has intended substituents. By introducing, modifying or converting precursor groups for intended substituents in the intermediate (2) and/or the raw material (6) by combining a wide range of various kinds of synthesis methods known to persons skilled in the art, the compound (1) having intended substituents can be produced. These synthesis methods can be arbitrarily combined, and protection, deprotection and the like may be appropriately performed if necessary.

The spiro compound synthesized through the above-described method, the intermediate, the raw material and the like may be used in the next step in a state of a reaction solution or a crude product, or used after being isolated through a common purification method known to persons skilled in the art. Examples of the purification method associated

with isolation include methods obtained by appropriately selecting or combining various types of chromatography (column or thin-layer and normal phase or reversed phase), distillation, sublimation, precipitation, crystallization and centrifugation.

(Dihydroquinazolinone-Based Compound)

The nicotinamide-type tankyrase inhibitory compound according to the present invention is preferably a compound of the formula (II) or a pharmacologically acceptable salt thereof (dihydroquinazolinone-based compound):

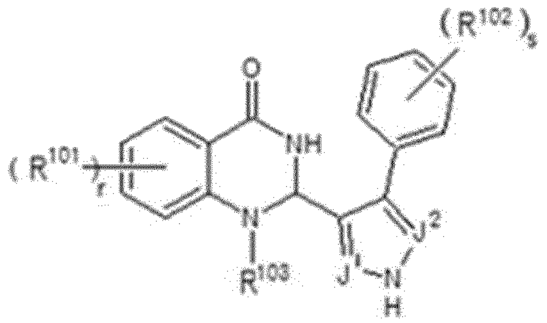

(11)

where $J_1$ and $J_2$ each represent CH or N, with the proviso that both $J_1$ and $J_2$ do not represent CH;
r represents 0 to 4;
each $R^{101}$ is the same or different when r is 2 or more, and represents a halogen atom, a $C_{1-6}$ alkyl group optionally substituted with a halogen atom, $OR^{111}$, or a group represented by the formula: $-N(R^{112a})-R^{112b}$,
where $R^{111}$, $R^{112a}$ and $R^{112b}$ are each independently a hydrogen atom or a $C_{1-6}$ alkyl group, and
s represents 0 to 5;
each $R^{102}$ is the same or different when s is 2 or more, and represents a halogen atom, a $C_{1-6}$ alkyl group, $OR^{113}$, a group represented by the formula: $-N(R^{114a})-R^{114b}$, a group represented by the formula: $-NH-C(=O)-R^{115}$, a group represented by the formula: $-C(=O)-R^{116}$, an optionally substituted aryl group, an optionally substituted heteroaryl group, a nitro group, or a cyano group,
where $R^{113}$ is a hydrogen atom, an optionally substituted alkyl group, an optionally substituted aryl group, an optionally substituted arylalkyl group, or an optionally substituted heteroaryl group,
$R^{114a}$ and $R^{114b}$ are each independently a hydrogen atom or a $C_{1-6}$ alkyl group,
$R^{115}$ is an optionally substituted alkyl group, an optionally substituted aryl group, an optionally substituted heteroaryl group, or a group represented by the formula: $-NH-R^{121}$,
$R^{116}$ is an optionally substituted alkyl group, an optionally substituted aryl group, an optionally substituted heteroaryl group, $OR^{122}$, or a group represented by the formula: $-N(R^{123a})-R^{123b}$,
$R^{121}$ is an optionally substituted alkyl group, an optionally substituted aryl group, or an optionally substituted heteroaryl group,
$R^{122}$ is a hydrogen atom, an optionally substituted alkyl group, an optionally substituted aryl group, or an optionally substituted heteroaryl group, and
$R^{123a}$ and $R^{123b}$ are each independently a hydrogen atom, an optionally substituted alkyl group, an optionally substituted aryl group, or an optionally substituted heteroaryl group, or $R^{123a}$ and $R^{123b}$ are linked together to form a cyclic amine;
$R^{103}$ represents a hydrogen atom, a $C_{1-6}$ alkyl group, a $C_{3-6}$ cycloalkyl group, or a $C_{3-6}$ cycloalkyl $C_{1-6}$ alkyl group; and
$R^{101}$ and $R^{103}$ are optionally linked together to form a five- to seven-membered ring hetero ring when $R^{101}$ is present at the 8-position.

When used in combination with any of anticancer agents other than the following microtubule inhibitory compounds, more specifically alkylating agents (e.g., temozolomide and melphalan), antimetabolites (e.g., gemcitabine, cytarabine (Ara-C), fluorouracil (5-FU), pemetrexed, mercaptopurine and methotrexate), plant alkaloids (e.g., irinotecan (SN-38) and etoposide (VP-16)), topoisomerase inhibitors (which include irinotecan (SN-38) and etoposide (VP-16)), agents serving as both a plant alkaloid and a topoisomerase inhibitor (e.g., irinotecan (SN-38) and etoposide (VP-16)), anticancer antibiotics (e.g., actinomycin D, daunorubicin, doxorubicin, bleomycin and mitoxantrone) and platinating agents (e.g., oxaliplatin, carboplatin and cisplatin), the dihydroquinazolinone-based compound exhibit an excellent anticancer action without causing inhibition of each other's anticancer action. When used in combination with any of the following microtubule inhibitory compounds, the dihydroquinazolinone-based compound exhibits a further excellent synergistic anticancer action.

In the formula (11), unless otherwise specified, the number associated with "position" indicates a position number at which $R^{101}$ is substituted in the dihydroquinazolinone-based compound, and a position number of quinazolinone is directly applied to the position number. Further, in the formula (11), unless otherwise specified, the alphabet (o, m or p) associated with "position" indicates a position number of a benzene substituent with which $R^{102}$ is substituted.

In the formula (11), the "alkyl group" is preferably a "$C_{1-6}$ alkyl group" having 1 to 6 carbon atoms, more preferably a "$C_{1-3}$ alkyl group" having 1 to 3 carbon atoms.

In the formula (11), the "aryl group" is preferably a monocyclic ring (phenyl group) which has a binding site at any possible position.

In the formula (11), the "heteroaryl group" is preferably quinolyl, isoquinolyl or 1H-pyrrolo[2,3-b]pyridinyl.

In the formula (11), the "cycloalkyl group" is preferably a monocyclic ring, more preferably a "$C_{3-8}$ cycloalkyl group" having 3 to 8 carbon atoms, still more preferably a "$C_{3-6}$ cycloalkyl group" having 3 to 6 carbon atoms.

In the formula (11), the "hetero ring" is preferably a five- to seven-membered ring, more preferably a five- or six-membered ring, still more preferably 2,3-dihydro-1H-pyrrole, 1,2,3,4-tetrahydropyrazine or 3,4-dihydro-2H-1,4-oxazine which has a binding site at any possible position.

In the formula (11), the "heterocycloalkyl group" is preferably piperazinyl, pyrrolidinyl or morpholinyl which has a binding site at any possible position.

In the formula (11), the "arylalkyl group" is preferably an "aryl $C_{1-6}$ alkyl group (aryl $C_{1-6}$ alkylene group)" in which the alkyl group has 1 to 6 carbon atoms, more preferably an "aryl $C_{1-3}$ alkyl group (aryl $C_{1-3}$ alkylene group)" in which the alkyl group has 1 to 3 carbon atoms. The "cycloalkylalkyl group" is preferably a "cycloalkyl $C_{1-6}$ alkyl group (cycloalkyl $C_{1-6}$ alkylene group)" in which the alkyl group has 1 to 6 carbon atoms, more preferably a "cycloalkyl $C_{1-3}$ alkyl group (cycloalkyl $C_{1-3}$ alkylene group)" in which the alkyl group has 1 to 3 carbon atoms, preferably a "$C_{3-6}$ cycloalkyl $C_{1-6}$ alkyl group ($C_{3-6}$ cycloalkyl $C_{1-6}$ alkylene group)" or "$C_{3-6}$ cycloalkyl $C_{1-3}$ alkyl group ($C_{3-6}$ cycloalkyl $C_{1-3}$ alkylene group)" in which the cycloalkyl group has 3 to 6 carbon atoms. Further, the "heterocycloalkylalkyl group" is preferably a "heterocycloalkyl $C_{1-6}$ alkyl group (heterocycloalkyl $C_{1-6}$ alkylene group)" in which the alkyl group has 1 to 6 carbon atoms, more preferably a "heterocycloalkyl $C_{1-3}$ alkyl group (heterocycloalkyl $C_{1-3}$ alkylene group)" in which the alkyl group has 1 to 3 carbon atoms.

In the formula (11), the "cyclic amine" is preferably pyrrolidine, piperidine, piperazine or morpholine.

In the formula (1), examples of the optional substituents include halogen atoms (fluorine atom, chlorine atom, bromine atom and iodine atom), a cyano group, a hydroxyl group, a thiol group, a nitro group, $C_{1-6}$ alkyl groups (preferably $C_{1-3}$ alkyl groups), aryl groups, heteroaryl groups, cycloalkyl groups (preferably $C_{3-6}$ cycloalkyl groups), heterocycloalkyl groups, arylalkyl groups (preferably aryl $C_{1-3}$ alkyl groups), heteroarylalkyl groups (preferably heteroaryl $C_{1-3}$ alkyl groups), cycloalkylalkyl groups (preferably $C_{3-6}$ cycloalkyl $C_{1-3}$ alkyl groups), heterocycloalkylalkyl groups (preferably heterocycloalkyl $C_{1-3}$ alkyl groups); groups represented by the formula: $-N(R^{133a})$ $-R^{133b}$ [where $R^{133a}$ and $R^{133b}$ are each independently a hydrogen atom, a $C_{1-6}$ alkyl group (preferably a $C_{1-3}$ alkyl group), a cycloalkyl group (preferably $C_{3-6}$ cycloalkyl group), an aryl group, a heteroaryl group, or a group represented by the formula: $-C(=O)-R^{133c}$ [where $R^{133c}$ represents a $C_{1-6}$ alkyl group (preferably a $C_{1-3}$ alkyl group) or a cycloalkyl group (preferably a $C_{3-6}$ cycloalkyl group), an aryl group or a heteroaryl group], or $R^{133a}$ and $R^{133b}$ are linked together to form a four- to seven-membered ring cyclic amine]; groups represented by the formula: $-R^{134}-C(=O)-R^{135}$ [where $R^{134}$ represents a single bond, or an alkylene group having 1 to 3 carbon atoms, and $R^{135}$ represents a hydrogen atom, a $C_{1-6}$ alkyl group (preferably a $C_{1-3}$ alkyl group), a cycloalkyl group (preferably a $C_{3-6}$ cycloalkyl group), an aryl group or a heteroaryl group]; groups represented by the formula: $-R^{136}-C(=O)-O-R^{137}$ [where $R^{136}$ represents a single bond, or an alkylene group having 1 to 3 carbon atoms, and $R^{137}$ represents a hydrogen atom, a $C_{1-6}$ alkyl group (preferably a $C_{1-3}$ alkyl group), a cycloalkyl group (preferably a $C_{3-6}$ cycloalkyl group), an aryl group or a heteroaryl group]; and groups of the formula: $-OR^{138}$ [where $R^{138}$ represents a $C_{1-6}$ alkyl group (preferably a $C_{1-3}$ alkyl group), cycloalkyl group (preferably a $C_{3-6}$ cycloalkyl group), a heterocycloalkyl group, an aryl group, a heteroaryl group, a cycloalkylalkyl group (preferably a $C_{3-6}$ cycloalkyl $C_{1-3}$ alkyl group), a heterocycloalkylalkyl group (preferably a heterocycloalkyl $C_{1-3}$ alkyl group), an arylalkyl group (preferably an aryl $C_{1-3}$ alkyl group) or a heteroarylalkyl group (preferably a heteroaryl $C_{1-3}$ alkyl group)]. Of the substituents and groups involved in formation of the substituents, the alkyl groups, aryl groups, heteroaryl groups, cycloalkyl groups and heterocycloalkyl groups may be further substituted with any substituent as in the definition described above.

In the formula (11), when the group substituted is an aryl group or a heteroaryl group which is represented by $R^{102}$, the substituent is especially preferably a halogen atom, a cyano group, a hydroxyl group, an optionally substituted piperazinyl group (more preferably a piperazinyl group optionally substituted with a $C_{1-6}$ alkyl group), a piperazinyl $C_{1-6}$ alkyl group such as a piperazinylmethyl group which is optionally substituted (more preferably a piperazinyl $C_{1-6}$ alkyl group such as a piperazinylmethyl group which is optionally substituted with $C_{1-6}$ alkyl group), morpholinyl; a group represented by the formula: $-N(R^{133a})$ $-R^{133b}$ [where preferably, $R^{133a}$ and $R^{133b}$ each independently represent a hydrogen atom or a $C_{1-3}$ alkyl group]; or a group represented by the formula: $-R^{136}-C(=O)-O-R^{137}$ [where preferably, $R^{136}$ represents an alkylene group having 1 to 3 carbon atoms, and $R^{137}$ represents a hydrogen atom or a $C_{1-3}$ alkyl group].

In the formula (11), when the group substituted is an arylalkylene group represented by $R^{113}$, or an alkyl group, an aryl group or a heteroaryl group which is represented by $R^{113}$ or $R^{122}$, the substituent is especially preferably a halogen atom, a hydroxyl group or a $C_{1-3}$ alkyl group; a group represented by the formula: - $N(R^{133a})$ -$R^{133b}$ [where preferably, $R^{133a}$ and $R^{133b}$ each independently represent a hydrogen atom or a $C_{1-3}$ alkyl group]; or a group represented by the formula: -$OR^{138}$ [where preferably, $R^{138}$ represents a $C_{1-3}$ alkyl group] .

Further, in the formula (11), when the group substituted is an alkyl group, an aryl group or a heteroaryl group which is represented by $R^{115}$ or $R^{116}$, the substituent is especially preferably a halogen atom, a hydroxyl group, a $C_{1-3}$ alkyl group; a group represented by the formula: -$N(R^{133a})$ -$R^{133b}$ [where preferably, $R^{133a}$ and $R^{133b}$ each independently represent a hydrogen atom or a $C_{1-3}$ alkyl group]; or a group represented by the formula: - $OR^{138}$ [where preferably, $R^{138}$ represents a $C_{1-3}$ alkyl group].

In the formula (11), when the group substituted is an alkyl group, an aryl group, an arylalkylene group, a heteroaryl group which is represented by $R^{121}$, $R^{123a}$ or $R^{123b}$, the substituent is especially preferably a halogen atom, a hydroxyl group, a $C_{1-3}$ alkyl group; a group represented by the formula: -$N(R^{133a})$ -$R^{133b}$ [where preferably, $R^{133a}$ and $R^{133b}$ each independently represent a hydrogen atom or a $C_{1-3}$ alkyl group]; or a group represented by the formula: -$OR^{138}$ [where preferably, $R^{138}$ represents a $C_{1-3}$ alkyl group].

In the formula (11), r represents the number of $R^{101}$, and is 0 to 4. r is preferably 0 (i.e. all the four groups corresponding to $R^{101}$ are hydrogen atoms), or 1 or 2. The position of $R^{101}$ is preferably the 7-position or the 8-position when r is 1 or 2.

In the formula (11), each $R^{101}$ is the same or different when r is 2 or more, and represents a $C_{1-6}$ alkyl group optionally substituted with a halogen atom, $OR^{111}$, a group represented by the formula: -$N(R^{112a})$-$R^{112b}$. Here, $R^{111}$, $R^{112a}$ and -$R^{112b}$ are each independently a hydrogen atom or a $C_{1-6}$ alkyl group.

$R^{101}$ is preferably an unsubstituted $C_{1-6}$ alkyl group; a $C_{1-6}$ alkyl group substituted with a halogen atom; or $OR^{111}$ in which $R^{111}$ is a hydrogen atom or a $C_{1-3}$ alkyl group. More specifically, $R^{101}$ is more preferably a methyl group, an ethyl group, a hydroxyl group or a trifluoromethyl group.

In the formula (11), s represents the number of $R^{102}$, and is 0 to 5. s is preferably 0 (i.e. all the five groups corresponding to $R^{102}$ are hydrogen atoms), or 1 to 3. The position of $R^{102}$ is preferably the m-position and/or the p-position when r is 1 to 3. The position of $R^{102}$ is preferably the p-position when r is 1.

In the formula (11), each $R^{102}$ is the same or different when s is 2 or more, and represents a halogen atom, $OR^{113}$, a group represented by the formula: -$N(R^{114a})$-$R^{114b}$, a group represented by the formula: -NH-C(=O)-$R^{115}$, a group represented by the formula: -C(=O)-$R^{116}$, an optionally substituted aryl group, an optionally substituted heteroaryl group, a nitro group or a cyano group.

Here, $R^{113}$ is a hydrogen atom, an optionally substituted alkyl group, an optionally substituted aryl group, an optionally substituted arylalkyl group, or an optionally substituted heteroaryl group; $R^{114a}$ and $R^{114b}$ are each independently a hydrogen atom or a $C_{1-6}$ alkyl group; $R^{115}$ is an optionally substituted alkyl group, an optionally substituted aryl group, an optionally substituted heteroaryl group, or a group represented by the formula: -NH-$R^{121}$; and $R^{116}$ is an optionally substituted alkyl group, an optionally substituted aryl group, an optionally substituted heteroaryl group, $OR^{122}$, or a group represented by the formula: -$N(R^{123a})$-$R^{123b}$.

Further, $R^{121}$ is an optionally substituted alkyl group, an optionally substituted aryl group, or an optionally substituted heteroaryl group; $R^{122}$ is a hydrogen atom, an optionally substituted alkyl group, an optionally substituted aryl group, or an optionally substituted heteroaryl group; and $R^{123a}$ and $R^{123b}$ are each independently a hydrogen atom, an optionally substituted alkyl group, an optionally substituted aryl group, or an optionally substituted heteroaryl group, or $R^{123a}$ and $R^{123b}$ are linked together to form a cyclic amine.

$R^{102}$ is preferably a halogen atom; $OR^{113}$ in which $R^{113}$ is an optionally substituted alkyl group, an optionally substituted aryl group or an optionally substituted arylalkyl group; a group represented by the formula: - $N(R^{114a})$-$R^{114b}$ in which $R^{114a}$ and $R^{114b}$ are each independently a hydrogen atom or a $C_{1-3}$ alkyl group; a group represented by the formula: -C(=O)-$R^{116}$ in which $R^{116}$ is $OR^{122}$; an optionally substituted aryl group; an optionally substituted heteroaryl group; a nitro group; or a cyano group, preferably a halogen atom; $OR^{113}$ in which $R^{113}$ is an optionally substituted alkyl group; an optionally substituted aryl group; an optionally substituted heteroaryl group. More specifically, $R^{102}$ is more preferably a halogen atom; a methoxy group; an ethoxy group; or a phenyl group optionally substituted with a hydroxyl group, a 4-methylpiperazin-1-yl group, a (4-methylpiperazin-1-yl)methyl group, a 4-morpholin-4-yl group or a dimethylamino group.

In the formula (II), $R^{103}$ is a hydrogen atom, a $C_{1-6}$ alkyl group, a $C_{3-6}$ cycloalkyl group, or a $C_{3-6}$ cycloalkyl $C_{1-6}$ alkyl group.

$R^{103}$ is preferably a hydrogen atom, a $C_{1-3}$ alkyl group, $C_{3-6}$ cycloalkyl $C_{1-3}$ alkyl group. More specifically, $R^{103}$ is more preferably a hydrogen atom, a methyl group, or a cyclopropylmethyl group.

In the formula (II), when $R^{101}$ is present at the 8-position, $R^{101}$ and $R^{103}$ present at the 1-position are optionally linked together to form a five- to seven-membered ring hetero ring. The five- to seven-membered ring hetero ring is preferably pyrrolidinyl, piperidinyl or morpholinyl, more preferably pyrrolidinyl or morpholinyl.

Examples of preferred aspects of compounds of the formula (11) according to the present invention include, but are not limited to, the following aspects (I) to (III), and combinations of two or three of the aspects.

(I) A compound of the above formula (11) in which r is 0 or r is 1, $R^{101}$ is present at the 7-position or the 8-position, and $R^{101}$ represents a $C_{1-3}$ alkyl group optionally substituted with a halogen atom, or a hydroxy group, or r is 1, $R^{101}$ is present at the 8-position, and $R^{101}$ and $R^{103}$ are linked together to form a five- or six-membered ring hetero ring.
(II) A compound of the above formula (11) in which $R^{103}$ represents a hydrogen atom, a $C_{1-3}$ alkyl group or a $C_{3-6}$ cycloalkyl $C_{1-3}$ alkyl group, or r is 1, $R^{101}$ is present at the 8-position, and $R^{101}$ and $R^{103}$ are linked together to form a five- or six-membered ring hetero ring.
(III) A compound of the above formula (11) in which s is 0 or S is 1, and $R^{102}$ is a halogen atom; $OR^{113}$ in which $R^{113}$ is an optionally substituted $C_{1-3}$ alkyl group; or an optionally substituted aryl group.

The compounds of the above formula (11) or pharmacologically acceptable salts thereof (dihydroquinazolinone-based compounds) can be produced through the methods shown below. The method for producing a dihydroquinazolinone-based compound according to the present invention is not limited to the methods shown below, and a range of the compounds according to the present invention is not limited to compounds produced by the following production methods.

Examples of the compounds of the formula (11) according to the present invention include compounds of the above formula (11) in which each of $J^1$ and $J^2$ is N. Examples thereof include compounds in which r is 1, $R^{101}$ is present at the 8-position, and $R^{103}$ is a hydrogen atom; and compounds in which r is 1, $R^{101}$ is present at the 8-position, and $R^{101}$ and $R^{103}$ are linked together to form a five- or six-membered ring hetero ring.

The method for producing a dihydroquinazolinone-based compound according to the present invention can be carried out by combining a wide range of various kinds of synthesis methods known to persons skilled in the art, methods obtained by making a modification or the like to the synthesis methods if necessary, and the like while using starting raw materials, precursors, reagents and solvents which are commercially available or can be synthesized through methods known to persons skilled in the art. The optional substituents, reaction apparatus, and cooling and heating are as described for the methods for producing a spiro compound.

The dihydroquinazolinone-based compound according to the present invention can be prepared through, for example, the following production method 3.

(Production method 3)

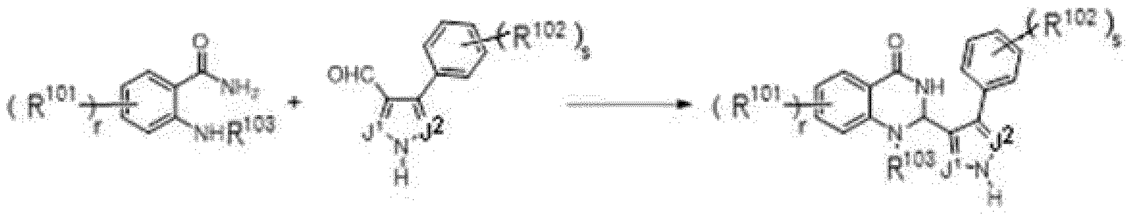

(12) (13) (11)

In the above formulae (12) and (13), $J^1$, $J^2$, r, $R^{101}$, s, $R^{102}$ and $R^{103}$ are each independently the same as $J^1$, $J^2$, r, $R^{101}$, s, $R^{102}$ and $R^{103}$, respectively, in the above formula (11).

The compound of the formula (12) (hereinafter, referred to as a "raw material (12)" and the compound of the formula (13) (hereinafter, referred to as a "raw material (13)" in the production method 3 can be obtained as commercially available reagents, or can be synthesized through known methods or methods based on the known methods.

In the production method 3, the raw material (12) and the raw material (13) are dissolved or suspended in an appropriate solvent, and reacted in the presence or non-presence of an acid catalyst to produce a dihydroquinazolinone-based compound according to the present invention (a compound of the formula (11) or a salt thereof; hereinafter, sometimes referred to as a "compound (11)").

In the production method 3, the raw material (12) and the raw material (13) are used generally at a molar ratio in the range of 1 : 1 to 3, preferably at a molar ratio in the range of 1 : 1 to 1.2.

Examples of the solvent to be used in the production method 3 include protic solvents such as water, methanol, ethanol, n-propanol, 2-propanol, n-butanol, 2-butanol and tert-butyl alcohol; hydrocarbon-based solvents such as petroleum ether, n-pentane, n-hexane, n-heptane, cyclohexane, benzene, toluene and xylene; halogen-based solvents such as carbon tetrachloride, dichloromethane, chloroform, 1,2-dichloroethane, chlorobenzene and trifluoromethylbenzene; ether-based solvents such as diethyl ether, diisopropyl ether, methyl tert-butyl ether, methyl cyclopentyl ether,

tetrahydrofuran, 2-methyltetrahydrofuran, 1,4-dioxane, 1,2-dimethoxyethane and diphenyl ether; ester-based solvents such as methyl acetate, ethyl acetate, n-propyl acetate, isopropyl acetate, n-butyl acetate, isobutyl acetate, tert-butyl acetate, benzyl acetate, methyl propionate, ethyl propionate, n-propyl propionate, isopropyl propionate, n-butyl propionate, isobutyl propionate and tert-butyl propionate; and aprotic polar solvents such as acetone, 2-butanone, methylisobutylketone, cyclohexanone, acetonitrile, propionitrile, N,N-dimethylformamide, N,N-dimethylacetamide, dimethyl sulfoxide and N-methyl-2-pyrrolidone. One of these solvents can be used alone, or two or more thereof can be mixed at an appropriate ratio and used. Of these, at least one of methanol, ethanol, n-propanol and N,N-dimethylformamide is preferably used as the solvent.

Examples of the acid to be used at the time of carrying out the reaction in the presence of the acid catalyst in the production method 3 include mineral acids such as hydrochloric acid, hydrobromic acid, hydroiodic acid, sulfuric acid, phosphoric acid and nitric acid; carboxylic acids such as formic acid, acetic acid, propionic acid and trifluoroacetic acid; and Lewis acids such as boron trifluoride-diethyl ether complexes, boron trichloride, boron tribromide, zinc chloride, stannic chloride, ferric chloride, aluminum chloride, titanium tetrachloride and zirconium tetrachloride. Of these, at least one of hydrochloric acid, formic acid, acetic acid and propionic acid is preferable, with acetic acid being more preferable, as the acid. The amount of the acid used is in the range of 0.01 to 20 equivalents, preferably in the range of 0.05 to 5 equivalents, more preferably in the range of 0.1 to 1 equivalents, or in the range of 0.01 to 20 equivalents, preferably in the range of 0.1 to 10 equivalents, more preferably 1 to 5 equivalents, based on the amount of the raw material (12) .

In the production method 3, the reaction temperature is in the range of 0 to 250°C, preferably 30 to 200°C, more preferably 60 to 160°C. In the production method 3, the reaction time is in the range of 1 minute to 2 days, preferably 5 minutes to 12 hours, more preferably 10 minutes to 6 hours.

In the production method 3, optional substituents with which $R^{102}$ is substituted may have intended substituents in the stage of the raw material (13). In this case, the resulting compound (11) has intended substituents. By introducing, modifying or converting precursor groups for intended substituents in the raw material (13) by combining a wide range of various kinds of synthesis methods known to persons skilled in the art, the compound (11) having intended substituents can be produced. These synthesis methods can be arbitrarily combined, and protection, deprotection and the like may be appropriately performed if necessary.

The raw material (13) according to the production method 3 can also be produced by, for example, methods shown in the following schemes 1 and 2.

(Scheme 1)

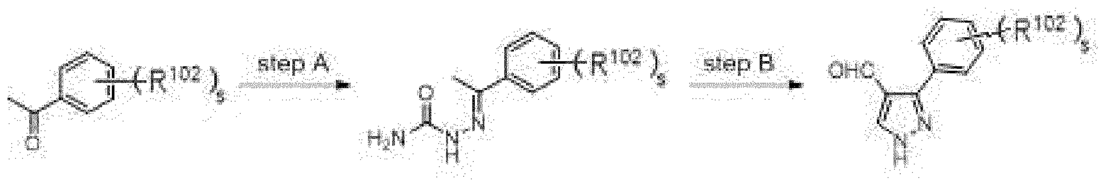


(14) (15) (13-1)

In the above formulae (14), (15) and (13-1), s and $R^{102}$ are each independently the same as s and $R^{102}$ in the above formula (11).

The compound of the formula (14) (hereinafter, referred to as a "raw material (14)") in the scheme 1 can be obtained as a commercially available reagent, or can be synthesized through a known method or a method based on the known method.

The compound of the formula (13-1) (hereinafter, referred to as a "raw material (13-1)") in the scheme 1 is a type of raw material (13). By using a known method such as the method described in Synthesis, 1998, 10, 1140., the raw material (13-1) can be produced from the compound of the formula (15) (compound (15)) which can be synthesized through a condensation reaction of the raw material (14) and semicarbazide hydrochloride. A series of all these synthesis methods are based on methodologies extensively described in general documents of organic chemistry, and the syntheses can be performed through the described methods themselves or modifications thereof.

(Scheme 2)

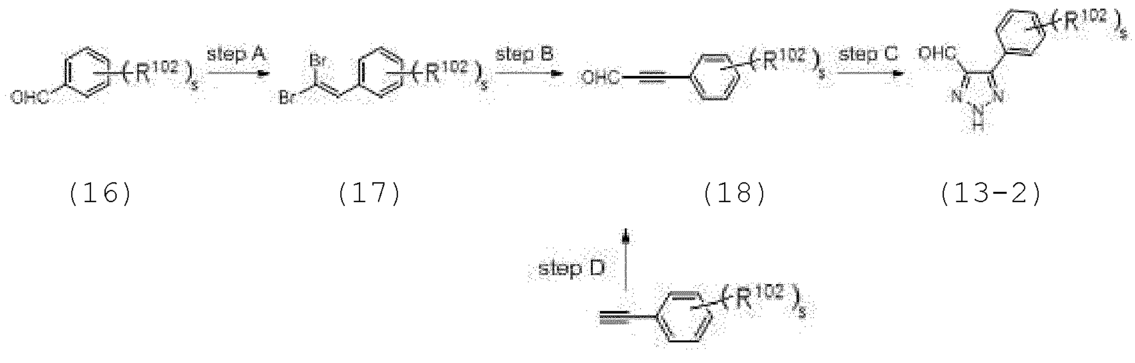


(16) (17) (18) (13-2)

(19)

In the above formulae (16), (17), (18), (19) and (13-2), s and $R^{102}$ are each independently the same as s and $R^{102}$ in the above formula (11).

The compound of the formula (16) (hereinafter, referred to as a "raw material (16)") and the compound of the formula (19) (hereinafter, referred to as a "raw material (19)") in the scheme 2 can be obtained as commercially available reagents, or can be synthesized through known method or methods based on the known methods.

The compound of the formula (13-2) (hereinafter, referred to as a "raw material (13-2)" in the scheme 2 is a type of raw material (13). The raw material (13-2) can be synthesized by adding sodium azide to the compound of the formula (18) (compound (18)) (step C: Tetrahedron Letters, 2001, 42, 9117.), and the compound (18) can be synthesized in two steps (step A and step B) from the raw material (16) or in one step (step D) from the raw material (19) (steps A and B: method described in Bioorganic & Medicinal Chemistry Letters, 2008, 184932., etc.; and step D: method described in Tetrahedron Letters, 2001, 42, 9117.). A series of all these synthesis methods are based on methodologies extensively described in general documents of organic chemistry, and the syntheses can be performed through the described methods themselves or modifications thereof.

The dihydroquinazolinone-based compound synthesized through the above-described method, the intermediate, the raw material and the like may be used in the next step in a state of a reaction solution or a crude product, or used after being isolated through a common purification method known to persons skilled in the art. Examples of the purification method associated with isolation include methods obtained by appropriately selecting or combining various types of chromatography (column or thin-layer and normal phase or reversed phase chromatography; gel permeation chromatography (GPC) and the like), distillation, sublimation, precipitation, crystallization and centrifugation.

As the nicotinamide-type tankyrase inhibitory compound according to the present invention, a compound such as G-631 can also be used.

Examples of the adenosine-type tankyrase inhibitory compound include the compounds disclosed in Patent Literature 3 and pharmacologically acceptable salts thereof (hereinafter, sometimes referred to as a "triazole derivative"), IWR-1, WIKI4 and JW55, and one of these compounds may be used alone, or two or more thereof may be used in combination.

(Triazole Derivative)

The adenosine-type tankyrase inhibitory compound according to the present invention is preferably at least one of a compound of the formula (I) or a compound of the formula (II) as disclosed in Patent Literature 3, an isomer thereof and a pharmacologically acceptable salt thereof (triazole derivatives). The triazole derivative is more preferably at least one of a compound of the formula (Ia), a compound of the formula (IIb), a compound of the formula (IIc) and a compound of the formula (IId) as disclosed in Patent Literature 3, an isomer thereof and a pharmacologically acceptable salt thereof. Examples of the triazole derivatives include compounds of the following formula which are also known as "G007-LK".

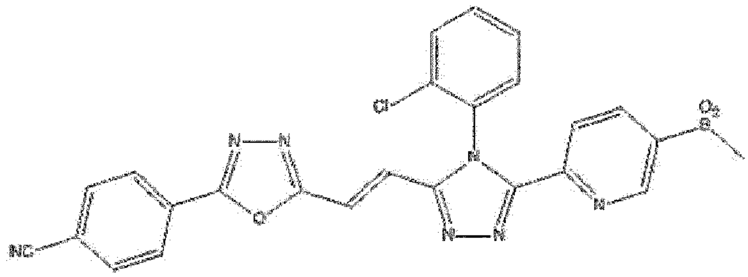

The triazole derivative can be produced by using the methods disclosed in Patent Literature 3 or by combining a wide range of various kinds of synthesis methods known to persons skilled in the art, methods obtained by making a modification or the like to the synthesis methods if necessary, and the like while using starting raw materials, precursors, reagents and solvents which are commercially available or can be synthesized through methods known to persons skilled in the art.

Examples of the dual-type tankyrase inhibitory compound include NVP-TNKS656 disclosed in Non Patent Literature 2.

The dual-type tankyrase inhibitory compound can be produced by combining a wide range of various kinds of synthesis methods known to persons skilled in the art, methods obtained by making a modification or the like to the synthesis methods if necessary, and the like while using starting raw materials, precursors, reagents and solvents which are commercially available or can be synthesized through methods known to persons skilled in the art.

<Microtubule inhibitory compound>

In the present invention, the microtubule inhibitory compound is a compound which has microtubule inhibitory activity and which is present as an active ingredient of a microtubule inhibitor that exhibits an antitumor effect by acting on microtubules playing important roles in maintenance of normal functions of cells, such as formation of a spindle in cell division, and arrangement of intracellular organelles and transportation of substances to the organs. The microtubule inhibitor is also referred to as a microtubule inhibitory drug, an anti-microtubule agent, a microtubule-targeted agent, a microtubule inhibition activator, a microtubule-directed agent, a microtubule-targeted and directed agent, a microtubule toxin, an agent to perturb the microtubule function, an ingredient which acts on microtubules, a microtubule-acting anticancer drug, or a medicament which inhibits microtubule functions. The microtubule inhibitors include microtubule polymerization inhibitors, microtubule depolymerization inhibitors, microtubule formation inhibitors, microtubule instability promoting agents, microtubule destabilizing agents, microtubule disrupting drugs, microtubule disintegrating drugs, microtubule dissociating agents, molecules which promote degradation of microtubules, and compounds which inhibit or disrupt polymerization of microtubules.

The microtubule inhibitory compound according to the present invention may be a microtubule depolymerization inhibitor which promotes polymerization of tubulins to stabilize and excessively form microtubules; a microtubule polymerization inhibitor which inhibits the polymerization; or a mixture thereof.

More specific examples of the microtubule inhibitory compound according to the present invention include paclitaxel, vinblastine, vincristine, vindesine, vinorelbine, docetaxel, cabazitaxel and eribulin, and one of these compounds may be used alone, or two or more thereof may be used in combination. The microtubule inhibitory compound according to the present invention may be a pharmacologically acceptable salt such as a sulfate, a tartrate, a mesylate, an acid adduct such as an acetone adduct, or a hydrate. As the microtubule inhibitory compound, a commercially available product such as Taxol (paclitaxel), Exal (vinblastine), Oncovin (vincristine sulfate), Fildesin (vindesine sulfate), Navelbin (vinorelbine tartrate), Taxotere (docetaxel hydrate), Onetaxotere (docetaxel hydrate), Jevtana (cabazitaxel acetone adduct) or Halaven (eribulin mesylate) may be appropriately used.

Of these, at least one selected from the group consisting of docetaxel, paclitaxel, vincristine and vinblastine is more preferable as the microtubule inhibitory compound according to the present invention because they tend to exhibit a particularly excellent cytocidal effect when combined with the tankyrase inhibitory compound.

<Anticancer Agent>

Examples of cancers which are targeted by the anticancer agent of the present invention include colorectal cancer, gastric cancer, esophagus cancer, colon cancer, liver cancer, pancreas cancer, breast cancer, lung cancer, gallbladder cancer, bile duct cancer, biliary duct cancer, rectal cancer, ovary cancer, uterus cancer, renal cancer, bladder cancer, prostate cancer, osteosarcoma, brain cancer, leukemia, myosarcoma, skin cancer, malignant melanoma, malignant lymphoma, tongue cancer, myeloma, thyroid cancer, cutaneous metastatic cancer and cutaneous melanoma.

Of these, lower digestive organ cancers are preferably targeted, and colorectal cancer is more preferably targeted.

The anticancer agent of the present invention is used in the form of a combination of the tankyrase inhibitory compound and the microtubule inhibitory compound. In the present invention, the combination may take any of a form in which a tankyrase inhibitor containing the tankyrase inhibitory compound as an active ingredient and a microtubule inhibitor containing the microtubule inhibitory compound as an active ingredient are simultaneously used or administered (simultaneous separate agents); a form in which the tankyrase inhibitor and the microtubule inhibitor are separately used or administered (intertemporal separate agents); and a form of a combined agent of the tankyrase inhibitor and the microtubule inhibitor (integrated agent).

Accordingly, the present invention provides an anticancer agent comprising a combination of the tankyrase inhibitor and the microtubule inhibitor; an anticancer agent which is used to be administered in combination with the microtubule inhibitor and which contains a tankyrase inhibitory compound as an active ingredient; an anticancer agent which is used to be administered in combination with the tankyrase inhibitor and which contains a microtubule inhibitory compound as an active ingredient; an anticancer agent in which a tankyrase inhibitory compound and a microtubule inhibitory compound are blended as active ingredients; and a kit including the tankyrase inhibitor and the microtubule inhibitor.

The tankyrase inhibitor, the microtubule inhibitor and the anticancer agent according to the present invention may each independently further contain other therapeutic agents. The other therapeutic agents may be simultaneously or intertemporally used in combination. Examples of the other therapeutic agents include other anticancer agents (antiproliferative agents, antineoplastic agents, DNA-damaging agents and combinations thereof), more specifically the above-described alkylating agents, antimetabolites, plant alkaloids, anticancer antibiotics, platinating agents; mitosis inhibitors; topoisomerase inhibitors; agents serving as both a plant alkaloid and a topoisomerase inhibitor; cell division inhibitors; growth factor function inhibitors such as EGFR antibodies; angiogenesis inhibition agonists such as VEGF antibodies and VEGFR antibodies; cancer cell metastasis suppression agonists such as metalloprotease inhibitors; antisense therapeutic drugs such as Ras antisense; and immunotherapeutic drugs with anti-PD-1 antibodies and T-cells. One of these therapeutic agents may be used alone, or two or more thereof may be used in combination.

The tankyrase inhibitor, the microtubule inhibitor and the anticancer agent according to the present invention may be administered through any of oral and parenteral administration routs such as routs of inhalation administration, nasal administration, ophthalmic administration, subcutaneous administration, intravenous administration, intramuscular administration, rectal administration and transdermal administration, and can be administered to humans or animals other than humans. Therefore, the tankyrase inhibitor, the microtubule inhibitor and the anticancer agent according to the present invention may each independently take an appropriate dosage form depending on an administration route.

Examples of the tankyrase inhibitor, the microtubule inhibitor and the anticancer agent according to the present invention each independently include oral agents such as tablets, pills, capsules, granules, powders, fine granules, troches, elixirs, suspensions, emulsions and syrups; solutions for external use such as inhalations, nasal solutions and ophthalmic solutions; injections such as intravenous injections and intramuscular injections; and parenteral agents such as rectal administration agents, suppositories, lotions, sprays, ointments, creams and patches.

The tankyrase inhibitor, the microtubule inhibitor and the anticancer agent according to the present invention may further contain, depending on the dosage form, excipients such as diluents, extenders, humectants, surfactants, disintegrants, binders, lubricating agents, dispersants, buffering agents, preservatives, solubilizing agents, antiseptics, correctives, soothing agents, stabilizers, lubricants and colorants which are commonly used in the area of pharmaceuticals. Production can be performed through conventional methods using these additives. Examples of the additives include lactose, fructose, glucose, starch, gelatin, magnesium carbonate, synthetic magnesium silicate, talc, magnesium stearate, methylcellulose, carboxymethylcellulose or salts thereof, gum arabic, olive oil, propylene glycol, polyethylene glycol, syrup, vaseline, glycerin, ethanol, citric acid, sodium chloride, sodium sulfite and sodium phosphate.

In the tankyrase inhibitor and the anticancer agent containing the tankyrase inhibitory compound according to the present invention, the content of the tankyrase inhibitory compound according to the present invention (the total content of tankyrase inhibitory compounds in the case of a mixture; the same applies hereinbelow) is appropriately adjusted depending on a dosage form of the agent, and therefore may vary, and the content of the tankyrase inhibitory compound is typically 0.01 to 70 mass%, preferably 0.05 to 50 mass%, in terms of a free form, based on the total mass of the tankyrase inhibitor or the anticancer agent. The dosage of the tankyrase inhibitory compound according to the present invention is appropriately adjusted depending on an individual case with consideration given to the dose regimen and the age, the body weight, the sex, the type of disease and the severity of a symptom of a patient, and the like, and therefore may vary, and the dosage of the tankyrase inhibitory compound is typically 0.1 to 2,000 mg, preferably 1 to 1,000 mg, per day per adult. This amount of the tankyrase inhibitory compound is administered once or in several divided doses a day.

In the microtubule inhibitor and the anticancer agent containing the microtubule inhibitory compound according to the present invention, the content of the microtubule inhibitory compound according to the present invention (the total content of microtubule inhibitory compounds in the case of a mixture; the same applies hereinbelow) is appropriately

adjusted depending on a dosage form of the agent, and therefore may vary, and the content of the microtubule inhibitory compound is typically 0.01 to 70 mass%, preferably 0.05 to 50 mass%, in terms of a free form, based on the total mass of the microtubule inhibitor or the anticancer agent. The dosage of the microtubule inhibitory compound according to the present invention is appropriately adjusted depending on an individual case with consideration given to the dose regimen and the age, the body weight, the sex, the type of disease and the severity of a symptom of a patient, and the like, and therefore may vary, and the dosage of the microtubule inhibitory compound is typically 0.1 to 2,000 mg, preferably 1 to 1,000 mg, per day per adult. This amount of the microtubule inhibitory compound is administered once or in several divided doses a day.

In the anticancer agent containing the tankyrase inhibitory compound and the microtubule inhibitory compound (integrated agent) according to the present invention, the mass ratio of the content of the tankyrase inhibitory compound and the content of the microtubule inhibitory compound (tankyrase inhibitory compound : microtubule inhibitory compound) is appropriately adjusted, and therefore may vary, and the mass ratio of the contents of the compounds is, for example, 1 : 20,000 to 20,000 : 1, preferably 1 : 1,000 to 1,000 to 1, more preferably 1 : 100 to 100 : 1 in terms of a free form.

When the tankyrase inhibitory compound and the microtubule inhibitory compound according to the present invention are used as separate agents, the mass ratio of the dosage of the tankyrase inhibitory compound and the dosage of the microtubule inhibitory compound (tankyrase inhibitory compound : microtubule inhibitory compound) is appropriately adjusted, and therefore may vary, and the mass ratio of the dosages of the compounds is, for example, 1 : 20,000 to 20,000 : 1, preferably 1 : 1,000 to 1,000 to 1, more preferably 1 : 100 to 100 : 1 in terms of a free form.

The integrated agent can be used in combination with the separate agents, and the integrated agent and/or the separate agents can be used in combination with single agents of the tankyrase inhibitor and/or the microtubule inhibitor. For example, an agent for intravenous injection prepared as the integrated agent may be infused first one or several times, followed by orally administrating only the tankyrase inhibitor for several days.

## Examples

Hereinafter, the present invention will be described in more detail on the basis of Examples, which should not be construed as limiting the scope of the present invention. Various applications, changes, modifications and the like can be made without departing from the scope of the present invention. Further, methods for producing compounds used in Examples will be described in Compound Examples, which are examples shown for explaining implementation of the present invention in detail, and are should not be construed as limiting the scope of the present invention. Various applications, changes, modifications and the like can be made without departing from the scope of the present invention.

Abbreviations used in Examples and Compound Examples below have the following meanings.

M: mol/L
$^1$H-NMR: proton nuclear magnetic resonance spectrum (270 MHz, 400 MHz or 500 MHz)
MS (ESI): mass spectrum (electrospray ionization method)
DMSO: dimethyl sulfoxide
Bzl and Bn: benzyl
MPM: 4-methoxyphenylmethyl, 4-methoxybenzyl
TBS: tert-butyldimethylsilyl
Cbz: benzyloxycarbonyl
Boc: tert-butyloxycarbonyl
Ts: para-toluenesulfonyl

(Compound Example 1)

2-(4,6-Difluoro-1-(2-hydroxyethyl)spiro[indolin-3,4'-piperidin]-1'-yl)-5,6,7,8-tetrahydroquinazolin-4(3H)-one

a) 1'-Benzyloxycarbonyl-1-tert-butyloxycarbonyl-4,6-difluorospiro[indolin-3,4'-piperidine]

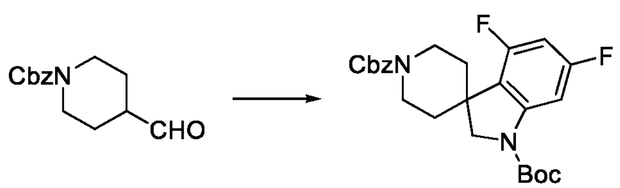

278.9 mg of 1-benzyloxycabonyl-4-formylpiperidine was dissolved in 5 ml of chloroform, 244.4 mg of 3,5-difluorophenylhydrazine hydrochloride and 0.26 ml of trifluoroacetic acid were added, the mixture was stirred under heating and reflux for 1 hour, 358.5 mg of sodium triacetoxyborohydride was then added, and the mixture was stirred at room temperature for 1 hour. To the reaction mixture was added a saturated sodium hydrogencarbonate aqueous solution, and the mixture was extracted with chloroform. The extract was dried over anhydrous magnesium sulfate, and concentrated under reduced pressure, the residue thus obtained was dissolved in 5 ml of chloroform, 295.4 mg of di-tert-butyl decarbonate and 165.4 mg of dimethylaminopyridine were added, and the mixture was stirred at room temperature for 1 hour. To the reaction mixture was added a saturated sodium hydrogencarbonate aqueous solution, and the mixture was extracted with chloroform. The extract was dried over anhydrous magnesium sulfate, and concentrated under reduced pressure, and the residue thus obtained was purified by silica gel chromatography (hexane/ethyl acetate = 80/20) to give 82.8 mg of the title compound. MS(ESI)m/z: 459 $[M+H]^+$

b) 2-(1-tert-Butyloxycarbonyl-4,6-difluorospiro[indolin-3,4'-piperidin]-1'-yl)-5,6,7,8-tetrahydroquinazolin-4(3H)-one

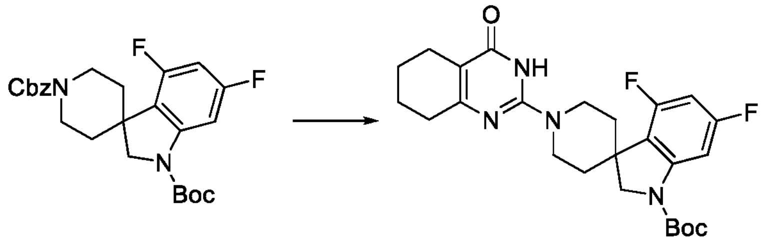

275.6 mg of 1'-benzyloxycarbonyl-1-tert-butyloxycarbonyl-4,6-difluorospiro[indolin-3,4'-piperidine] was dissolved in 3 ml of methanol, 64.0 mg of 10% palladium carbon and 189.5 mg of ammonium formate were added, and the mixture was heated and refluxed for 1 hour. The reaction solution was filtered through Celite, and the filtrate was concentrated under reduced pressure. The residue thus obtained was dissolved in 3 ml of acetonitrile, 105.7 mg of 1-amidinopyrazole hydrochloride and 0.251 ml of triethylamine were added, and the mixture was stirred at room temperature for 1 hour. The reaction mixture was concentrated, the residue thus obtained was dissolved in 5 ml of ethanol, 0.12 ml of ethyl 2-oxocyclohexanecarboxylate and 0.58 ml of a 21% sodium ethoxide ethanol solution were added, and the mixture was stirred under heating and reflux for 3 hours. To the reaction mixture was added an ammonium chloride aqueous solution, and the mixture was extracted with chloroform. The extract was dried over anhydrous magnesium sulfate, and concentrated under reduced pressure, and the residue thus obtained was purified by silica gel column chromatography (methanol/chloroform = 10/90) to give 48.5 mg of the title compound.
MS(ESI)m/z: 473 $[M+H]^+$

c) 2-(4,6-Difluorospiro[indolin-3,4'-piperidin]-1'-yl)-5,6,7,8-tetrahydroquinazolin-4(3H)-one

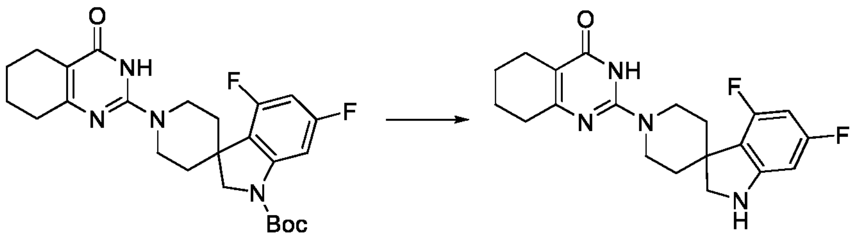

48.5 mg of 2-(1-tert-butyloxycarbonyl-4,6-difluorospiro[indolin-3,4'-piperidin]-1'-yl)-5,6,7,8-tetrahydroquinazolin-4(3H)-one was dissolved in 1 ml of chloroform, 1 ml of trifluoroacetic acid was added, and the mixture was stirred at room temperature for 1 hour. To the reaction mixture was added a saturated sodium hydrogencarbonate aqueous solution, and the mixture was extracted with chloroform. The extract was dried over anhydrous magnesium sulfate, and concentrated under reduced pressure, and the residue thus obtained was purified by silica gel column chromatography (chloroform/methanol = 90/10) to give 2.0 mg of the title compound.
$^1$H-NMR (400M Hz, $CDCl_3$) δ: 1.66-1.78 (m, 6H), 1.80-1.86 (m, 2H), 2.19-2.28 (m, 2H), 2.36-2.41 (m, 2H), 2.45-2.50

(m, 2H), 2.93-3.02 (m, 2H), 3.60 (s, 2H), 3.98 (br.s, 1H), 4.32-4.39 (m, 2H), 6.07-6.14 (m, 2H), 10.71 (br.s, 1H) .
MS (ESI) m/z: 373 $[M+H]^+$.

d) 2-(4,6-Difluoro-1-(2-hydroxyethyl)spiro[indolin-3,4'-piperidin]-1'-yl)-5,6,7,8-tetrahydroquinazolin-4(3H)-one

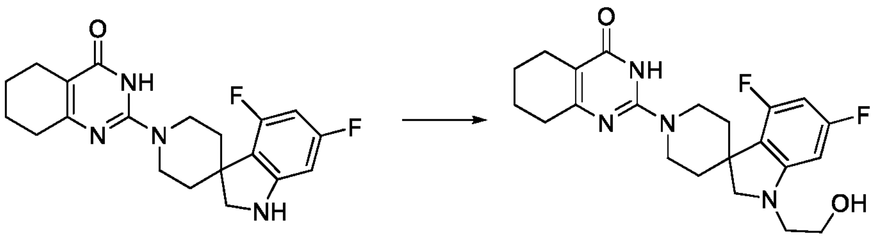

31.8 mg of 2-(4,6-difluorospiro[indolin-3,4'-piperidin]-1'-yl)-5,6,7,8-tetrahydroquinazolin-4(3H)-one was dissolved in chloroform, 15.4 mg of a glycolaldehyde dimer, 54.3 mg of sodium triacetoxyborohydride and acetic acid were added, and the mixture was stirred at room temperature for 14 hours. To the reaction mixture, a saturated sodium hydrogencarbonate aqueous solution was added, and the mixture was extracted with chloroform. The extract was dried over anhydrous magnesium sulfate, and concentrated under reduced pressure, and the residue thus obtained was purified by silica gel column chromatography (methanol/chloroform = 10/90) to give 12.4 mg of the title compound.
$^1$H-NMR (400M Hz, $CDCl_3$) δ: 1.64-1.83 (m, 6H), 2.20-2.30 (m, 2H), 2.33-2.38 (m, 2H), 2.45-2.50 (m, 2H), 2.91-3.00 (m, 2H), 3.30 (t, J = 5.4 Hz, 2H), 3.54 (s, 2H), 3.84 (t, J = 5.4 Hz, 2H), 4.42-4.49 (m, 2H), 6.00-6.09 (m, 2H), 11.69 (br.s, 1H).
MS (ESI) m/z: 417 $[M+H]^+$.

(Compound Example 5)

4-Fluoro-1-methyl-1'-(8-methyl-4-oxo-3,4,5,6,7,8-hexahydropyrido[2,3-d]pyrimidin-2-yl)-6-(4-methylpiperazin-1-yl)spiro[indolin-3,4'-piperidin]-2-one

a) tert-Butyl 2-cyano-2-(2,6-difluoro-4-methoxyphenyl)acetate

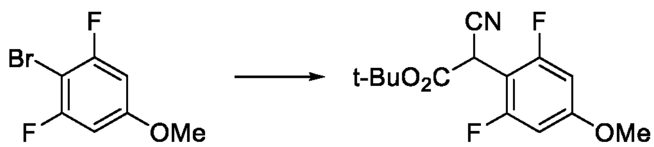

1.1151 g of 4-bromo-3,5-difluoroanisole, 1.2013 g of sodium tert-butoxide, 45.8 mg of tris(dibenzylideneacetone)dipalladium (0) and 29.0 mg of tri-tert-butylphosphine tetrafluoroborate were dissolved in 1,4-dioxane, 0.856 ml of tert-butyl cyanoacetate was added, and the mixture was stirred under microwave irradiation at 180°C for 30 minutes. Water was added to the reaction mixture, the mixture was filtered through Celite, and the filtrate was extracted with ethyl acetate. The extract was dried over anhydrous magnesium sulfate, and concentrated under reduced pressure, and the residue thus obtained was purified by silica gel column chromatography (ethyl acetate/hexane = 20/80) to give 1.2791 g of the title compound.
$^1$H-NMR (400M Hz, $CDCl_3$) δ: 1.50 (s, 9H), 3.81 (s, 3H), 4.92 (br.s, 1H), 6.50-6.55 (m, 2H).
MS (ESI) m/z: 284 $[M+H]^+$.

b) 2-(2,6-Difluoro-4-methoxyphenyl)acetonitrile

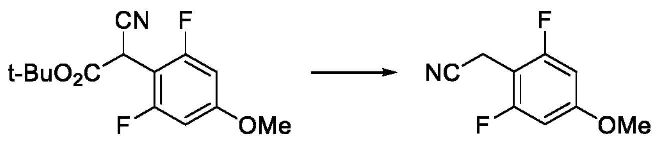

1.2791 g of tert-butyl 2-cyano-2-(2,6-difluoro-4-methoxyphenyl)acetate was dissolved in toluene, 85.9 mg of para-toluenesulfonic acid-hydrate was added, and the mixture was stirred under heating and reflux for 1 hour. To the reaction mixture was added a saturated sodium hydrogencarbonate aqueous solution, and the mixture was extracted with ethyl acetate. The extract was dried over anhydrous magnesium sulfate, and concentrated under reduced pressure, and the residue thus obtained was purified by silica gel column chromatography (ethyl acetate/hexane = 20/80) to give 698.8 mg of the title compound.
$^1$H-NMR (400M Hz, $CDCl_3$) δ: 3.64 (s, 2H), 3.80 (s, 3H), 6.48-6.54 (m, 2H).
MS (ESI) m/z: 184 $[M+H]^+$.

c) 1-(tert-Butyloxycarbonyl)-4-(2,6-difluoro-4-methoxyphenyl)piperidine-4-carbonitrile

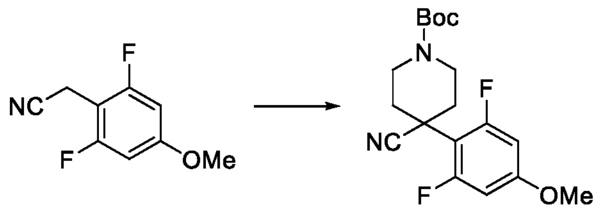

698.8 mg of 2-(2,6-difluoro-4-methoxyphenyl)acetonitrile was dissolved in N,N-dimethylformamide, 1.1086 g of N-(tert-butyloxycarbonyl)-N,N-bis(2-chloroethyl)amine and 457.8 mg of sodium hydride were added, and the mixture was stirred at 80°C for 3 hours. To the reaction mixture was added a saturated ammonium chloride aqueous solution, and the mixture was extracted with ethyl acetate. The extract was dried over anhydrous magnesium sulfate, and concentrated under reduced pressure, and the residue thus obtained was purified by silica gel chromatography (ethyl acetate/hexane = 20/80) to give 881.4 mg of the title compound.
$^1$H-NMR (400M Hz, $CDCl_3$) δ: 1.48 (s, 9H), 2.11-2.20 (m, 2H), 2.30-2.36 (m, 2H), 3.17-3.29 (m, 2H), 3.79 (s, 3H), 4.10-4.27 (m, 2H), 6.46-6.51 (m, 2H).
MS (ESI) m/z: 353 $[M+H]^+$.

d) 1-(tert-Butyloxycarbonyl)-4-(2,6-difluoro-4-methoxyphenyl)piperidin-4-carboxamide

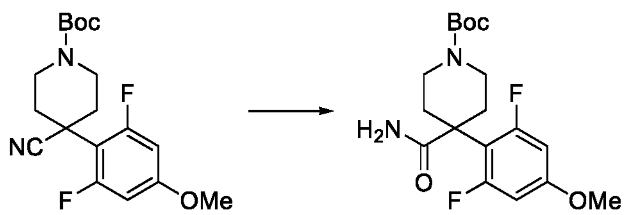

2.0886 g of 1-(tert-butyloxycarbonyl)-4-(2,6-difluoro-4-methoxyphenyl)piperidine-4-carbonitrile was dissolved in dimethyl sulfoxide, 2.5 ml of 5 N sodium hydroxide and 2.5 ml of a 30% hydrogen peroxide aqueous solution were added, and the mixture was stirred at 80°C for 14 hours. To the reaction mixture was added a saturated ammonium chloride aqueous solution, and the mixture was extracted with ethyl acetate. The extract was dried over anhydrous magnesium sulfate, and concentrated under reduced pressure, and the residue thus obtained was purified by silica gel column chromatography (ethyl acetate/hexane = 20/80) to give 2.1733 g of the title compound.
MS(ESI)m/z: 371 $[M+H]^+$

e) 1'-(tert-Butyloxycarbonyl)-4-fluoro-6-methoxy-1-methylspiro[indolin-3,4'-piperidin]-2-one

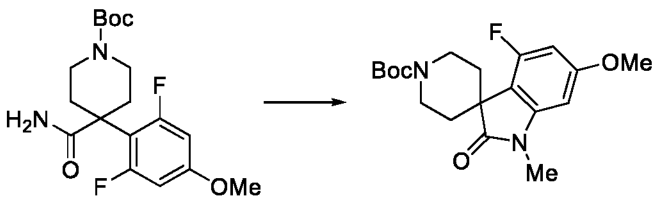

2.1733 g of 1-(tert-butyloxycarbonyl)-4-(2,6-difluoro-4-methoxyphenyl)piperidin-4-carboxamide was dissolved in N,N-dimethylformamide, 139.9 mg of lithium hydride was added, the mixture was stirred at 120°C for 14 hours, 1.1 ml of iodomethane was then added, and the mixture was stirred for 1 hour. To the reaction mixture was added a saturated ammonium chloride aqueous solution, and the mixture was extracted with ethyl acetate. The extract was dried over anhydrous magnesium sulfate, and concentrated under reduced pressure, and the residue thus obtained was purified by silica gel column chromatography (ethyl acetate/hexane = 20/80) to give 1.8156 g of the title compound.
$^1$H-NMR (400M Hz, $CDCl_3$) δ: 1.50 (s, 9H), 1.69-1.76 (m, 2H), 2.05-2.14 (m, 2H), 3.16 (s, 3H), 3.66-3.98 (m, 7H), 6.22-6.28 (m, 2H).
MS (ESI) m/z: 365 $[M+H]^+$.

f) 1'-(tert-Butyloxycarbonyl)-4-fluoro-6-hydroxy-1-methylspiro[indolin-3,4'-piperidin]-2-one

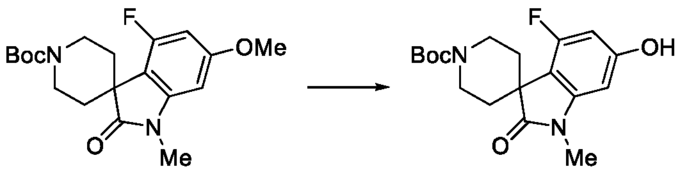

27.7 mg of 1'-(tert-butyloxycarbonyl)-4-fluoro-6-methoxy-1-methylspiro[indolin-3,4'-piperidin]-2-one was dissolved in chloroform, 1.5 ml of boron tribromide (17% dichloromethane solution, about 1 mol/l) was added, and the mixture was stirred at room temperature for 14 hours. To the reaction mixture were added methanol, a 5 N sodium hydroxide aqueous solution and 0.026 ml of di-tert-butyl decarbonate, and the mixture was stirred for 1 hour. To the reaction mixture was added a 1 N hydrochloric acid aqueous solution, and the mixture was extracted with chloroform. The extract was dried over anhydrous magnesium sulfate, and concentrated under reduced pressure, and the residue thus obtained was purified by silica gel column chromatography (ethyl acetate/hexane = 50/50) to give 26.6 mg of the title compound. $^1$H-NMR (400M Hz, $CDCl_3$) δ: 1.51 (s, 9H), 1.70-1.77 (m, 2H), 2.03-2.12 (m, 2H), 3.68-3.94 (m, 4H), 6.23-6.28 (m, 2H), 8.05 (br.s, 1H).
MS (ESI) m/z: 351 $[M+H]^+$.

g) 1'-(tert-Butyloxycarbonyl)-4-fluoro-1-methyl-2-oxospiro[indolin-3,4'-piperidin]-6-yl trifluoromethanesulfonate

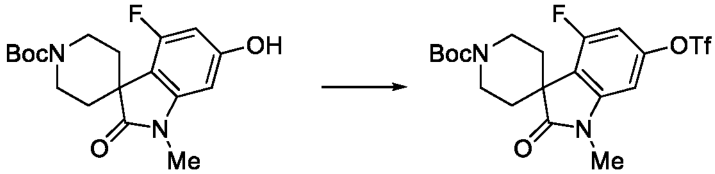

175.2 mg of 1'-(tert-butyloxycarbonyl)-4-fluoro-6-hydroxy-1-methylspiro[indolin-3,4'-piperidin]-2-one was dissolved in chloroform, 0.21 ml of triethylamine and 0.12 ml of trifluoromethanesulfonic anhydride were added, and the mixture was stirred at room temperature for 1 hour. To the reaction mixture was added a saturated sodium hydroxide

aqueous solution, and the mixture was extracted with chloroform. The extract was dried over anhydrous magnesium sulfate, and concentrated under reduced pressure, and the residue thus obtained was purified by silica gel column chromatography (ethyl acetate/hexane = 20/80) to give 159.2 mg of the title compound.
MS(ESI)m/z: 483 [M+H]+

h) 4-Fluoro-1-methyl-1'-(8-methyl-4-oxo-3,4,5,6,7,8-hexahydropyrido[2,3-d]pyrimidin-2-yl)-6-(4-methylpiperazin-1-yl)spiro[indolin-3,4'-piperidin]-2-one

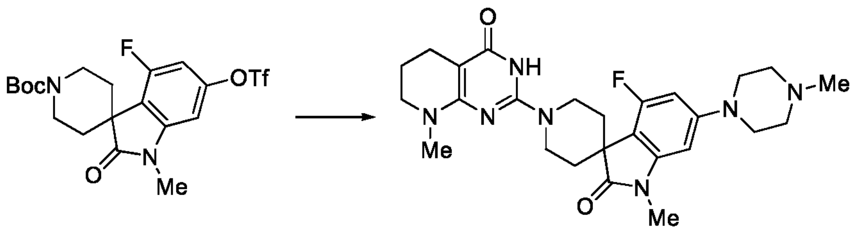

48.2 mg of 1'-(tert-butyloxycarbonyl)-4-fluoro-1-methyl-2-oxospiro[indolin-3,4'-piperidin]-6-yl trifluoromethanesulfonate, 48.9 mg of cesium carbonate, 2.2 mg of palladium acetate and 12.5 mg of rac-2,2'-bis(diphenylphosphino)-1,1'-binaphthyl were dissolved in toluene, 0.013 ml of N-methylpiperazine was added, and the mixture was stirred under heating and reflux for 14 hours. The reaction mixture was filtered through Celite, the filtrate was concentrated under reduced pressure, and the residue thus obtained was purified by silica gel column chromatography (methanol/chloroform = 10/90). The product thus obtained was dissolved in chloroform, trifluoroacetic acid was added, and the mixture was stirred at room temperature for 1 hour. To the reaction mixture was added a saturated sodium hydroxide aqueous solution, and the mixture was extracted with chloroform. The extract was dried over anhydrous magnesium sulfate, and concentrated under reduced pressure, the residue thus obtained was dissolved in ethanol, 24.0 mg of 2-chloro-8-methyl-5,6,7,8-tetrahydropyrido[2,3-d]pyrimidin-4(3H)-one and 0.017 ml of triethylamine were added, and the mixture was stirred under microwave irradiation at 150°C for 30 minutes. To the reaction mixture was added a saturated sodium hydrogencarbonate aqueous solution, and the mixture was extracted with chloroform. The extract was dried over anhydrous magnesium sulfate, and concentrated under reduced pressure, and the residue thus obtained was purified by silica gel column chromatography (methanol/chloroform = 10/90) to give 31.5 mg of the title compound.
$^1$H-NMR (400M Hz, $CDCl_3$) δ: 1.80-1.87 (m, 4H), 2.11-2.20 (m, 2H), 2.36 (s, 3H), 2.44-2.49 (m, 2H), 2.55-2.60 (m, 4H), 3.07 (s, 3H), 3.17 (s, 3H), 3.21-3.27 (m, 6H), 3.89-3.98 (m, 2H), 4.10-4.17 (m, 2H), 6.19-6.25 (m, 2H), 10.32 (br.s, 1H).
MS (ESI) m/z: 496 [M+H]+.

(Compound Example 19)

6-(cis-2,6-Dimethylmorpholin-4-yl)-4-fluoro-1-methyl-1'-(8-methyl-4-oxo-3,4,5,6,7,8-hexahydropyrido[2,3-d]pyrimidin-2-yl)spiro[indolin-3,4'-piperidin]-2(1H)-one

a) 1'-(tert-Butyloxycarbonyl)-6-(cis-2,6-dimethylmorpholino)-4-fluoro-1-methylspiro[indolin-3,4'-piperidin]-2-one

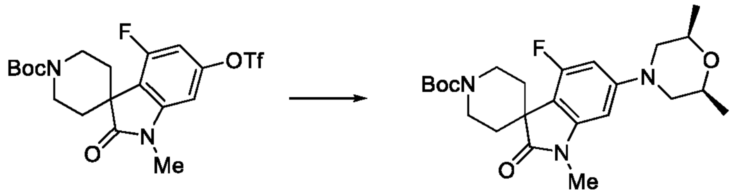

48.2 mg of 1'-(tert-butyloxycarbonyl)-4-fluoro-1-methyl-2-oxospiro[indolin-3,4'-piperidin]-6-yl trifluoromethanesulfonate, 48.9 mg of cesium carbonate, 2.2 mg of palladium acetate and 6.2 mg of rac-2,2'-bis(diphenylphosphino)-1,1'-binaphthyl were dissolved in toluene, 0.015 ml of cis-2,6-dimethylmorpholine was added, and the mixture was stirred under heating and reflux for 14 hours. The reaction mixture was filtered through Celite, the filtrate was concentrated

under reduced pressure, and the residue thus obtained was purified by silica gel column chromatography (ethyl acetate/hexyl = 50/50) to give 18.2 mg of the title compound.
$^1$H-NMR (400M Hz, $CHCl_3$) δ: 1.27 (d, J = 6.2 Hz, 6H), 1.49 (s, 9H), 1.70-1.77 (m, 2H), 2.01-2.11 (m, 2H), 2.41-2.49 (m, 2H), 3.17 (s, 3H), 3.40-3.45 (m, 2H), 3.68-3.94 (m, 6H), 6.15-6.23 (m, 2H).
MS (ESI) m/z: 448 $[M+H]^+$.

b) 6-(cis-2,6-Dimethylmorpholin-4-yl)-4-fluoro-1-methyl-1'-(8-methyl-4-oxo-3,4,5,6,7,8-hexahydropyrido[2,3-d]pyrimidin-2-yl)spiro[indolin-3,4'-piperidin]-2(1H)-one

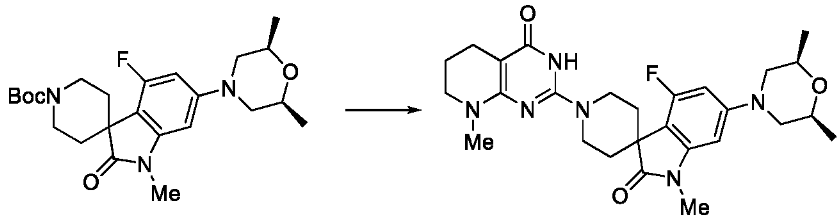

29.9 mg of 1'-(tert-butyloxycarbonyl)-6-(cis-2,6-dimethylmorpholino)-4-fluoro-1-methylspiro[indolin-3,4'-piperidin]-2-one was dissolved in 1 ml of chloroform, 1 ml of trifluoroacetic acid was added, and the mixture was stirred at room temperature for 1 hour. To the reaction mixture was added a saturated sodium hydroxide aqueous solution, and the mixture was extracted with chloroform. The extract was dried over anhydrous magnesium sulfate, and concentrated under reduced pressure, the residue thus obtained was dissolved in ethanol, 16.0 mg of 2-chloro-8-methyl-5,6,7,8-tetrahydropyrido[2,3-d]pyrimidin-4(3H)-one and 0.028 ml of triethylamine were added, and the mixture was stirred under microwave irradiation at 150°C for 30 minutes. To the reaction mixture was added a saturated sodium hydrogencarbonate aqueous solution, and the mixture was extracted with chloroform. The extract was dried over anhydrous magnesium sulfate, and concentrated under reduced pressure, and the residue thus obtained was purified by silica gel column chromatography (methanol/chloroform = 10/90) to give 22.6 mg of title compound.
$^1$H-NMR (400M Hz, $CDCl_3$) δ: 1.27 (d, J = 6.2 Hz, 6H), 1.79-1.87 (m, 4H), 2.11-2.20 (m, 2H), 2.42-2.49 (m, 4H), 3.07 (s, 3H), 3.19 (s, 3H), 3.23-3.27 (m, 2H), 3.40-3.45 (m, 2H), 3.73-3.82 (m, 2H), 3.89-3.97 (m, 2H), 4.07-4.15 (m, 2H), 6.16-6.23 (m, 2H).
MS (ESI) m/z: 511 $[M+H]^+$.

The compounds of Compound Examples 2 to 4, 6 to 18 and 20 to 24 were prepared using the methods of Compound Examples 1, 5 and 19 and methods based thereon, and methods disclosed in literatures and methods based thereon. Tables 1 to 7 below show the compounds of Compound Examples 1 to 24. By X-ray diffraction described below, the compound prepared in Compound Example 1 was confirmed to be a nicotinamide-type compound which binds to the nicotinamide binding pocket of tankyrase.

<X-Ray Diffraction>

X-ray diffraction data of a tankyrase-2 crystal soaked with the compound prepared in Compound Example 1 was measured with BL26B2 Beam Line in a large synchrotron radiation facility Spring-8 (Sayo-gun, Hyogo). The wavelength of the X-ray was 1.000 Å, and MX-225 (Rayonix, LLC) was used as a detector. The diffraction data was treated with XDS (Kabsch, 2010) and the CCP4 program (Winn et al., 2011).

[Table 1]

| Compound Example | Compound name | Structure | 1H NMR δ ppm | Solvent | ESI MS m/z |
|---|---|---|---|---|---|
| 1 | 2-[4,6-Difluoro-1-(2-hydroxyethyl)spiro [indolin-3,4'-piperidin]-1'-yl]-5,6,7,8-tetrahydroquinazolin-4(3H)-one | 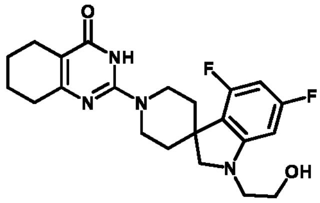 | 1.64-1.83 (m, 6 H), 2.20-2.30 (m, 2 H), 2.33-2.38 (m, 2 H), 2.45-2.50 (m, 2 H), 2.91-3.00 (m, 2 H), 3.30 (t, J = 5.4 Hz, 2 H), 3.54 (s, 2 H), 3.84 (t, J = 5.4 Hz, 2 H), 4.42-4.49 (m, 2 H), 6.00-6.09 (m, 2 H), 11.69 (br. s, 1H). | CDCl3 | 417 [M+H] + |
| 2 | 4-Fluoro-1-(2-hydroxyethyl)-6-methoxy-1'-(8-methyl-4-oxo-3,4,5,6,7,8-hexahydropyrido[2,3-d]pyrimidin-2-yl)spiro [indolin-3,4'-piperidin]-2-one | 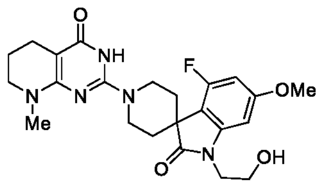 | 1.81-1.88 (m, 4 H), 2.17-2.25 (m, 2 H), 2.47-2.51 (m, 2 H), 3.08 (s, 3 H), 3.24-3.29 (m, 2 H), 3.80 (s, 3 H), 3.83-3.93 (m, 6 H), 4.06-4.13 (m, 2 H), 6.26 (dd, *J* = 11.6, 2.1, 1 H), 6.34 (d, J = 2.1, 1H). | CDCl3 | 458 [M+H] + |
| 3 | 2-(4,6-Difluoro-1'-(8-methyl-4-oxo-3,4,5,6,7,8-hexahydropyrido[2,3-d]pyrimidin-2-yl)-2-oxospiro[indolin-3,4'-piperidin]-1-yl) acetonitrile | 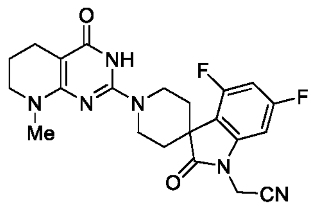 | 1.80-1.87 (m, 4 H), 2.26-2.34 (m, 2 H), 2.43-2.47 (m, 2 H), 3.07 (s, 3 H), 3.23-3.27 (m, 2 H), 3.76-3.85 (m, 2 H), 4.30-4.37 (m, 2 H), 4.63 (s, 2 H), 6.56-6.62 (m, 2 H), 11.11 (br.s, 1 H). | CDCl3 | 441 [M+H] + |

(continued)

| Compound Example | Compound name | Structure | 1H NMR δ ppm | Solvent | ESI MS m/z |
|---|---|---|---|---|---|
| 4 | 2-[4-Fluoro-6-methoxy-1'-(8-methyl-4-oxo-3,4,5,6,7,8-hexahydropyrido[2,3-d]pyrimidin-2-yl)-2-oxospiro[indolin-3,4'-piperidin]-1-yl] acetonitrile | 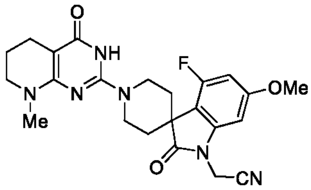 | 1.80-1.87 (m, 4 H), 2.21-2.30 (m, 2 H), 2.43-2.48 (m, 2 H), 3.07 (s, 3 H), 3.23-3.27 (m, 2 H), 3.78-3.87 (m, 5 H), 4.23-4.30 (m, 2 H), 4.61 (s, 2 H), 6.35 (dd, J = 11.6, 2.0 Hz, 1 H), 6.39 (d, J = 2.1 Hz, 1 H), 10.84 (br.s, 1 H). | CDCl3 | 453 [M+H] + |

[Table 2]

| Compound Example | Compound name | Structure | 1H NMR δ ppm | Solvent | ESI MS m/z |
|---|---|---|---|---|---|
| 5 | 4-Fluoro-1-methyl-1'-(8-methyl-4-oxo-3,4,5,6,7,8-hexahydropyrido [2,3-d]pyrimidin-2-yl)-6-(4-methylpiperazin-1-yl)spiro[indolin-3,4'-piperidin]-2-one | 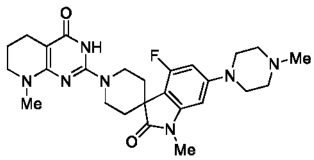 | 1.80-1.87 (m, 4 H), 2.11-2.20 (m, 2 H), 2.36 (s, 3 H), 2.44-2.49 (m, 2 H), 2.55-2.60 (m, 4 H), 3.07 (s, 3 H), 3.17 (s, 3 H), 3.21-3.27 (m, 6 H), 3.89-3.98 (m, 2 H), 4.10-4.17 (m, 2 H), 6.19-6.25 (m, 2 H), 10.32 (br. s, 1H). | CDCl3 | 496 [M+H] + |
| 6 | 4-Fluoro-1-methyl-1'-(8-methyl-4-oxo-3,4,5,6,7,8-hexahydropyrido [2,3-d]pyrimidin-2-yl)-6-(oxetan-3-ylmethoxy)spiro [indolin-3,4'-piperidin]-2-one | 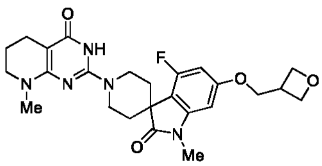 | 1.79-1.86 (m, 4 H), 2.15-2.24 (m, 2 H), 2.43-2.48 (m, 2 H), 3.07 (s, 3 H), 3.17 (s, 3 H), 3.22-3.27 (m, 2 H), 3.40-3.47 (m, 1 H), 3.88-3.97 (m, 2 H), 4.17-4.24 (m, 4 H), 4.54-4.58 (m, 2 H), 4.87-4.92 (m, 2 H), 6.25-6.30 (m, 2 H), 10.76 (br. s, 1 H). | CDCl3 | 484 [M+H] + |

(continued)

| Compound Example | Compound name | Structure | 1H NMR δ ppm | Solvent | ESI MS m/z |
|---|---|---|---|---|---|
| 7 | 4-Fluoro-1-methyl-1'-(8-methyl-4-oxo-3,4,5,6,7,8-hexahydropyrido [3,3-d]pyrimidin-2-yl)-6-[(tetrahydro-2 H-pyran-4-yl)oxy] spiro[indolin-3,4'-piperidin]-2-one | 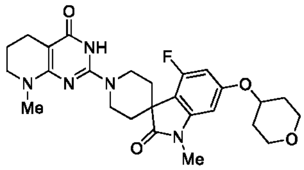 | 1.75-1.86 (m, 6 H), 1.99-2.07 (m, 2 H), 2.15-2.24 (m, 2 H), 2.43-2.48 (m, 2 H), 3.07 (s, 3 H), 3.17 (s, 3 H), 3.22-3.27 (m, 2 H), 3.56-3.63 (m, 2 H), 3.88-4.01 (m, 4 H), 4.16-4.23 (m, 2 H), 4.43-4.49 (m, 1 H), 6.24-6.28 (m, 2 H), 10.63 (br. s, 1 H). | CDCl3 | 498 [M+H] + |

[Table 3]

| Compound Example | Compound name | Structure | 1H NMR δ ppm | Solvent | ESI MS m/z |
|---|---|---|---|---|---|
| 8 | 4-Fluoro-1'-(8-methyl-4-oxo-3,4,5,6,7,8-hexahydropyrido[2,3-d]pyrimidin-2-yl)-6-[2-(4-methylpiparazin-1-yl)ethoxy]-1-(2,2,2-trifluoroethyl)spiro[indolin-3,4'-piperidin]-2(1H)-one | 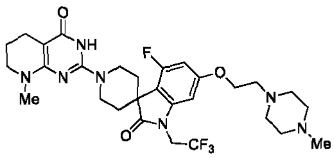 | 1.78-1.87 (m, 4 H), 2.21-2.32 (m, 5 H), 2.41-2.68 (m, 10 H), 2.81 (t, J=5.8 Hz, 2 H), 3.06 (s, 3 H), 3.22-3.27 (m, 2 H), 3.80-3.89 (m, 2 H), 4.08 (t, J=5.8 Hz, 2 H), 4.22-4.33 (m, 4 H), 6.29-6.36 (m, 2 H). | CDCl3 | 608 [M+H] + |
| 9 | {[4-Fluoro-1'-(8-methyl-4-oxo-3,4,5,6,7,8-hexahydropyrido[2,3-d]pyrimidin-2-yl)-2-oxo-1-(2,2,2-trifluoroethyl)spiro[indolin-3,4'-piperidin]-6-yl]oxy} acetic acid | 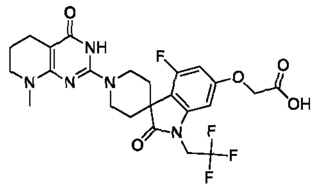 | 1.71-1.81 (m, 4 H), 1.98-2.08 (m, 2 H), 2.29-2.35 (m, 2 H), 3.00 (s, 3 H), 3.20-3.25 (m, 2 H), 3.61-3.71 (m, 2 H), 4.11-4,19 (m, 2 H), 4.74 (s, 2 H), 6.53 (dd, J=12.0, 2.0 Hz, 1 H), 6.83-6.86 (m, 1 H). | CDCl3 | 540 [M+H] + |

(continued)

| Compound Example | Compound name | Structure | 1H NMR δ ppm | Solvent | ESI MS m/z |
|---|---|---|---|---|---|
| 10 | 6-[(1 H-Tetrazol-5-yl)methoxy]-4-fluoro-1'-(8-methyl-4-oxo-3,4,5,6,7,8-hexahydropyrido[2,3-d]pyrimidin-2-yl)-1-(2,2,2-trifluoroethyl)spiro[indolin-3,4'-piperidin]-2( 1 H)-one | 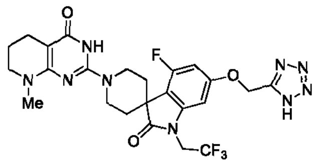 | 1.75-1.82 (m, 2 H), 2.00-2.10 (m, 2 H), 2.34-2.39 (m, 2 H), 3.04 (s, 3 H), 3.25-3.30 (m, 2 H), 3.65-3.74 (m, 2 H), 4.13-4.21 (m, 2 H), 4.64 (q, J=9.4 Hz, 2 H), 5.51-5.55 (m, 2 H), 6.73 (dd, J=11.9, 2.0 Hz, 2 H), 6.92-6.94 (m, 1 H). | DMSO-d6 | 564 [M+H]+ |

[Table 4]

| Compound Example | Compound name | Structure | 1H NMR δ ppm | Solvent | ESI MS m/z |
|---|---|---|---|---|---|
| 11 | 1-[4-Fluoro-1-methyl-1'-(8-methyl-4-oxo-3,4,5,6,7,8-hexahydropyrido[2,3-d]pyrimidin-2-yl)-2-oxospiro[indolin-3,4'-piperidin]-6-yl]piperidin-4-carboxylic acid | 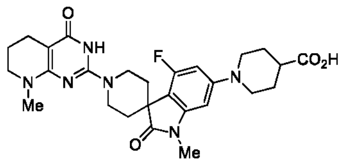 | 1.67-1.93 (m, 8 H), 2.02-2.12 (m, 2 H), 2.48-2.54 (m, 2 H), 2.55-2.64 (m, 1 H), 3.00-3.08 (m, 2 H), 3.09 (s, 3 H), 3.16 (s, 3 H), 3.28-3.33 (m, 2 H), 3.62-3.70 (m, 2 H), 3.92-4.01 (m, 2 H), 4.09-4.18 (m, 2 H), 6.11-6.18 (m, 2 H). | CDCl3 | 525 [M+H]+ |
| 12 | 1-[4-Fluoro-1-methyl-1'-(8-methyl-4-oxo-3,4,5,6,7,8-hexahydropyrido[2,3-d]pyrimidin-2-yl)-2-oxospiro[indolin-3,4'-piperidin]-6-yl]azetidin-3-carboxylic acid | 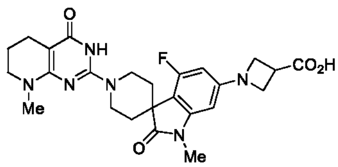 | 1.76-1.90 (m, 4 H), 2.01-2.10 (m, 2 H), 2.48-2.53 (m, 2 H), 3.10 (s, 3 H), 3.17 (s, 3 H), 3.29-3.34 (m, 2 H), 3.59-3.68 (m, 1 H), 3.87-4.02 (m, 4 H), 4.07-4.16 (m, 4 H), 5.70-7.75 (m, 2 H). | CDCl3 | 497 [M+H]+ |

(continued)

| Compound Example | Compound name | Structure | 1H NMR δ ppm | Solvent | ESI MS m/z |
|---|---|---|---|---|---|
| 13 | 4-Chloro-1 '-(4-hydroxy-8-methyl-6,7-dihydro-5H-pyrido[2,3-d] pyrimidin-2-yl)-1-methyl-7-(oxolan-3-yloxy)spiro[indol-3,4'-piperidin]2-one | 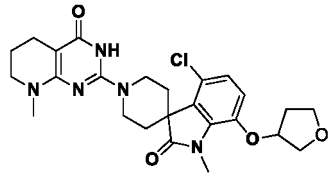 | 1.55 -1.61 (m, 2 H), 1.81 - 1.90 (m, 2 H), 2.15-2.29 (m, 2 H), 2.49 (t, J=6.1 Hz, 2 H), 2.68 - 2.80 (m, 2 H), 3.08 (s, 3 H), 3.23 - 3.30 (m, 2 H), 3.46 (s, 3 H), 3.82 - 4.04 (m, 6 H), 4.17 - 4.29 (m, 2 H), 4.94 - 4.99 (m, 1 H), 6.71 (d, J=8.9 Hz, 1 H), 6.89 (d, J=8.9 Hz, 1H). | CDCl3 | 500 [M+H] + |
| 14 | 4-Chloro-1'-(4-hydroxy-8-methyl-6,7-dihydro-5H-pyrido[2,3-d] pyrimidin-2-yl)-1-(2-hydroxy-2-methoxypropyl [indol-3,4'-piperidin]-2-one | 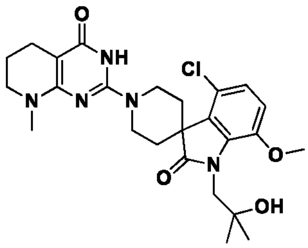 | 1.23 (s, 6 H), 1.55 - 1.66 (m, 2 H), 1.74 -1.90 (m, 2 H), 2.49 (t, J=6.3 Hz, 2 H), 2.73 - 2.87 (m, 2 H), 3.08 (s, 3 H), 3.22 - 3.36 (m, 2 H), 3.81 - 3.92 (m, 5 H), 4.13 (s, 2 H), 4.23 - 4.35 (m, 2 H), 6.82 (d, J=8.9 Hz, 1 H), 6.95 (d, J=8.9 Hz, 1 H). | CDCl3 | 502 [M+H] + |

[Table 5]

| Compoun d Example | Compound name | Structure | 1H NMR δ ppm | Solven t | ESI MS m/z |
|---|---|---|---|---|---|
| 15 | 4-Fluoro-1'-(4-hydroxy-8-methyl-6,7-dihydro-5H-pyrido[2,3-d] pyrimidin-2-yl)-1-(2-hydroxy-2-methylpropyl)spiro[indol-3,4'-piperidin]-2-one | 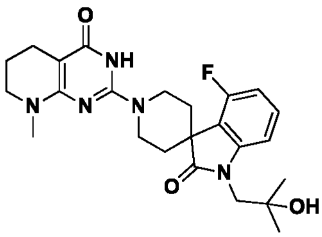 | 1.29 (s, 6 H), 1.76 - 1.91 (m, 4 H), 2.25 - 2.42 (m, 2 H), 2.47 (br t, J=6.1 Hz, 2 H), 3.08 (s, 3 H), 3.22 - 3.32 (m, 2 H), 3.73 (s, 2 H), 3.83 - 3.99 (m, 2 H), 4.20 - 4.33 (m, 1 H), 6.70 - 6.80 (m, 1 H), 6.80 - 6.87 (m, 1 H), 7.19 - 7.25 (m, 1 H). | CDCl3 | 456 [M+H] + |

(continued)

| Compound Example | Compound name | Structure | 1H NMR δ ppm | Solvent | ESI MS m/z |
|---|---|---|---|---|---|
| 16 | 4-Fluoro-1'-(4-hydroxy-8-methyl-6,7-dihydro-5H-pyrido[2,3-d] pyrimidin-2-yl)-6-(1-methylpyrazol-4-yl) spiro[2-benzofuran-3,4'-piperidin]-1-one | 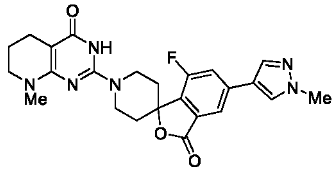 | 1.71 - 1.90 (m, 4 H), 2.34 - 2.55 (m, 4 H), 3.08 (s, 3 H), 3.19 - 3.30 (m, 2 H), 3.38 (br t, J=12.5 Hz, 2 H), 3.98 (s, 3 H), 4.68 (br d, J=13.2 Hz, 2 H), 7.42 (dd, J=10.1, 1.2 Hz, 1 H), 7.69 (s, 1 H), 7.74 - 7.82 (m, 2 H). | CDCl3 | 465 [M+H] + |
| 17 | 4-Fluoro-1-methyl-1'-(8-methyl-4-oxo-3,4,5,6,7,8-hexahydropyrido[2.3-d]pyrimidin-2-yl)-6-[4-(methylsulfonyl) pipe razin-1-yl]spiro [indolin-3,4'-piperidin]-2(1H)-one | 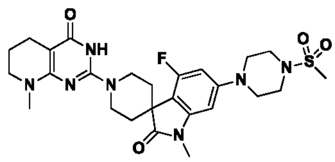 | 1.77-1.87 (m, 4 H), 2.10-2.20 (m, 2 H), 2.39-2.50 (m, 2 H), 2.84 (s, 3 H), 3.07 (s, 3 H), 3.18 (s, 3 H), 3.20-3.28 (m, 2 H), 3.28-3.35 (m, 4 H), 3.36-3.43 (m, 4 H), 3.86-3.98 (m, 2 H), 4.09-4.22 (m, 2 H), 6.18-6.28 (m, 2 H), 10.55 (br. s, 1 H). | CDCl3 | 560 [M+H] + |
| 18 | Methyl 4-[4-fluoro-1-methyl-1'-(8-methyl-4-oxo-3,4,5,6,7,8-hexahydropyrido[2, 3-d]pyrimidin-2-yl)-2-oxospiro[indolin-3,4'-pyperidin]-6-yl] piperazin-1-carboxylate | 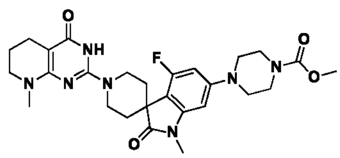 | 1.76-1.89 (m, 4 H), 2.10-2.21 (m, 2 H), 2.41-2.49 (m, 2 H), 3.06 (s, 3H), 3.14-3.21 (m, 7 H), 3.22-3.29 (m, 2 H), 3.74 (s, 3 H), 3.57-3.68 (m, 4 H), 3.88-3.99 (m, 2 H), 4.10-4.21 (m, 2 H), 6.16-6.25 (m, 2 H), 10.59 (br. s, 1H). | CDCl3 | 540 [M+H] + |

[Table 6]

| Compound Example | Compound name | Structure | 1H NMR δ ppm | Solvent | ESI MS m/z |
|---|---|---|---|---|---|
| 19 | 6-(cis-2,6-Dimethylmorpholin-4-yl)-4-fluoro-1-methyl-1'-(8-methyl-4-oxo-3,4,5,6,7,8-hexahydropyrido [2,3-d]pyrimidin-2-yl)spiro[indolin-3,4'-piperidin]-2(1H)-one | 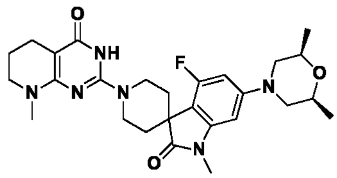 | 1.27 (d, J=6.2 Hz, 6 H), 1.79-1.87 (m, 4 H), 2.11-2.20 (m, 2 H), 2.42-2.49 (m, 4 H), 3.07 (s, 3 H), 3.19 (s, 3 H), 3.23-3.27 (m, 2 H), 3.40-3.45 (m, 2 H), 3.73-3.82 (m, 2 H), 3.89-3.97 (m, 2 H), 4.07-4.15 (m, 2 H), 6.16-6.23 (m, 2 H). | CDCl3 | 511 [M+H] + |
| 20 | 4-Fluoro-6-(4-methoxypiperidin-1-yl)-1-methyl-1'-(8-methyl-4-oxo-3,4,5,6,7,8-hexahydropyrido [2,3-d]pyrimidin-2-yl)spiro[indolin-3,4'-piperidin]-2(1H)-one | 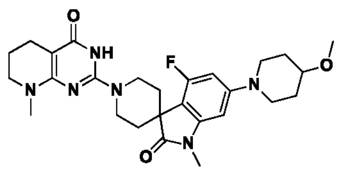 | 1.66-1.76 (m, 2 H), 1.79-1.87 (m, 4 H), 1.96-2.04 (m, 2 H), 2.10-2.20 (m, 2 H), 2.43-2.48 (m, 2 H), 2.96-3.04 (m, 2 H), 3.06 (s, 3 H), 3.17 (s, 3 H), 3.22-3.27 (m, 2 H), 3.36-3.44 (m, 4 H), 3.47-3.55 (m, 2 H), 3.89-3.98 (m, 2 H), 4.09-4.17 (m, 2 H), 6.19-6.24 (m, 2 H). | CDCl3 | 511 [M+H] + |
| 21 | 1-[4-Fluoro-1-methyl-1'-(8-methyl-4-oxo-3,4,5,6,7,8-hexahydropyrido [2,3-d]pyrimidin-2-yl)-2-oxospiro [indolin-3,4'-piperidin]-6-yl] piperidin-4-carbonitrile | 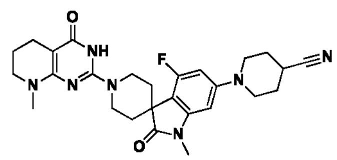 | 1.79-1.87 (m, 4 H), 1.95-2.21 (m, 6 H), 2.44-2.49 (m, 2 H), 244-2.49 (m, 2 H), 2.80-2.88 (m, 1 H), 3.07 (s, 3 H), 3.13-3.21 (m, 5 H), 3.22-3.27 (m, 2 H), 3.40-3.48 (m, 2 H), 3.88-3.97 (m, 2 H), 4.09-4.17 (m, 2 H), 6.18-6.25 (m, 2 H). | CDCl3 | 506 [M+H] + |

[Table 7]

| Compound Example | Compound name | Structure | 1H NMR δ ppm | Solvent | ESI MS m/z |
|---|---|---|---|---|---|
| 22 | 4-Fluoro-6-(4-hydroxycyclohexyl)-1-methyl-1'-(8-methyl-4-oxo-3,4,5,6,7,8-hexahydropyrido [2,3-d]pyrimidin-2-yl) spiro[indolin-3,4'-piperizin]-2(1H)-one | 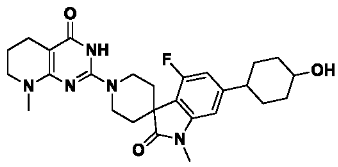 | 1.37-1.59 (m, 4 H), 1.78-1.87 (m, 4 H), 1.90-1.98 (m, 2 H), 2.04 (s, 1 H), 2.07-2.15 (m, 2 H), 2.15-2.28 (m, 2 H), 2.39-2.55 (m, 3 H), 3.07 (s, 3 H), 3,19 (s, 3H), 3.21-3.26 (m, 2 H), 3.64-3.74 (m, 1 H), 3.88-3.98 (m, 2 H), 4.16-4.25 (m, 2 H), 6.48 (d, J=1.0 Hz, 1 H), 6.58 (dd, J=11.0, 1.0 Hz, 1 H). | CDCl3 | 495 [M+H]+ |
| 23 | 6-(cis-2,6-Dimethylmorpholin-4-yl)-4-fluoro-1-methyl-1'-(4-oxo-3,4,5,6,7,8-hexahydropyrido [2,3-d]pyrimidin-2-yl) spiro[indolin-3,4'-piperidin]-2(1H)-one | 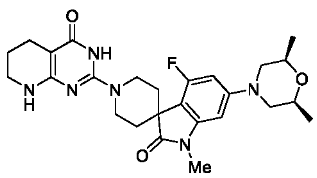 | 1.26 (s, 3H), 1.29 (s, 3H), 1.77 -1.87 (m, 4 H), 2.07 - 2.24 (m, 2 H), 2.37 - 2.52 (m, 4 H), 3.19 (s, 3 H), 3.25 - 3.35 (m, 2 H), 3.37 -3.50 (m, 2H), 3.72 - 4.00 (m, 4 H), 4.06 - 4.22 (m, 2 H), 4.64 (brs, 1 H), 6.12 - 6.27 (m, 2 H). | CDCl3 | 497 [M+H]+ |
| 24 | 6-(cis-2,6-Dimethylmorpholin-4-yl)-4-fluoro-1-(2,2,2-trifluoroethyl)-1'-(4-oxo-3,4,5,6,7,8-hexahydropyrido [2,3-d]pyrimidin-2-yl) spiro[indolin-3,4'-piperidin]-2(1H)-one | 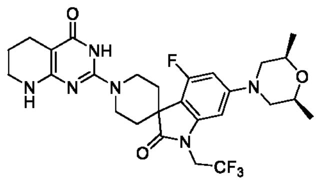 | 1.26 (s, 3 H), 1.28 (s, 3 H), 1.77 - 1.87 (m, 4 H), 2.14 - 3.31 (m, 2 H), 2.37 - 2.53 (m, 4 H), 3.24 - 3.46 (m, 4 H), 3.69 - 3.93 (m, 4 H), 4.15 -4.39 (m, 4 H), 4.65 (brs, 1 H), 6.16 - 6.34 (m, 2 H). | CDCl3 | 565 [M+H]+ |

(Compound Example 101)

(2R)-2-[5-(4-Ethoxyphenyl)-2H-1,2,3-triazol-4-yl]-8-methyl-2,3-dihydro-1H-quinazolin-4-one

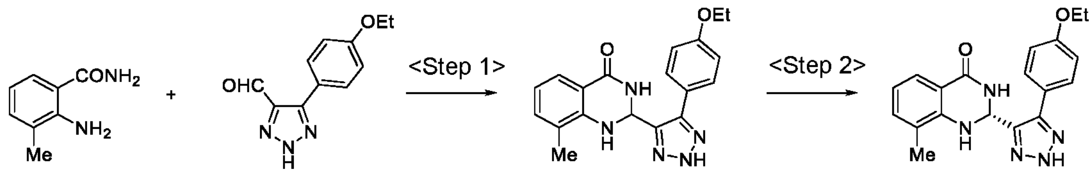


<Step 1>

2-amino-3-methylbenzamide (1.00 g) and 5-(4-ethoxyphenyl)-2H-1,2,3-triazol-4-carbaldehyde (1.74 g) were suspended in ethanol (30 mL), and acetic acid (100 μL) was added. The suspension was heated to 150°C and reacted for 3 hours in a microwave reaction apparatus. The suspension was cooled to room temperature, and stirred for 10 hours, and the precipitated solid was taken by filtration. The solid was washed with ethanol (3 mL) three times, and then dried by heating (45°C) under reduced pressure to give 2-[5-(4-ethoxyphenyl)-2H-1,2,3-triazol-4-yl]-8-methyl-2,3-dihydro-1H-quinazolin-4-one (1.45 g) as a white solid.
$^1$H-NMR (270M Hz, DMSO-$d_6$) δ: 1.33 (t, J = 6.9 Hz, 3H), 2.04 (s, 3H), 4.06 (q, J = 6.9 Hz, 2H), 6.06 (s, 1H), 6.34 (brs, 1H), 6.69 (t, J = 7.4 Hz, 1H), 7.01 (d, J = 8.6 Hz, 2H), 7.15 (d, J = 6.9 Hz, 1H), 7.56 (d, J = 7.3 Hz, 1H), 7.72 (d, J = 8.6 Hz, 2H), 8.23 (brs, 1H). MS (ESI) m/z: 350.36 $[M+H]^+$.

<Step 2>

2-[5-(4-Ethoxyphenyl)-2H-1,2,3-triazol-4-yl]-8-methyl-2,3-dihydro-1H-quinazolin-4-one (136 mg) was optically resolved by high-performance liquid chromatography (methanol/TFA = 100/0.05) with a chiral column (CHIRALPAC-IC, DAICEL) to give the title compound (59.4 mg) as a white solid.
$^1$H-NMR (270M Hz, DMSO-$d_6$) δ: 1.33 (brt, J = 6.9 Hz, 3H), 2.03 (brs, 3H), 3.96-4.16 (m, 2H), 6.07 (brs, 1H), 6.32 (brs, 1H), 6.68 (brs, 1H), 7.01 (brd, J = 7.3 Hz, 2H), 7.15 (brd, J = 6.9 Hz, 1H), 7.56 (brd, J = 6.9 Hz, 1H), 7.62-7.86 (m, 2H), 8.20 (brs, 1H), 14.84 (brs, 1H). MS (ESI) m/z: 350.26 $[M+H]^+$.
[α] $D^{24}$ = -148 ° (C = 0.1, MeOH)

The compounds of Compound Examples 102 to 108 and 113 were prepared using the method of Compound Example 101 and methods based thereon, and methods disclosed in literatures and methods based thereon. Tables 8 to 10 below show the compounds of Compound Examples 101 to 108 and 113. By X-ray diffraction performed under the same conditions as described above, the compound prepared in Compound Example 101 was confirmed to be a nicotinamide-type compound which binds to the nicotinamide binding pocket of tankyrase.

[Table 8]

| Compound Example | Compound name | Structure | $^1$H NMR δ ppm | Solvent | ESI MS m/z |
|---|---|---|---|---|---|
| 101 | (2R)-2-[5-(4-Ethoxyphenyl)-2H-1,2,3-triazol-4-yl]-8-methyl-2,3-dihydro-1 H-quinazolin-4-one | 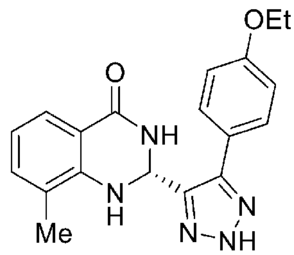 | 1.33 (br t, J=6.9 Hz, 3 H), 2.03 (br s, 3 H), 3.96 - 4.16 (m, 2 H), 6.07 (br s, 1 H), 6.32 (br s, 1 H), 6.68 (br s, 1 H), 7.01 (br d, J=7.3 Hz, 2 H), 7.15 (br d, J=6.9 Hz, 1 H), 7.56 (br d, J=6.9 Hz, 1 H), 7.62 - 7.86 (m, 2 H), 8.20 (br s, 1 H), 14.84 (br s, 1 H) | DMSO-d6 | 350.26 [M+H]+ |
| 102 | 2-[3-(3-Fluorophenyl)-1 H-pirazol-4-yl]-1-methyl-2,3-dihydroquinazoli n-4-one | 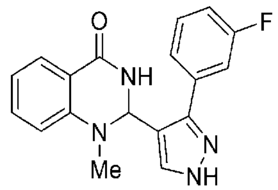 | 5.75 (br s, 1 H), 6.70 (br d, J=8.1 Hz, 1 H), 6.83 (br t, J=7.3 Hz, 1 H), 7.05 - 7.61 (m, 6 H), 7.72 (br d, J=7.6 Hz, 1 H), 8.48 (br s, 1 H), 12.63 - 13.46 (m, 1 H) | DMSO-d6 | 323.28 [M+H]+ |

(continued)

| Compound Example | Compound name | Structure | $^1H$ NMR δ ppm | Solvent | ESI MS m/z |
|---|---|---|---|---|---|
| 103 | 2-[5-(4-Ethoxyphenyl)-1 H-pyrazol-4-yl]-8-methyl-2,3-dihydro-1 H-quinazolin-4-one | 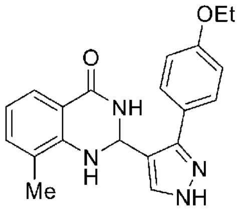 | 1.33 (t, J=6.9 Hz, 3 H), 2.07 (s, 3 H), 4.05 (q, J=6.9 Hz, 2 H), 5.75 (s, 1 H), 6.02 (s, 1 H), 6.69 (t, J=7.6 Hz, 1 H), 7.01 (br d, J=8.6 Hz, 2 H), 7.16 (d, J=6.6 Hz, 1 H), 7.51 - 7.62 (m, 3 H), 7.77 (br. s, 1 H), 8.13 (s, 1 H), 12.99 (br s, 1 H) | DMSO-d6 | 349.40 [M+H]+ |
| 104 | 2-[5-(4-Methoxyphenyl)-2H-1,2,3-triazol-4-yl]-1-methyl-2,3-dihydroquinazolin-4-one | 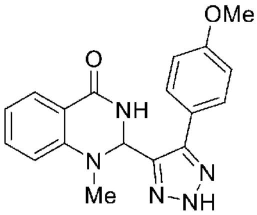 | 3.80 (s, 3 H), 5.98 (d, J=3.0 Hz, 1 H), 6.68 (d, J=8.2 Hz, 1 H), 6.81 (t, J=7.6 Hz, 1 H), 7.04 (d, J=8.6 Hz, 2 H), 7.31 - 7.40 (m, 1 H), 7.61 - 7.75 (m, 3 H), 8.50 (d, J=30 Hz, 1 H) | DMSO-d6 | 336.33 [M+H]+ |
| 105 | 2-[5-(4-Methoxyphenyl)-2H-1,2,3-triazol-4-yl]-8-methyl-2,3-dihydro-1 H-quinazolin-4-one | 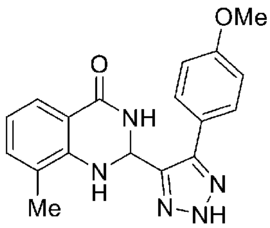 | 2.04 (s, 3 H), 3.79 (s, 3 H), 6.07 (s, 1 H), 6.35 (br s, 1 H), 6.69 (t, J=7.4 Hz, 1 H), 7.03 (d, J=8.9 Hz, 2 H), 7.15 (d, J=7.3 Hz, 1 H), 7.56 (d, J=6.9 Hz, 1 H), 7.74 (br d, J=8.6 Hz, 2 H), 8.23 (br s, 1 H) | DMSO-d6 | 336.23 [M+H]+ |

[Table 9]

| Compound Example | Compound name | Structure | $^1H$ NMR 6 ppm | Solvent | ESI MS m/z |
|---|---|---|---|---|---|
| 106 | 11-[5-(4-Methoxyphenyl)-2H-1,2,3-triazol-4-yl]-1,10-diazatricyclo[6.3. 1.0$^{4,12}$] dodeca-4(12),5,7 -trien-9-one | 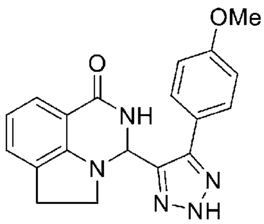 | 2.82 - 3.12 (m, 4 H), 3.77 (s, 3 H), 5.82 (s, 1 H), 6.75 - 6.89 (m, 1 H), 6.93 - 7.11 (m, 2 H), 7.27 (br d, J=7.3 Hz, 1 H), 7.37 (br d, J=7.9 Hz, 1 H), 7.64 - 7.94 (m, 2 H), 8.16 - 8.27 (m, 1 H), 15.02 (br s, 1 H) | DMSO-d6 | 348.44 [M+H] + |

(continued)

| Compound Example | Compound name | Structure | $^1$H NMR 6 ppm | Solvent | ESI MS m/z |
|---|---|---|---|---|---|
| 107 | 8-Methyl-2-[5-(4-(trideuteriomethoxy) phenyl)-2H-1,2,3-triazol-4-yl]-2,3-dihydro-1H-quinazolin-4-one | 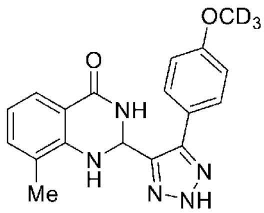 | 2.03 (s, 3 H), 6.07 (br s, 1 H), 6.34 (br. s, 1 H), 6.69 (t, J=7.9 Hz, 1 H), 7.02 (br. d, J=8.2 Hz, 2 H), 7.15 (br d, J=7.3 Hz, 1 H), 7.56 (br d, J=7.9 Hz, 1 H), 7.66 - 7.83 (m, 2 H), 8.22 (br s, 1 H), 14.86 (br s, 1 H) | DMSO-d6 | 339.28 [M+H] + |
| 108 | 2-[4-[4-(2,2-Difluoroethoxy) phenyl]-2H-1,2,3-triazol-5-yl]-8-methyl-2,3-dihydro-1 H-quinazolin-4-one | 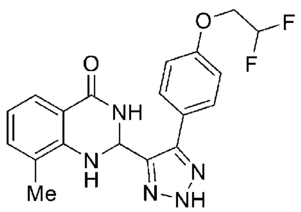 | 2.03 (s, 3 H), 4.26 - 4.45 (m, 2 H), 6.08 (br s, 1 H), 6.16 - 6.79 (m, 3 H), 7.00 - 7.23 (m, 3 H), 7.56 (br d, J=7.3 Hz, 1 H), 7.64 - 7.90 (m, 2 H), 8.22 (br s, 1 H), 14.90 (br s, 1 H) | DMSO-d6 | 386.27 [M+H] + |

[Table 10]

| Example | Compound name | Structural formula | $^1$H NMR δ ppm | Solvent | ESI MS m/z |
|---|---|---|---|---|---|
| 113 | 2-[4-(4-Methoxyphenyl)-1 H-pyrazol-3-yl]-8-methyl-2,3-dihydro-1 H-quinazolin-4-one | 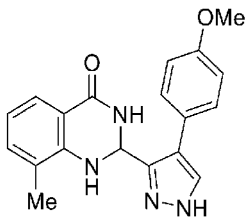 | | | 335.32 [M+H] + |

(Compound Example 109)

2-[5-(4-Methoxyphenyl)-1H-pyrazol-4-yl]-1-methyl-2,3-dihydroquinazolin-4-one (CAS No. 1252133-21-3)

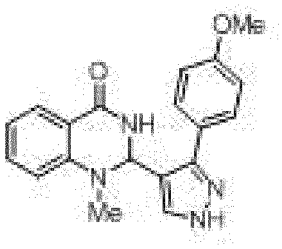

The title compound was prepared using the method of Compound Example 101.

(Compound Example 110)

4-(5-((E)-2-(4-(2-Chlorophenyl)-5-(5-(methylsulfonyl)pyridin-2-yl)-4H-1,2,4-triazol-3-yl)ethenyl)-1,3,4-oxazol-2-yl]benzonitrile[4-(5-( (E)-2-(4-(2-chlorophenyl)-5-(5-(methylsulfonyl)pyridin-2-yl)-4H-1,2,4-triazol-3-yl)ethenyl)-1,3,4-oxadiazol-2-yl)benzonitrile; G007-LK]

The title compound was prepared in accordance with the synthesis method disclosed in Patent Literature 3 etc. The compound is an adenosine-type compound which binds to the adenosine binding pocket of tankyrase.

(Compound Example 111)

N-(8-Quinolyl)-4-[[(3aR,7aS)-1,3-dioxo-4β,7β-methano-1,3,3a,4,7,7a-hexahydro-2H-isoindol]-2-yl]benzamide (IWR-1)

The title compound was prepared in accordance with the synthesis method as disclosed in known literatures etc. The compound is an adenosine-type compound which binds to the adenosine binding pocket of tankyrase.

(Compound Example 112)

N-(Cyclopropylmethyl)-4-(4-methoxybenzoyl)-N-[(3,5,7,8-tetrahydro-4-oxo-4H-pyrano[4,3-d]pyrimidin-2-yl)methyl]-1-(piperidinacetamide) (NVP-TNKS656)

The title compound was prepared in accordance with a synthesis method as disclosed in Non Patent Literature 2 etc. The compound is a dual-type compound which acts on both the nicotinamide binding pocket and the adenosine binding pocket of tankyrase.

(Test Example 1) Tankyrase Inhibitory Activity Test

The enzymatic activity of tankyrase 1 and the enzymatic activity of tankyrase 2 were measured by the ELISA method based on assessment of auto-poly(ADP-ribosyl)ation to evaluate the tankyrase inhibitory activity of the compound prepared in each of Compound Examples (test compound) (inhibitory activity against tankyrase 1 (TNKS1) and inhibitory activity against tankyrase 2 (TNKS2)). First, Flag-tagged tankyrase 1 (1,024-1, 327aa, SAM+PARP) and tankyrase 2 (613-1, 116aa, ANK5+SAM+PARP) were synthesized with a cell-free protein expression system, and diluted with a Tris buffer solution (50 mM Tris-HCl (pH 8.0), 150 mM NaCl, 10% glycerol). 50 μL of the diluted tankyrase 1 or tankyrase 2 was added to a plate with an anti-FLAG M2 monoclonal antibody immobilized thereon (Anti-FLAG High-Sensitivity M2-Coated Plate) (Sigma-Aldrich), and the mixture was left to stand overnight at 4°C. Thereafter, the plate was washed four times with a PBS (PBST) buffer containing 0.1% TritonX-100.

Subsequently, the test compound diluted with an assay buffer (50 mM Tris-HCl (pH 8.0), 4 mM $MgCl_2$, 0.2 mM DTT) (DMSO was used as a control) was added to each well of the plate, and the mixture was left to stand at room temperature for 10 minutes. Thereafter, a biotin-labeled NAD solution (225 μM NAD, 25 μM 6-Biotin-17-NAD (Travigen, Inc.)) was added and mixed as a donor substrate, and the mixture was reacted at 30°C for 45 minutes. To blank wells was added distilled water instead of the biotin-labeled NAD solution. After the reaction, the plate was washed with a PBST buffer four times. Thereafter, HRP (horseradish peroxidase)-labeled streptavidin (Travigen, Inc.) was diluted with a PBS buffer by 1,000 times, and added to each well, and the mixture was reacted at room temperature for 20 minutes. The plate was washed with a PBST buffer four times, a chemiluminescent substrate solution TACS-Sapphire (Travigen, Inc.) was then added to each well, the mixture was reacted at room temperature for 20 minutes, and thechemiluminescence intensity was measured using a chemiluminescence measuring apparatus.

The residual enzymatic activity in the presence of the test compound was determined from the following expression. On the basis of the residual enzymatic activity at each of multiple concentrations of the test compound, the enzyme inhibitory activity was calculated in terms of a 50%-inhibition concentration ($IC_{50}$ value) using data analysis software Origin (LightStone Corp.).

Residual activity (%) = {(chemiluminescence intensity with test compound added) - (chemiluminescence intensity of blank)}/{(chemiluminescence intensity of control) - (chemiluminescence intensity of blank)}

For the TNKS1 (inhibitory activity against tankyrase 1) and the TNKS2 (inhibitory activity against tankyrase 2) of each test compound as measured through the above-mentioned method, a test compound having an $IC_{50}$ value of less than 0.02 μM was rated "A", a test compound having an $IC_{50}$ value of 0.02 μM or more and less than 0.2 μM was rated "B", a test compound having an $IC_{50}$ value of 0.2 μM or more and less than 1 μM was rated "C", and a test compounds having an $IC_{50}$ value of 1 μM or more was rated "D". Tables 11 and 12 below show the results.

(Test Example 2) Cell proliferation inhibitory Activity Test

The cell proliferation inhibitory activity of the compound, which had been prepared in each of Compound Examples, against the human colorectal cancer cell line COLO-320DM was evaluated by Celltiter-Glo Luminescent Cell Viability Assay (Promega Corporation; G7573). COLO-320DM cells were cultured in RPMI-1640 medium containing 2mM glutamine (Wako Pure Chemical Industries, Ltd; 189-02025) supplemented with 10% fetal bovine serum. The cultured cells were washed with PBS, and then dissociated with trypsin/EDTA, and a cell solution with $3 \times 10^4$ cells/mL was prepared.

Subsequently, the cell solution was seeded in a 96-well microplate (Thermo/Nunc Company; 136101) in an amount of 70 μL per well, and cultured overnight under the condition of 37°C and 5% $CO_2$. The next day, a test compound solution obtained by diluting the test compound (DMSO solution) with a cell culture medium (final concentration of DMSO was 1%) was added in an amount of 10 μL per well, and the mixture was reacted under the condition of 37°C and 5% $CO_2$ for 96 hours (a 1% DMSO solution was used as a control). Thereafter, a Celltiter-Glo Luminescent Cell Viability Assay reagent was added (Promega Corporation; G7573) in an amount of 80 μL per well, the mixture was stirred with a shaker for 2 minutes while light was blocked with an aluminum foil, and the mixture was incubated at room temperature for 10 minutes.

Thereafter, a luminescence signal was measured with a luminometer (Biotech Company; Synergy). The ratio of cell proliferation in each compound addition group to cell proliferation in a control group containing no test compound solution, where cell proliferation in the control group is 100%, was determined, and the value of a compound concentration necessary for suppressing the amount of residual cells to 50% of that in the control (G150) was calculated as the cell proliferation inhibitory activity. For the COLO-320DM (cell proliferation inhibitory activity against COLO-320DM) of each test compound as measured through the above-mentioned method, a test compound having a G150 value of less than 1 μM was rated "A", a test compound having a G150 value of 1 μM or more and less than 10 μM was rated "B", a test compound having a G150 value of 10 μM or more was rated "C", and an unevaluated test compound was designated as "NT". Tables 11 and 12 show the results along with the results of the tankyrase inhibitory activity test.

[Table 11]

| Compound Example | TNKS 1 | TNKS2 | COLO-320DM |
|---|---|---|---|
| 1 | A | A | B |
| 2 | A | A | A |
| 3 | A | A | A |
| 4 | A | A | A |
| 5 | A | A | A |
| 6 | A | A | A |
| 7 | A | A | A |

(continued)

| Compound Example | TNKS 1 | TNKS2 | COLO-320DM |
|---|---|---|---|
| 8 | B | B | A |
| 9 | A | A | A |
| 10 | A | A | A |
| 11 | A | A | A |
| 12 | A | A | A |
| 13 | A | A | A |
| 14 | A | A | A |
| 15 | A | A | A |
| 16 | A | A | A |
| 17 | A | A | A |
| 18 | B | A | A |
| 19 | A | A | A |
| 20 | B | A | A |
| 21 | B | A | A |
| 22 | A | B | A |
| 23 | A | A | A |
| 24 | A | A | A |

[Table 12]

| Example | TNKS1 | TNKS2 | COLO-320DM |
|---|---|---|---|
| 101 | A | A | A |
| 102 | A | A | B |
| 103 | A | A | B |
| 104 | A | A | B |
| 105 | A | A | A |
| 106 | A | A | B |
| 107 | A | A | A |
| 108 | A | A | B |
| 109 | A | A | C |
| 113 | A | B | C |

As shown in Tables 11 and 12, specified compounds prepared in Compound Examples were confirmed to have sufficient tankyrase inhibitory activity, and these compounds were confirmed to be tankyrase inhibitory compounds having sufficient tankyrase inhibitory activity.

(Test Example 3) MTT Assay

The ratio of residual cells after treatment of the human colorectal cancer cell line DLD-1 with a combination of the compound prepared in each Compound Example (test compound or tankyrase inhibitory compound) and a microtubule inhibitory compound was measured to evaluate the cell killing effect of the combination on the human colorectal cancer cell line DLD-1. The compounds of the test compound and the microtubule inhibitory compound are as follows:

(Example 1): combination of the compound prepared in Compound Example 1 and docetaxel, paclitaxel, vincristine or vinblastine
(Example 2): combination of the compound prepared in Compound Example 5 and docetaxel, paclitaxel, vincristine or vinblastine
(Example 3): combination of the compound prepared in Compound Example 19 and docetaxel, paclitaxel, vincristine or vinblastine
(Example 4): combination of the compound prepared in Compound Example 101 and docetaxel, paclitaxel, vincristine or vinblastine
(Example 5): combination of the compound prepared in Compound Example 110 (G007-LK) and docetaxel, paclitaxel, vincristine or vinblastine
(Example 6): combination of the compound prepared in Compound Example 111 (IWR-1) and docetaxel, paclitaxel, vincristine or vinblastine
(Example 7): combination of the compound prepared in Compound Example 112 (NVP-TNKS656) and docetaxel, paclitaxel, vincristine or vinblastine

Taxotere (docetaxel hydrate, Sigma-Aldrich) was used as docetaxel, Taxol (Sigma-Aldrich) was used as paclitaxel, Oncovin (vincristine sulfate, Sigma-Aldrich) was used as vincristine, and vinblastine sulfate (Wako Pure Chemical Industries, Ltd.) was used as vinblastine.

First, the human colorectal cancer cell line DLD-1 was suspended in RPMI1640 medium (hereinafter, medium) containing inactivated 10% fetal bovine serum, and the cells were seeded in a 96-well microplate at 250 cells (75 μL) per well. The cells were subjected to stationary culture overnight in a $CO_2$ incubator at 37°C, and a medium (25 μL) containing a combination of a test compound and a microtubule inhibitory compound in each of above Examples was added in triplicate in such a manner that the final concentrations of the test compound were 0, 1 and 3 μM and that the final concentrations of the microtubule inhibitory compound were 0, 0.0412, 0.123, 0.37, 1.11, 3.3, 10, 30 and 90 nM. As a control, 25 μL of a medium containing only dimethyl sulfoxide (DMSO) which is a solvent was added. The cells were cultured in a $CO_2$ incubator at 37°C for 120 hours, MTT ([3-(4,5-dimethyl-thiazol-2-yl)-2,5-diphenyltetrazolium bromide])/phosphate buffer physiological saline was then added in such a manner that the final concentration was 0.5 mg/mL, and the mixture was left to stand in a $CO_2$ incubator at 37°C for 4 hours. The medium was removed, and dimethyl sulfoxide was added in an amount of 100 μL per well. The mixture was stirred at room temperature for 1 hour, and the absorbance of each well (570 nm to 630 nm) was then measured with xMark Microplate Spectrophotometer (Bio-Rad Laboratories, Inc.). The concentration of the microtubule inhibitor at which the relative number of cells compared to the number of cells without the microtubule inhibitory compound is 50% was defined as an IC50 value for each concentration of the test compound.

Figures 1A to 7D show relationships between the concentration of each microtubule inhibitory compound and the relative number of cells (IC50 curve) when the final concentrations of the test compound are 0 μM (single agent), 1 μM and 3 μM. Table 13 below shows IC50 values when the final concentrations of the test compound are 0 μM (single agent), 1 μM and 3 μM. In the drawings, the IC50 curves of systems in which the final concentrations of the test compound are 1 μM and 3 μM are curves in which the IC50 value when the concentration of each microtubule inhibitory compound such as docetaxel is 0 μM (i.e. there is no microtubule inhibitory compound) is calculated as 100%. Hence, if the effect exhibited by a combination of each test compound and each microtubule inhibitory compound is simply an additive effect, the IC50 curves for the concentrations of microtubule inhibitory compounds of systems in which the final concentrations of the test compound are 1 μM and 3 μM each coincide with the IC50 curve in which the final concentration of the test compound is 0 μM (single agent). In other words, since the IC50 value of the microtubule inhibitor is smaller when the final concentration of the test compound is 1 μM or 3 μM than when the final concentration of the test compound is 0 μM (single agent), the cell killing effect of a combination of the test compound and the microtubule inhibitory compound on the human colorectal cancer cell line DLD-1 is not an additive effect but a synergic effect of the compounds.

[Table 13]

| Example | | 0 μM (single agent) | 1 μM | 3 μM |
|---|---|---|---|---|
| | Microtubule inhibitory compound | IC50 (nM) | | |
| 1 | Docetaxel | 2.987 | 2.084 | 1.006 |
| | Paclitaxel | 9.385 | 5.507 | 3.557 |
| | Vincristine | 7.639 | 3.103 | 2.140 |
| | Vinblastine | 2.377 | 1.490 | 0.932 |

(continued)

| Example | Microtubule inhibitory compound | 0 μM (single agent) | 1 μM | 3 μM |
|---|---|---|---|---|
| | | IC50 (nM) | | |
| 2 | Docetaxel | 2.987 | 1.802 | 0.799 |
| | Paclitaxel | 9.385 | 5.041 | 2.405 |
| | Vincristine | 7.639 | 2.651 | 1.263 |
| | Vinblastine | 2.377 | 1.013 | 0.768 |
| 3 | Docetaxel | 2.987 | 1.372 | 0.778 |
| | Paclitaxel | 9.385 | 5.050 | 2.797 |
| | Vincristine | 7.639 | 2.711 | 0.978 |
| | Vinblastine | 2.377 | 1.076 | 0.872 |
| 4 | Docetaxel | 2.696 | 2.196 | 0.849 |
| | Paclitaxel | 7.967 | 5.247 | 1.506 |
| | Vincristine | 7.439 | 2.804 | 0.732 |
| | Vinblastine | 2.679 | 1.496 | 0.423 |
| 5 | Docetaxel | 2.987 | 1.650 | 0.891 |
| | Paclitaxel | 9.385 | 6.201 | 3.484 |
| | Vincristine | 7.639 | 2.591 | 2.081 |
| | Vinblastine | 2.377 | 1.652 | 1.034 |
| 6 | Docetaxel | 3.180 | 2.019 | 1.954 |
| | Paclitaxel | 15.222 | 6.355 | 5.886 |
| | Vincristine | 13.655 | 6.293 | 4.703 |
| | Vinblastine | 5.590 | 2.446 | 2.347 |
| 7 | Docetaxel | 3.180 | 2.135 | 1.925 |
| | Paclitaxel | 15.222 | 7.270 | 6.209 |
| | Vincristine | 13.655 | 6.731 | 5.322 |
| | Vinblastine | 5.590 | 2.877 | 2.535 |

As shown in Figures 1A to 7D and Table 13, a combination of a test compound and a microtubule inhibitory compound was confirmed to exhibit a synergic cell killing effect on the human colorectal cancer cell line DLD-1, and the combination according to the present invention was confirmed to be useful as an anticancer agent effective for treating and/or preventing proliferative diseases such as a cancer.

When instead of the microtubule inhibitory compound, any of other anticancer agents (temozolomide, melphalan, gemcitabine, cytarabine (Ara-C), fluorouracil (5-FU), pemetrexed, mercaptopurine, methotrexate, irinotecan (SN-38), etoposide (VP-16), actinomycin D, daunorubicin, doxorubicin, bleomycin, mitoxantrone, oxaliplatin, carboplatin and cisplatin) was used, and the test compound prepared in Compound Example 5 was further added thereto at a predetermined concentration, the cell survival rate decreased to about 50%, where the cell survival rate was 100% when only the anticancer agent was added at a predetermined concentration. Thus, such a combination was confirmed to exhibit at least an additive cell killing effect without cancelling each other's action (Example 8 below shows the results of specific examples).

That is, the present invention also relates to an anticancer agent in which a tankyrase inhibitor containing at least one compound of the formula (1) or (11) as an active ingredient is combined with at least one anticancer agent selected from the group consisting of an alkylating agent (e.g., temozolomide or melphalan), an antimetabolite (e.g., gemcitabine, cytarabine (Ara-C), fluorouracil (5-FU), pemetrexed, mercaptopurine or methotrexate), a plant alkaloid (with the exclusion of microtubule inhibitory compounds; e.g., irinotecan (SN-38) or etoposide (VP-16)), a topoisomerase

inhibitor (e.g., irinotecan (SN-38) or etoposide (VP-16)), an agent serving as both a plant alkaloid and a topoisomerase inhibitor (e.g., irinotecan (SN-38) or etoposide (VP-16)), an anticancer antibiotic (e.g., actinomycin D, daunorubicin, doxorubicin, bleomycin or mitoxantrone) and a platinating agent (e.g., oxaliplatin, carboplatin or cisplatin).

(Test Example 4) MTT Assay

The ratio of residual cells in treatment of the human colorectal cancer cell line COLO-320DM with a combination of the compound prepared in each Compound Example (test compound or tankyrase inhibitory compound) and each of the anticancer agents was measured to evaluate the cell killing effect of the combination on the human colorectal cancer cell line COLO-320DM. The combinations of test compounds and anticancer agents are as follows.

(Example 8): combination of the compound prepared in Compound Example 5 and temozolomide, melphalan, gemcitabine, cytarabine (Ara-C), fluorouracil (5-FU), pemetrexed, mercaptopurine, methotrexate, irinotecan (SN-38), etoposide (VP-16), actinomycin D, daunorubicin, doxorubicin, bleomycin, mitoxantrone, oxaliplatin, carboplatin or cisplatin
(Example 9): combination of the compound prepared in Compound Example 110 (G007-LK) and irinotecan (SN-38)
(Example 10): combination of the compound prepared in Compound Example 11 and irinotecan (SN-38)
(Example 11): combination of the compound prepared in Compound Example 19 and irinotecan (SN-38)
(Example 12): combination of the compound prepared in Compound Example 101 and irinotecan (SN-38)
(Example 13): combination of the compound prepared in Compound Example 1 and irinotecan (SN-38)
(Example 14): combination of the compound prepared in Compound Example 113 and irinotecan (SN-38)

Commercially available compounds were used for temozolomide (manufactured by Sigma-Aldric), melphalan (manufactured by Signa-Aldrich), gemcitabine (Gemcitabine hydrochloride; manufactured by Sigma-Aldric), cytarabine (Cytosine β-D-arabinofuranoside (Ara-C); manufactured by Sigma-Aldrich), fluorouracil (5-Fluorouracil (5-FU); manufactured by Sigma-Aldrich), pemetrexed (Pemetrexed disodium heptahydrate; manufactured by Sigma-Aldrich), mercaptopurine (6-Mercaptopurine monohydrate; manufactured by Sigma-Aldrich), methotrexate (Methotrexate hydrate; manufactured by Sigma-Aldrich), irinotecan (7-Ethyl-10-hydroxycamptothecin (SN-38); manufactured by Sigma-Aldrich), etoposide (Etoposide (VP-16); manufactured by Enzo Life Sciences, Inc.), actinomycin D (manufactured by nacalai tesque), daunorubicin (Daunorubicin hydrochloride; Calbiochem), doxorubicin (Doxorubicin hydrochloride; manufactured by Sigma-Aldrich), bleomycin (Bleomycin sulfate; manufactured by Enzo Life Sciences, Inc.), mitoxantrone (Mitoxantrone dihydrochloride; manufactured by Sigma-Aldrich), oxaliplatin (manufactured by Sigma-Aldrich), carboplatin (manufactured by Sigma-Aldrich) and cisplatin (manufactured by Enzo Life Sciences, Inc.).

First, the human colorectal cancer cell line COLO-320DM was suspended in RPMI1640 medium (hereinafter, medium) containing inactivated 10% fetal bovine serum, and the cells were seeded in a 96-well microplate at 350 cells (75 μL) per well. The cells were subjected to stationary culture overnight in a $CO_2$ incubator at 37°C. Thereafter, in Example 8, a medium (25 μL) containing a combination of the compound prepared in Compound Example 5 and each of the anticancer agents was added in triplicate in such a manner that the final concentrations of the test compound (compound prepared in Compound Example 5) were 0 nM or 30 nM and that the final concentrations of each anticancer agent were those shown in Table 14. In Examples 9 to 14, a medium (25 μL) containing a combination of the compound prepared in each of Compound Examples and each of the anticancer agents was added in triplicate in such a manner that the final concentrations of the test compound were 0 nM or those shown in Figures 9 to 14. As a control, 25 μL of a medium containing only dimethyl sulfoxide (DMSO) which is a solvent was added. The cells were cultured in a $CO_2$ incubator at 37°C for 120 hours, MTT ([3-(4,5-dimethyl-thiazol-2-yl)-2,5-diphenyltetrazolium bromide])/phosphate buffer physiological saline was then added in such a manner that the final concentration was 0.5 mg/mL, and the mixture was left to stand in a $CO_2$ incubator at 37°C for 4 hours. The medium was removed, and dimethyl sulfoxide was added in an amount of 100 μL per well. The mixture was stirred at room temperature for 1 hour, and the absorbance of each well (570 nm to 630 nm) was then measured with xMark Microplate Spectrophotometer (Bio-Rad Laboratories, Inc.). The concentration of the anticancer agent at which the relative number of cells compared to the number of cells without the anticancer agent is 50% was defined as an IC50 value for each concentration of the test compound.

Figures 8A to 8C and Figures 9 to 14 show relationships between the concentration of each anticancer agent and the relative number of cells (IC50 curve) when the final concentrations of the test compound are 0 μM (single agent) or specified concentrations.

[Table 14]

| Anticancer agent | Treatment concentration |
|---|---|
| Temozolomide | 0, 0.0412, 0.123, 0.37, 1.11, 3.3, 10, 30, 90 μM |

(continued)

| Anticancer agent | Treatment concentration |
|---|---|
| Melphalan | 0, 0.0412, 0.123, 0.37, 1.11, 3.3, 10, 30, 90 μM |
| Gemcitabine | 0, 0.0412, 0.123, 0.37, 1.11, 3.3, 10, 30, 90 nM |
| Cytarabine | 0, 0.412, 1.23, 3.7, 11.1, 33.3, 100, 300, 900 nM |
| Fluorouracil | 0, 0.00412, 0.0123, 0.037, 0.11, 0.33, 1, 3, 9 μM |
| Pemetrexed | 0, 0.412, 1.23, 3.7, 11.1, 33.3, 100, 300, 900 nM |
| Mercaptopurine | 0, 0.0412, 0.123, 0.37, 1.11, 3.3, 10, 30, 90 nM |
| Methotrexate | 0, 0.0412, 0.123, 0.37, 1.11, 3.3, 10, 30, 90 nM |
| Irinotecan | 0, 0.0412, 0.123, 0.37, 1.11, 3.3, 10, 30, 90 nM |
| Etoposide | 0, 0.00412, 0.0123, 0.037, 0.11, 0.33, 1, 3, 9 μM |
| Actinomycin D | 0, 0.0412, 0.123, 0.37, 1.11, 3.3, 10, 30, 90 nM |
| Daunorubicin | 0, 0.412, 1.23, 3.7, 11.1, 33.3, 100, 300, 900 nM |
| Doxorubicin | 0, 0.412, 1.23, 3.7, 11.1, 33.3, 100, 300, 900 nM |
| Bleomycin | 0, 0.00412, 0.0123, 0.037, 0.11, 0.33, 1, 3, 9 μM |
| Mitoxantrone | 0, 0.412, 1.23, 3.7, 11.1, 33.3, 100, 300, 900 nM |
| Oxaliplatin | 0, 0.00412, 0.0123, 0.037, 0.11, 0.33, 1, 3, 9 μM |
| Carboplatin | 0, 0.0412, 0.123, 0.37, 1.11, 3.3, 10, 30, 90 μM |
| Cisplatin | 0, 0.00412, 0.0123, 0.037, 0.11, 0.33, 1, 3, 9 μM |

As shown in Figures 8A to 8C, where the cell survival rate was 100% when only each anticancer agent was added in an amount of 30 nM, the cell survival rate decreased to about 50% when the test compound prepared in Compound Example 5 was further added to the anticancer agent in an amount of 30 nM. Thus, such a combination was confirmed to exhibit at least an additive cell killing effect without cancelling each other's action. As shown in Figures 9 to 14, where the cell survival rate was 100% when only the anticancer agent (irinotecan) was added in an amount of 30 nM, the cell survival rate decreased to about 50% when the test compound prepared in each of Compound Examples 110, 11, 19, 101, 1 and 113 was added in the concentration shown in each of the figures. Thus, such a combination was confirmed to exhibit at least an additive cell killing effect without cancelling each other's action.

Industrial Application

As described above, the present invention can provide a novel anticancer agent and a kit useful for treating and/or preventing proliferative diseases such as a cancer.

## Claims

**1.** An anticancer agent for preventing and/or treating a cancer, comprising a combination of a tankyrase inhibitor containing a tankyrase inhibitory compound as an active ingredient and a microtubule inhibitor containing a microtubule inhibitory compound as an active ingredient.

**2.** An anticancer agent for preventing and/or treating a cancer, which is used to be administered in combination with a microtubule inhibitor containing a microtubule inhibitory compound as an active ingredient and which contains a tankyrase inhibitory compound as an active ingredient.

**3.** An anticancer agent for preventing and/or treating a cancer, which is used to be administered in combination with a tankyrase inhibitor containing a tankyrase inhibitory compound as an active ingredient and which contains a microtubule inhibitory compound as an active ingredient.

**4.** An anticancer agent for preventing and/or treating a cancer, comprising a tankyrase inhibitory compound and a microtubule inhibitory compound as active ingredients.

**5.** The anticancer agent according to any one of claims 1 to 4, wherein the tankyrase inhibitory compound acts on at least one of a nicotinamide binding pocket of tankyrase and an adenosine binding pocket of tankyrase.

**6.** The anticancer agent according to any one of claims 1 to 5, wherein the tankyrase inhibitory compound is at least one compound selected from the group consisting of:

a compound of the formula (1):

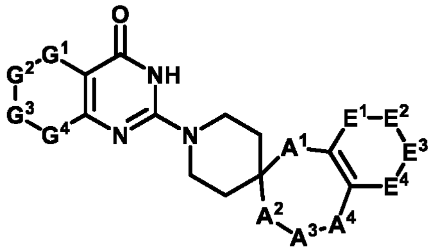

(1)

wherein $A^1$, $A^2$, $A^3$ and $A^4$ form a structure in which $A^1$ and $A^2$ each represent a single bond, one of $A^1$ and $A^2$ represents a single bond and the other represents $CH_2$, or $A^1$ represents a single bond and $A^4$ represents $CH_2$,

with the proviso that one of $A^3$ and $A^4$ is $CH_2$ or CO and the other is O or $NR^1$ when $A^1$ and $A^2$ each represent a single bond or $A^1$ represents $CH_2$ and $A^2$ represents a single bond, one of $A^3$ and $A^4$ is $NR^1$ and the other is $CH_2$ or CO when $A^1$ represents a single bond and $A^2$ represents $CH_2$, and one of $A^2$ and $A^3$ is $NR^1$ and the other is $CH_2$ or CO when $A^1$ represents a single bond and $A^4$ represents $CH_2$,

where $R^1$ represents a hydrogen atom, an optionally substituted $C_{1-6}$ alkyl group, an optionally substituted heteroaryl group, an optionally substituted $C_{3-8}$ cycloalkyl $C_{1-3}$ alkyl group, an optionally substituted aryl $C_{1-3}$ alkyl group, an optionally substituted heteroaryl $C_{1-3}$ alkyl group, an optionally substituted three- to seven-membered ring heterocycloalkyl $C_{1-3}$ alkyl group, a group represented by the formula: $-(CH_2)_m-C(=O)-L$, or a group represented by the formula: $-S(=O)_2-R^{13}$,

m is 0, 1, 2 or 3, L is $R^{11}$ when m is 0, and L is $R^{12}$ when m is 1, 2 or 3,

$R^{11}$ is a hydrogen atom, an optionally substituted $C_{1-6}$ alkyl group, $OR^{51}$, a group represented by the formula: $-C(=O)-OR^{52}$, or a group represented by the formula: $-N(R^{53a})-R^{53b}$,

$R^{51}$ is an optionally substituted aryl $C_{1-3}$ alkyl group,

$R^{52}$ is a hydrogen atom or an optionally substituted $C_{1-6}$ alkyl group,

$R^{53a}$ and $R^{53b}$ are each independently a hydrogen atom or an optionally substituted $C_{1-6}$ alkyl group, or $R^{53a}$ and $R^{53b}$ are linked together to form a three- to seven-membered ring heterocycloalkyl group optionally containing at least one atom or group selected from the group consisting of an oxygen atom, a sulfur atom and NR81,

$R^{81}$ is a hydrogen atom or an optionally substituted $C_{1-6}$ alkyl group,

$R^{12}$ is an optionally substituted aryl group, $OR^{54}$, or a group represented by the formula: $-N(R^{55a})-R^{55b}$,

$R^{54}$ is a hydrogen atom, an optionally substituted $C_{1-6}$ alkyl group, an optionally substituted aryl $C_{1-3}$ alkyl group, or an optionally substituted heteroaryl $C_{1-3}$ alkyl group,

$R^{55a}$ and $R^{55b}$ are each independently a hydrogen atom, an optionally substituted $C_{1-6}$ alkyl group, an optionally substituted aryl $C_{1-3}$ alkyl group, an optionally substituted heteroaryl $C_{1-3}$ alkyl group, or a group represented by the formula: $-(C=O)-R^{82}$, or $R^{55a}$ and $R^{55b}$ are linked together to form a three- to seven-membered ring heterocycloalkyl group optionally containing at least one atom or group selected from the group consisting of an oxygen atom, a sulfur atom and $NR^{83}$, or form an optionally substituted 6,8-dihydro-5H-imidazolo[1,2-a]pyrazin-7-yl group,

$R^{82}$ is a hydrogen atom, an optionally substituted $C_{1-6}$ alkyl group, or an optionally substituted aryl $C_{1-3}$ alkyl group,

$R^{83}$ is a hydrogen atom or an optionally substituted $C_{1-6}$ alkyl group, and

$R^{13}$ is an optionally substituted $C_{1-6}$ alkyl group;

the structure formed by $E^1$, $E^2$, $E^3$ and $E^4$ is a group represented by the formula: $-E^1-E^2-E^3-E^4-$ (where the bonds between $E^1$, $E^2$, $E^3$ and $E^4$ are single bonds or double bonds) in which $E^1$ is N or $CR^2$, $E^2$ is N or

$CR^3$, $E^3$ is N or $CR^4$ and $E^4$ is N or $CR^5$, a group represented by the formula: $-E^2-E^3=E^4-$ in which $E^2$ is O or S and each of $E^3$ and $E^4$ is CH with $E^1$ representing a single bond, or a group represented by the formula: $-E^2=E^3-E^4-$ in which each of $E^2$ and $E^3$ is CH and $E^4$ is O or S with $E^1$ representing a single bond,
where $R^2$, $R^3$, $R^4$ and $R^5$ are each independently a hydrogen atom, a fluorine atom, a chlorine atom, a bromine atom, an iodine atom, a cyano group, an optionally substituted $C_{1-6}$ alkyl group, an optionally substituted aryl group, an optionally substituted heteroaryl group, an optionally substituted three- to seven-membered ring heterocycloalkyl group, or a group represented by the formula: $-Q-(CH_2)_n-R^{14}$,
n is 0, 1, 2 or 3,
Q is a group represented by the formula: -CH=CH-, O, CO, a group represented by the formula: -C(=O)-O-, a group represented by the formula: $-C(=O)-N(R^{56})-$, $NR^{56}$, a group represented by the formula: $-N(R^{56})-C(=O)-$, or a group represented by the formula: $-N(R^{56})-C(=O)-O-$,
$R^{56}$ is a hydrogen atom, an optionally substituted $C_{1-3}$ alkyl group, or a group represented by the formula: $-C(=O)-R^{84}$,
$R^{84}$ is a hydrogen atom, an optionally substituted $C_{1-6}$ alkyl group, or an optionally substituted aryl group, an optionally substituted $C_{1-6}$ alkyloxy group, or an optionally substituted aryloxy group, and
$R^{14}$ is a hydrogen atom, an optionally substituted $C_{1-6}$ alkyl group, an optionally substituted aryl group, an optionally substituted heteroaryl group, an optionally substituted $C_{3-8}$ cycloalkyl group, or an optionally substituted three- to seven-membered ring heterocycloalkyl group; and
the structure formed by $G^1$, $G^2$, $G^3$ and $G^4$ is a group represented by the formula: $-G^1-G^2-G^3-G^4-$ (where the bonds between $G^1$, $G^2$, $G^3$ and $G^4$ are single bonds or double bonds) which is represented by the formula: $-CH=CH-CH=CR^6-$ (with the exception of cases where $A^1$ and $A^2$ each represent a single bond, with $A^3$ being O and $A^4$ being CO), the formula: -CH=CH-CH=N- (with the exception of cases where $A^1$ and $A^2$ each represent a single bond, with $A^3$ being O and $A^4$ being CO), the formula: $-CH_2-CH_2-CH_2-CH_2-$, the formula: $-CO-CH_2-CH_2-N(R^7)-$, the formula: $-CH_2-CF_2-CH_2-CH_2-$, the formula: $-CH_2-O-CH_2-CH_2-$, the formula: $-CH_2-S-CH_2-CH_2-$, the formula: $-CH_2-CH_2-N(R^7)-CH_2-$, the formula: $-CH_2-CH_2-CH_2-O-$, the formula: $-CH_2-CH_2-CH_2-N(R^7)-$ or the formula: $-O-CH_2-CH_2-N(R^7)-$, or a group represented by the formula: $-G^2-G^3-G^4-$ (where the bonds between $G^2$, $G^3$ and $G^4$ are single bonds or double bonds) which is represented by the formula: $-CH=CH-N(R^7)-$, the formula: $-CH_2-CH_2-N(R^7)-$, the formula: $-N=CH-N(R^7)-$ or the formula: $-N(R^7)-CH=N-$, with $G^1$ representing a single bond,
where $R^6$ is a hydrogen atom, a fluorine atom, a chlorine atom, a bromine atom, an iodine atom, an optionally substituted $C_{1-6}$ alkyl group, or an optionally substituted $C_{1-6}$ alkyloxy group,
$R^7$ is a hydrogen atom, an optionally substituted $C_{1-6}$ alkyl group, an optionally substituted $C_{3-8}$ cycloalkyl group, an optionally substituted $C_{3-8}$ cycloalkyl $C_{1-3}$ alkyl group, an optionally substituted three- to seven-membered ring heterocycloalkyl group, an optionally substituted three- to seven-membered ring heterocycloalkyl $C_{1-3}$ alkyl group, a group represented by the formula: $-C(=O)-R^{15}$, or a group represented by the formula $-(CH_2)_p-C(=O)-OR^{16}$,
p is 0, 1, 2 or 3,
$R^{15}$ is a hydrogen atom, an optionally substituted $C_{1-6}$ alkyl group or $OR^{57}$,
$R^{57}$ is an optionally substituted $C_{1-6}$ alkyl group, or an optionally substituted aryl $C_{1-3}$ alkyl group, and
$R^{16}$ is a hydrogen atom or an optionally substituted $C_{1-6}$ alkyl group;

a compound of the formula (11):

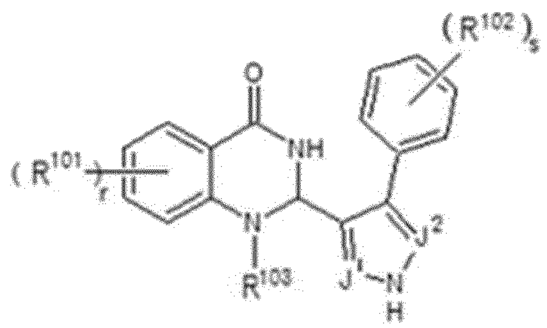

(11)

wherein $J_1$ and $J_2$ each represent CH or N, with the proviso that both $J_1$ and $J_2$ do not represent CH;
r represents 0 to 4;

each $R^{101}$ is the same or different when r is 2 or more, and represents a halogen atom, a $C_{1-6}$ alkyl group optionally substituted with a halogen atom, $OR^{111}$, or a group represented by the formula: $-N(R^{112a})-R^{112b}$, where $R^{111}$, $R^{112a}$ and $R^{112b}$ are each independently a hydrogen atom or a $C_{1-6}$ alkyl group, and
s represents 0 to 5;
each $R^{102}$ is the same or different when s is 2 or more, and represents a halogen atom, a $C_{1-6}$ alkyl group, $OR^{113}$, a group represented by the formula: $-N(R^{114a})-R^{114b}$, a group represented by the formula: $-NH-C(=O)-R^{115}$, a group represented by the formula: $-C(=O)-R^{116}$, an optionally substituted aryl group, an optionally substituted heteroaryl group, a nitro group, or a cyano group,
where $R^{113}$ is a hydrogen atom, an optionally substituted alkyl group, an optionally substituted aryl group, an optionally substituted arylalkyl group, or an optionally substituted heteroaryl group,
$R^{114a}$ and $R^{114b}$ are each independently a hydrogen atom or a $C_{1-6}$ alkyl group,
$R^{115}$ is an optionally substituted alkyl group, an optionally substituted aryl group, an optionally substituted heteroaryl group, or a group represented by the formula: $-NH-R^{121}$,
$R^{116}$ is an optionally substituted alkyl group, an optionally substituted aryl group, an optionally substituted heteroaryl group, $OR^{122}$, or a group represented by the formula: $-N(R^{123a})-R^{123b}$,
$R^{121}$ is an optionally substituted alkyl group, an optionally substituted aryl group, or an optionally substituted heteroaryl group,
$R^{122}$ is a hydrogen atom, an optionally substituted alkyl group, an optionally substituted aryl group, or an optionally substituted heteroaryl group, and
$R^{123a}$ and $R^{123b}$ are each independently a hydrogen atom, an optionally substituted alkyl group, an optionally substituted aryl group, or an optionally substituted heteroaryl group, or $R^{123a}$ and $R^{123b}$ are linked together to form a cyclic amine;
$R^{103}$ represents a hydrogen atom, a $C_{1-6}$ alkyl group, a $C_{3-6}$ cycloalkyl group, or a $C_{3-6}$ cycloalkyl $C_{1-6}$ alkyl group; and
$R^{101}$ and $R^{103}$ are optionally linked together to form a five- to seven-membered ring hetero ring when $R^{101}$ is present at the 8-position; and
a pharmacologically acceptable salt thereof.

**7.** The anticancer agent according to any one of claims 1 to 6, wherein the microtubule inhibitory compound is at least one compound selected from the group consisting of paclitaxel, vinblastine, vincristine, vindesine, vinorelbine, docetaxel, cabazitaxel, eribulin and a pharmacologically acceptable salt thereof.

**8.** The anticancer agent according to any one of claims 1 to 7, wherein the cancer is colorectal cancer.

**9.** A kit for preventing and/or treating a cancer, comprising a tankyrase inhibitor containing a tankyrase inhibitory compound as an active ingredient and a microtubule inhibitor containing a microtubule inhibitory compound as an active ingredient.

**10.** An anticancer agent for preventing and/or treating a cancer, comprising a combination of: a tankyrase inhibitor containing at least one compound selected from the group consisting of a compound of the formula (1) defined in claim 6, a compound of the formula (11) defined in claim 6, and a pharmacologically acceptable salt thereof, as an active ingredient; and at least one agent selected from the group consisting of an alkylating agent, an antimetabolite, a plant alkaloid, a topoisomerase inhibitor, an anticancer antibiotic substance and a platinating agent.

[FIG. 1A]

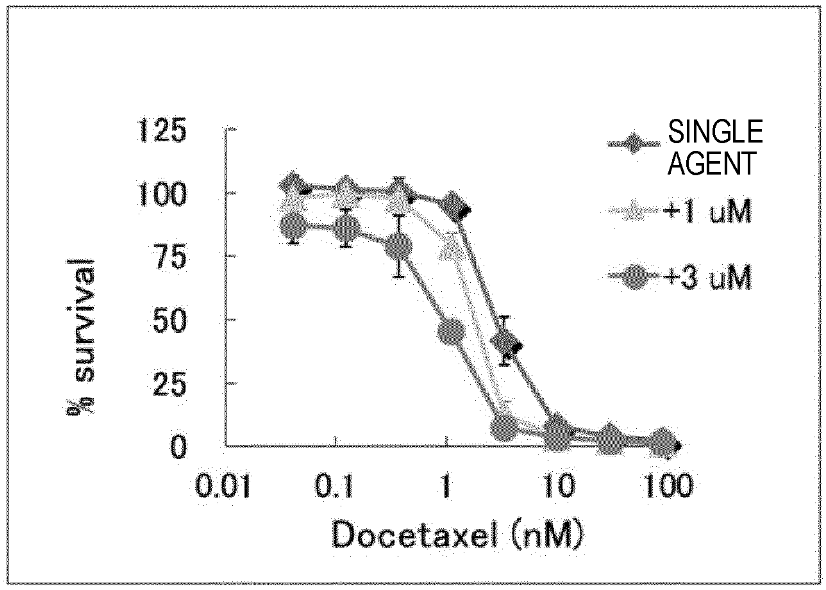
125
100
75
50
25
0
% survival
0.01
0.1
1
10
100
Docetaxel (nM)
SINGLE AGENT
+1 uM
+3 uM

[FIG. 1B]

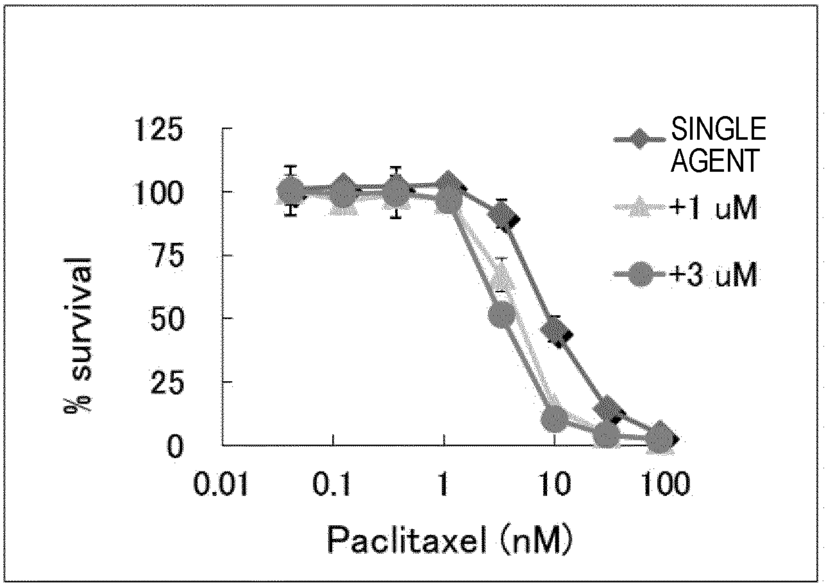
125
100
75
50
25
0
% survival
0.01
0.1
1
10
100
Paclitaxel (nM)
SINGLE AGENT
+1 uM
+3 uM

[FIG. 1C]

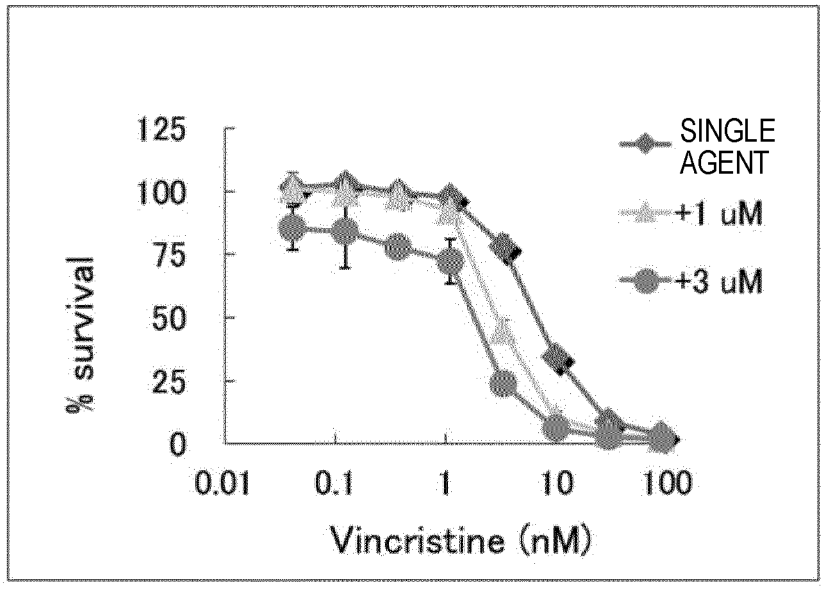
125
100
75
50
25
0
% survival
0.01
0.1
1
10
100
Vincristine (nM)
SINGLE AGENT
+1 uM
+3 uM

[FIG. 1D]

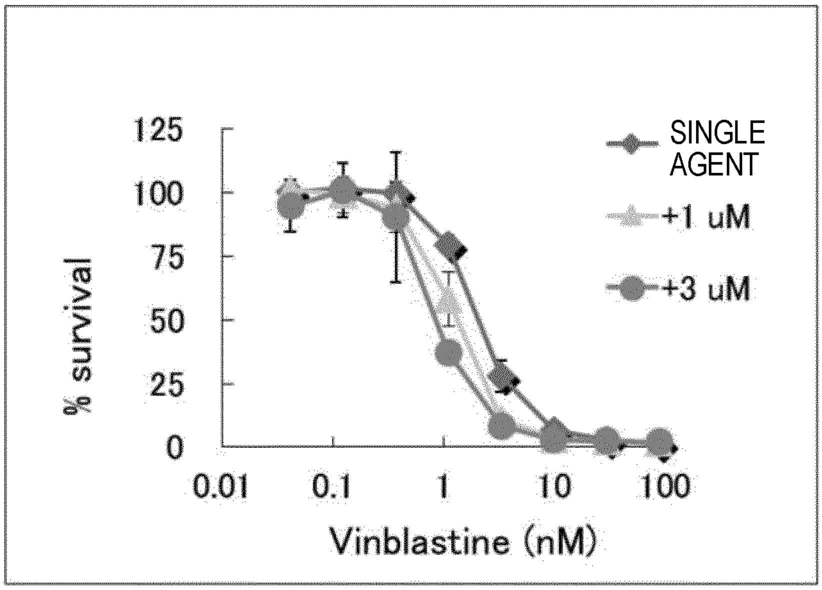
125
100
75
50
25
0
% survival
0.01
0.1
1
10
100
Vinblastine (nM)
SINGLE AGENT
+1 uM
+3 uM

[FIG. 2A]

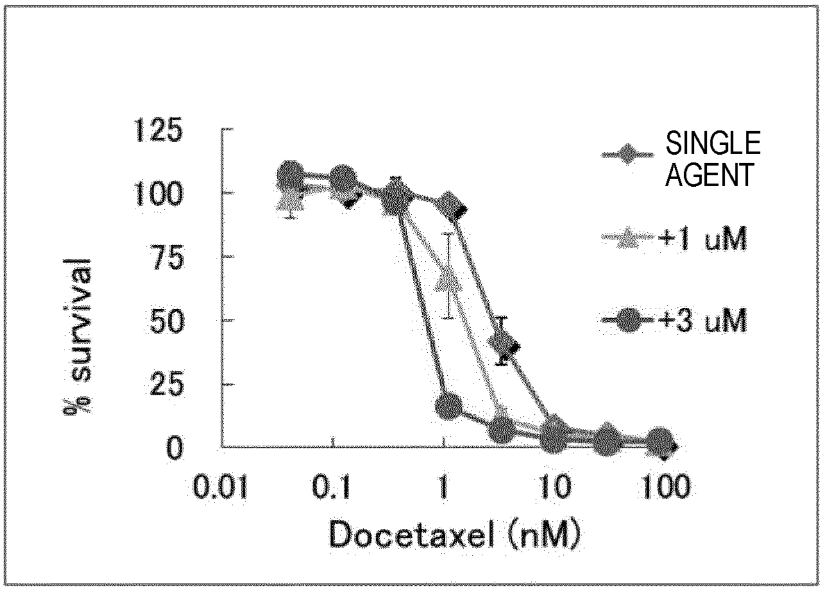
125
100
75
50
25
0
% survival
0.01
0.1
1
10
100
Docetaxel (nM)
SINGLE AGENT
+1 uM
+3 uM

[FIG. 2B]

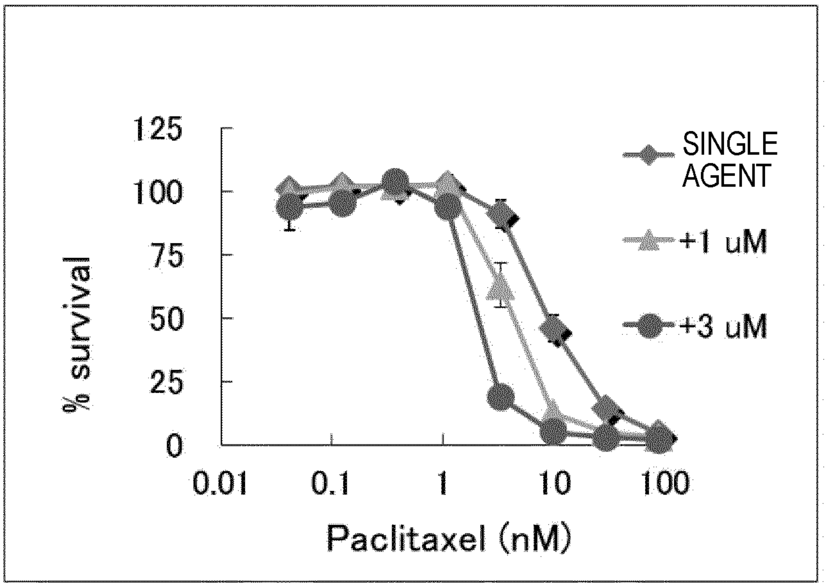
125
100
75
50
25
0
% survival
0.01
0.1
1
10
100
Paclitaxel (nM)
SINGLE AGENT
+1 uM
+3 uM

[FIG. 2C]

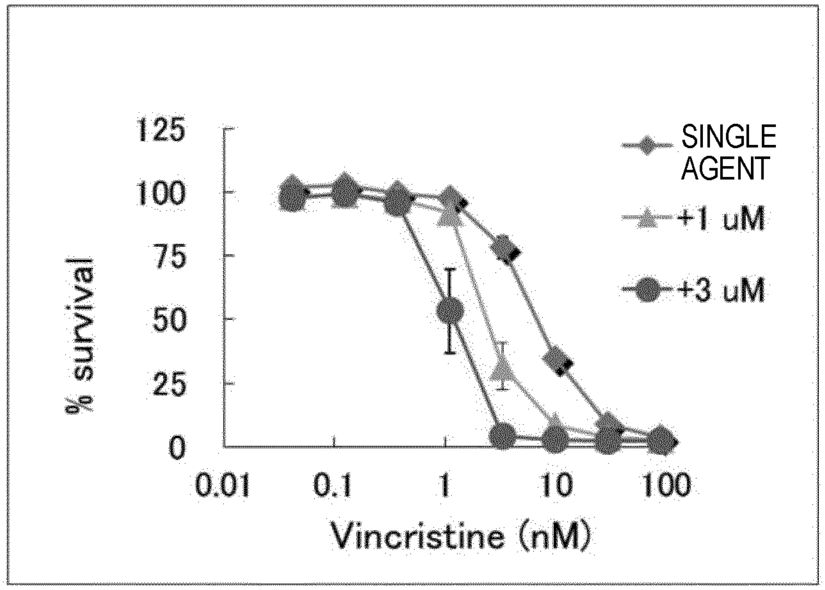
125
100
75
50
25
0
% survival
0.01
0.1
1
10
100
Vincristine (nM)
SINGLE AGENT
+1 uM
+3 uM

[FIG. 2D]

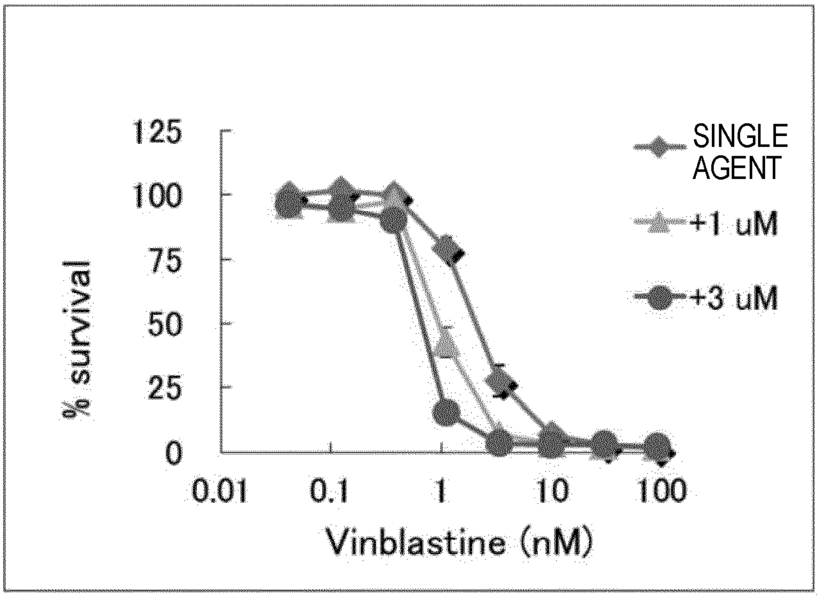
125
100
75
50
25
0
% survival
0.01
0.1
1
10
100
Vinblastine (nM)
SINGLE AGENT
+1 uM
+3 uM

[FIG. 3A]

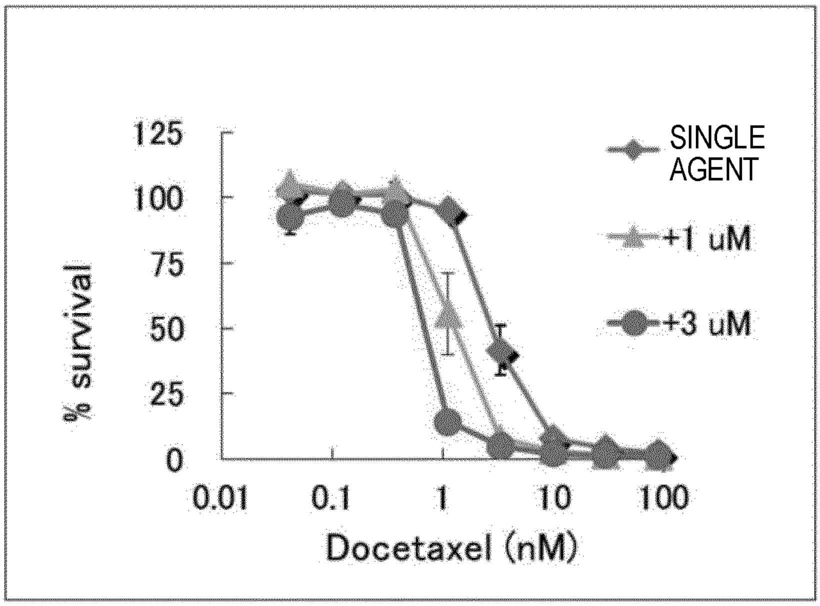
125
100
75
50
25
0
% survival
0.01
0.1
1
10
100
Docetaxel (nM)
SINGLE AGENT
+1 uM
+3 uM

[FIG. 3B]

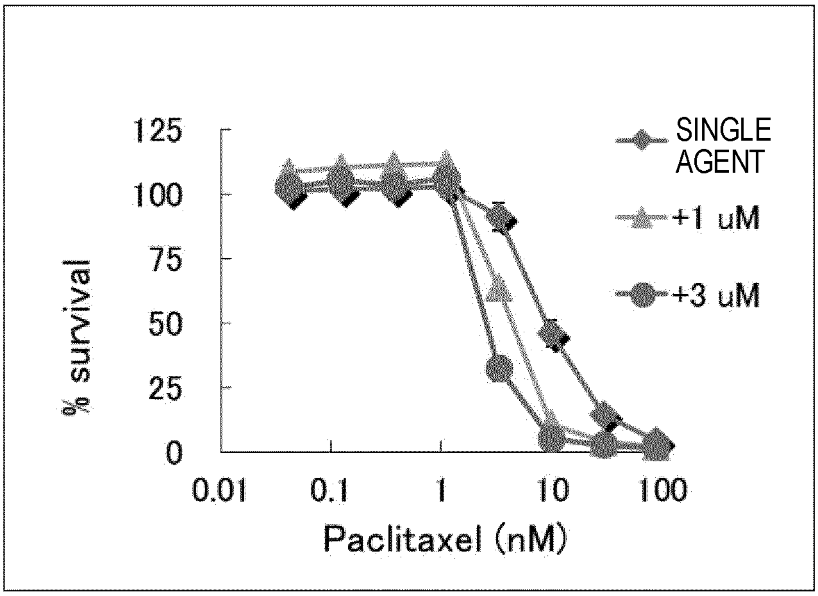
125
100
75
50
25
0
% survival
0.01
0.1
1
10
100
Paclitaxel (nM)
SINGLE AGENT
+1 uM
+3 uM

[FIG. 3C]

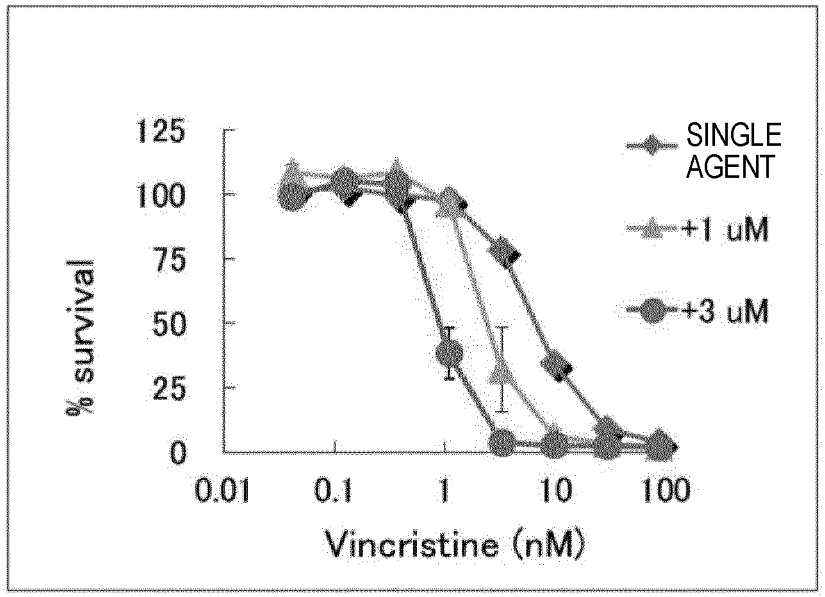
125
100
75
50
25
0
% survival
0.01
0.1
1
10
100
Vincristine (nM)
SINGLE AGENT
+1 uM
+3 uM

[FIG. 3D]

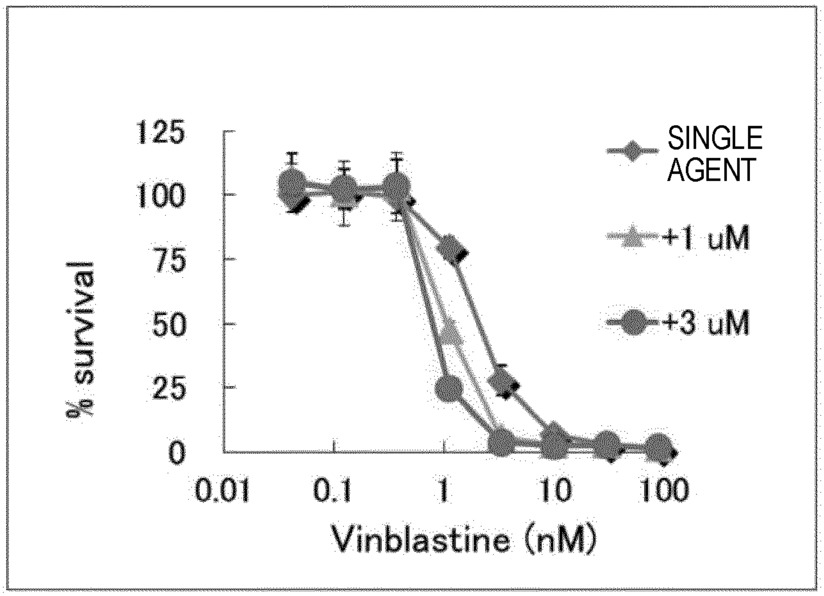
125
100
75
50
25
0
% survival
0.01
0.1
1
10
100
Vinblastine (nM)
SINGLE AGENT
+1 uM
+3 uM

[FIG. 4A]

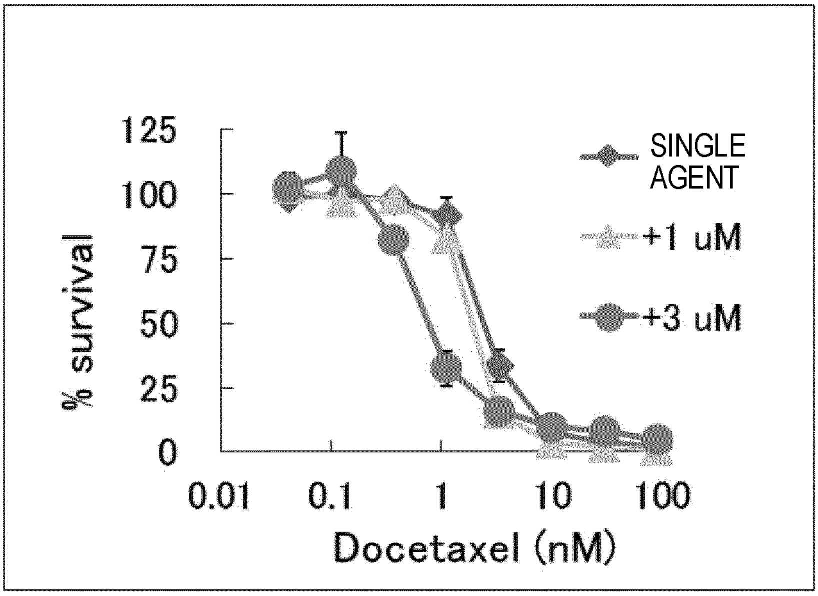
125
100
75
50
25
0
% survival
0.01
0.1
1
10
100
Docetaxel (nM)
SINGLE AGENT
+1 uM
+3 uM

[FIG. 4B]

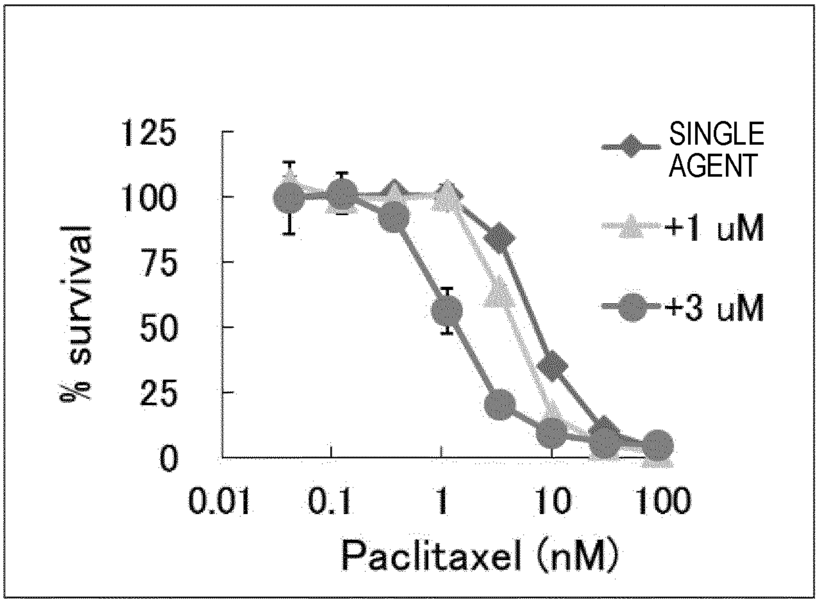
125
100
75
50
25
0
% survival
0.01
0.1
1
10
100
Paclitaxel (nM)
SINGLE AGENT
+1 uM
+3 uM

[FIG. 4C]

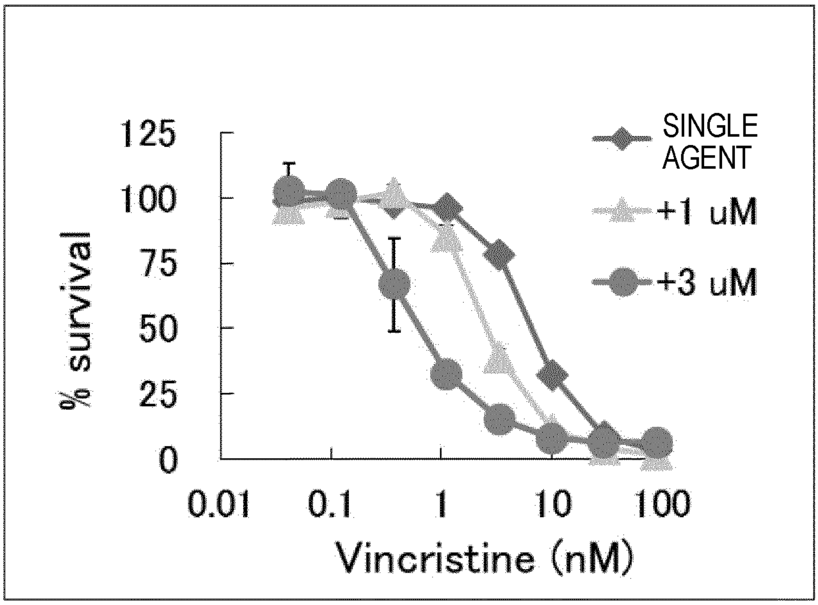
125
100
75
50
25
0
% survival
0.01
0.1
1
10
100
Vincristine (nM)
SINGLE AGENT
+1 uM
+3 uM

[FIG. 4D]

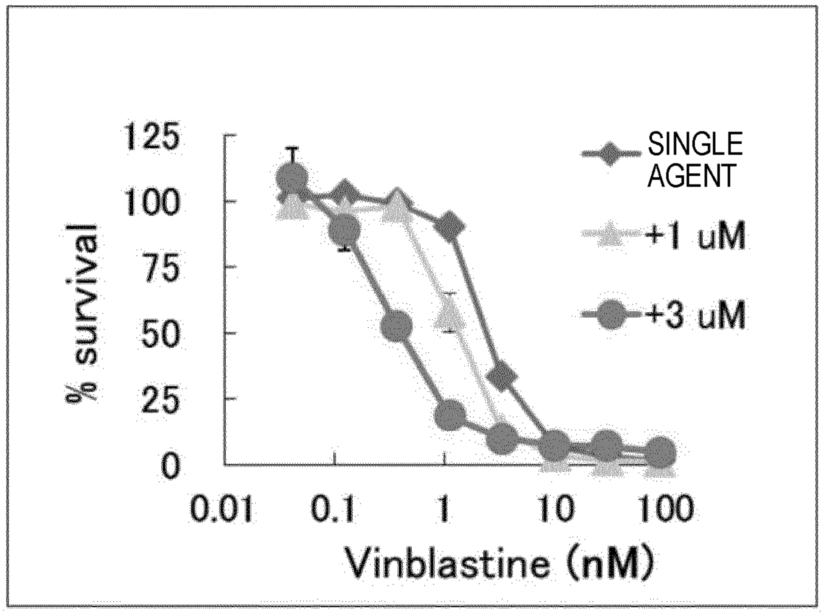
125
100
75
50
25
0
% survival
0.01
0.1
1
10
100
Vinblastine (nM)
SINGLE AGENT
+1 uM
+3 uM

[FIG. 5A]

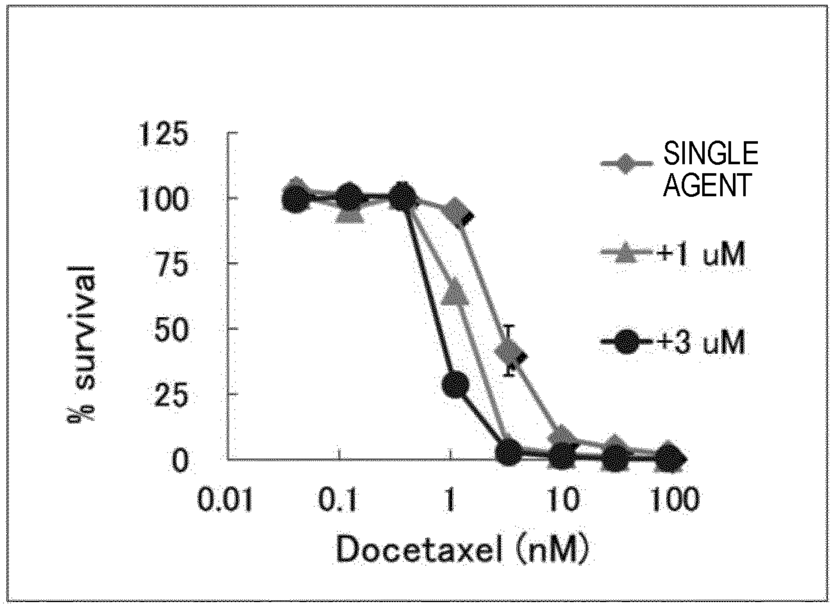
125
100
75
50
25
0
% survival
0.01
0.1
1
10
100
Docetaxel (nM)
SINGLE AGENT
+1 uM
+3 uM

[FIG. 5B]

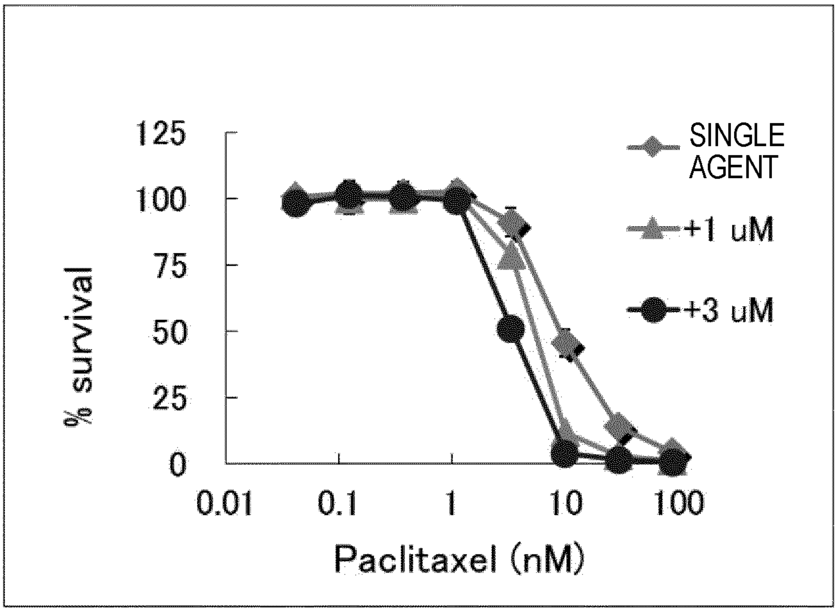
125
100
75
50
25
0
% survival
0.01
0.1
1
10
100
Paclitaxel (nM)
SINGLE AGENT
+1 uM
+3 uM

[FIG. 5C]

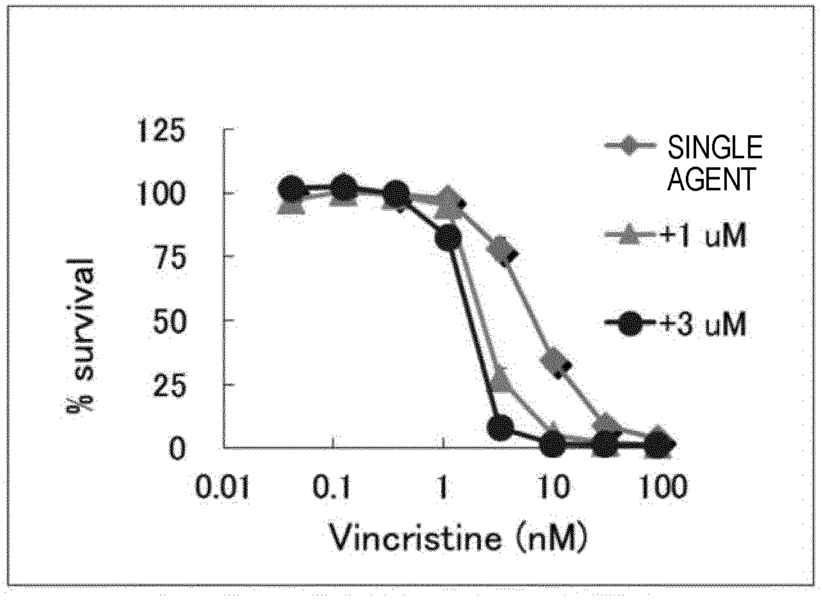
125
100
75
50
25
0
% survival
0.01
0.1
1
10
100
Vincristine (nM)
SINGLE AGENT
+1 uM
+3 uM

[FIG. 5D]

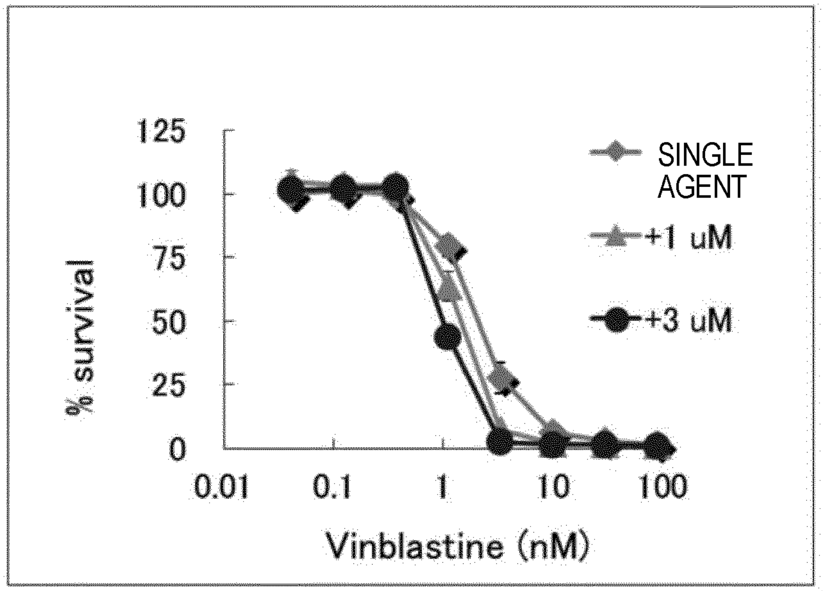
125
100
75
50
25
0
% survival
0.01
0.1
1
10
100
Vinblastine (nM)
SINGLE AGENT
+1 uM
+3 uM

[FIG. 6A]

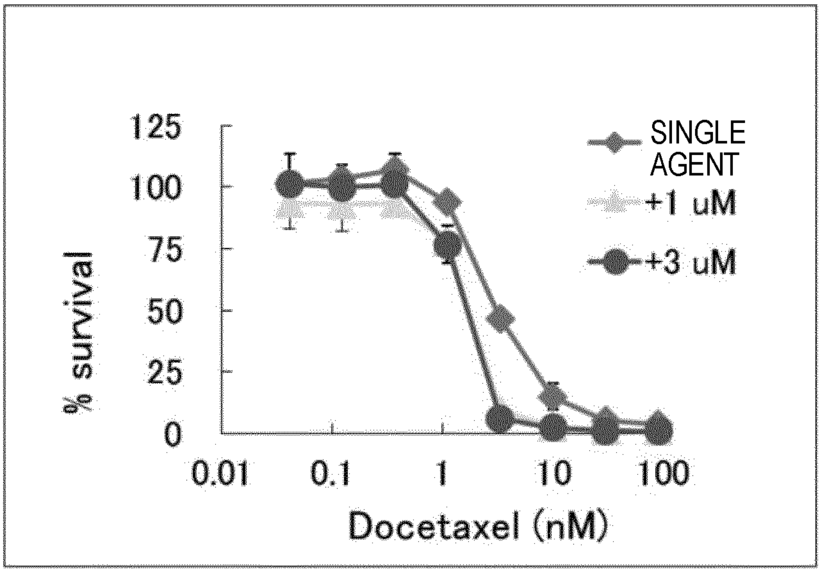
125
100
75
50
25
0
% survival
0.01
0.1
1
10
100
Docetaxel (nM)
SINGLE AGENT
+1 uM
+3 uM

[FIG. 6B]

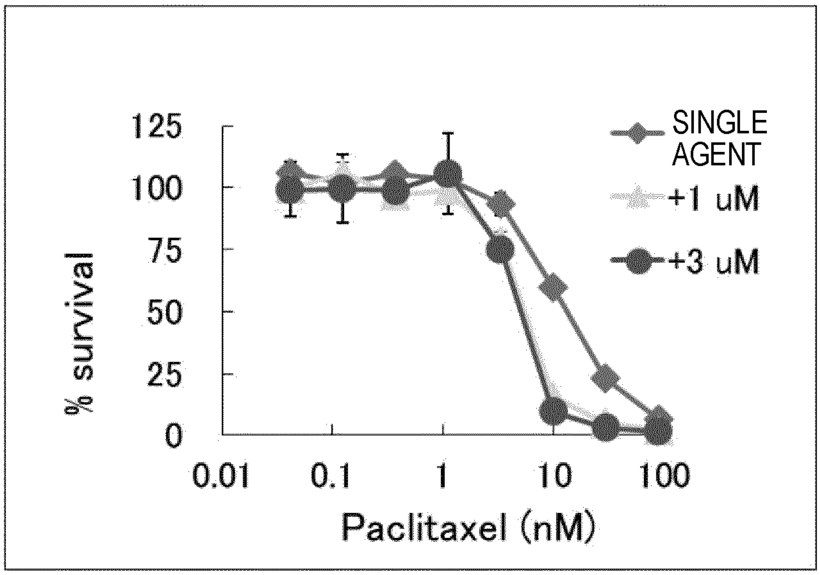
125
100
75
50
25
0
% survival
0.01
0.1
1
10
100
Paclitaxel (nM)
SINGLE AGENT
+1 uM
+3 uM

[FIG. 6C]

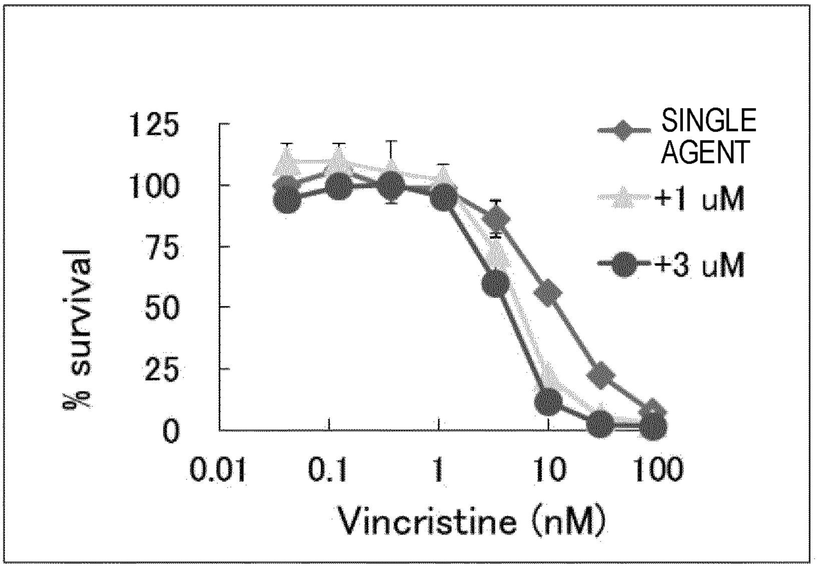
125
100
75
50
25
0
% survival
0.01
0.1
1
10
100
Vincristine (nM)
SINGLE AGENT
+1 uM
+3 uM

[FIG. 6D]

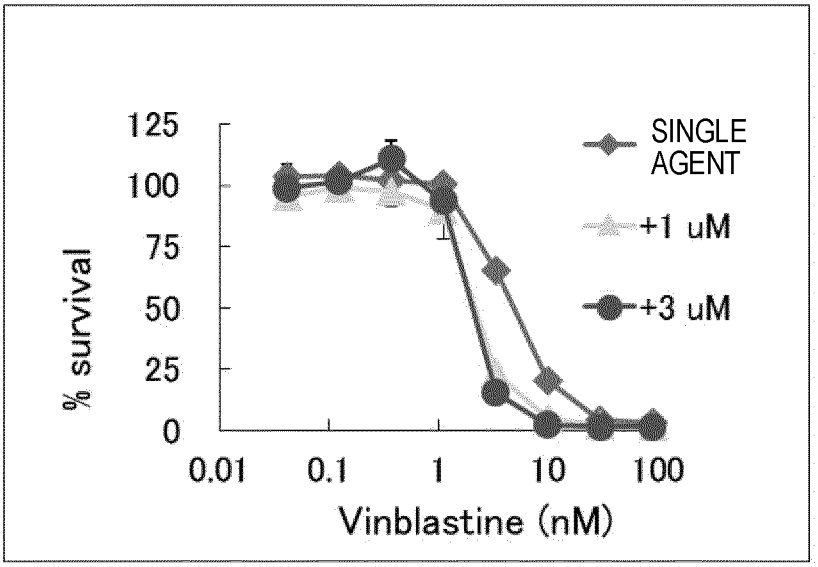
125
100
75
50
25
0
% survival
0.01
0.1
1
10
100
Vinblastine (nM)
SINGLE AGENT
+1 uM
+3 uM

[FIG. 7A]

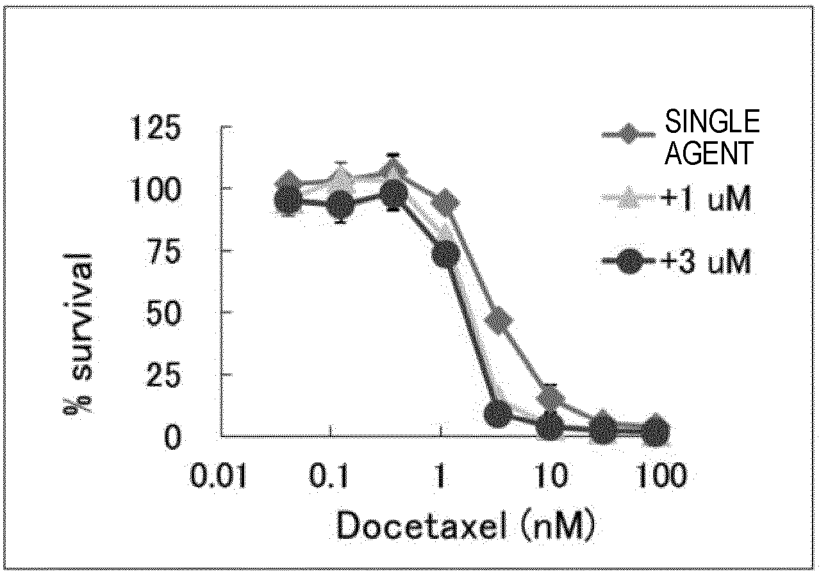
125
100
75
50
25
0
% survival
0.01
0.1
1
10
100
Docetaxel (nM)
SINGLE AGENT
+1 uM
+3 uM

[FIG. 7B]

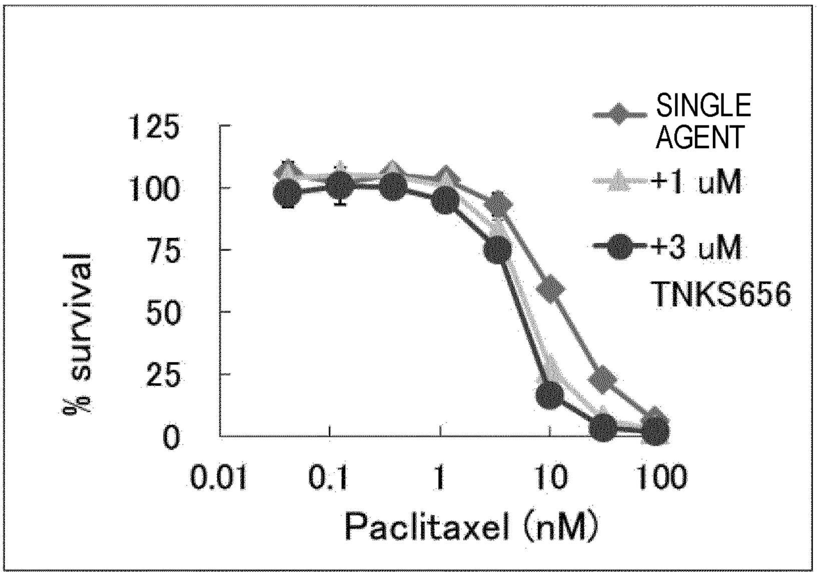
125
100
75
50
25
0
% survival
0.01
0.1
1
10
100
Paclitaxel (nM)
SINGLE AGENT
+1 uM
+3 uM
TNKS656

[FIG. 7C]

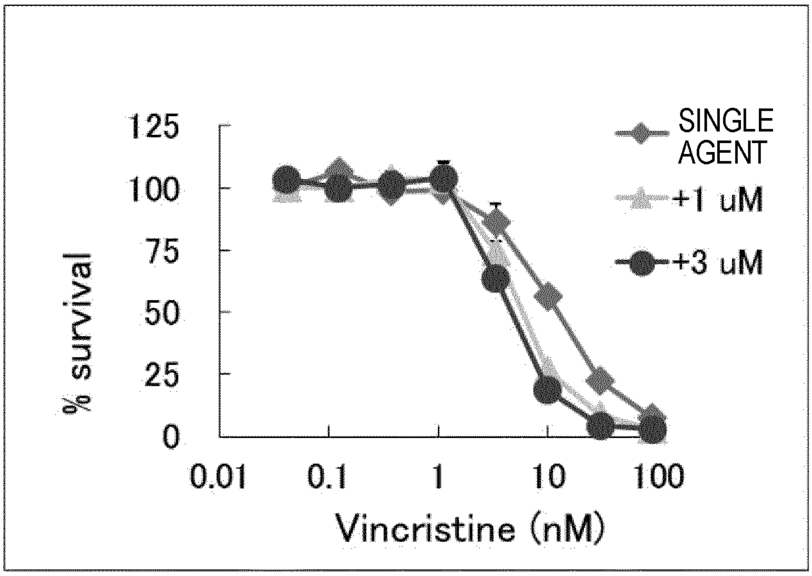
125
100
75
50
25
0
% survival
0.01
0.1
1
10
100
Vincristine (nM)
SINGLE AGENT
+1 uM
+3 uM

[FIG. 7D]

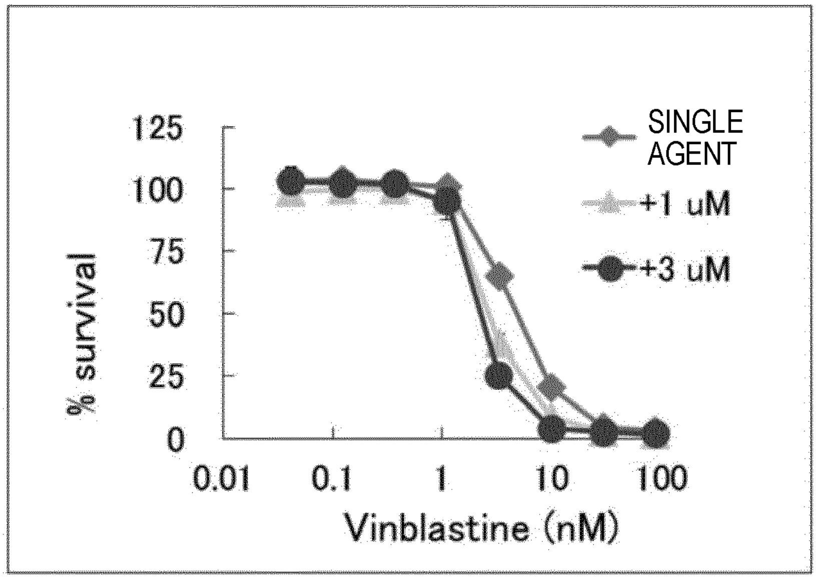
125
100
75
50
25
0
% survival
0.01
0.1
1
10
100
Vinblastine (nM)
SINGLE AGENT
+1 uM
+3 uM

[FIG. 8A]

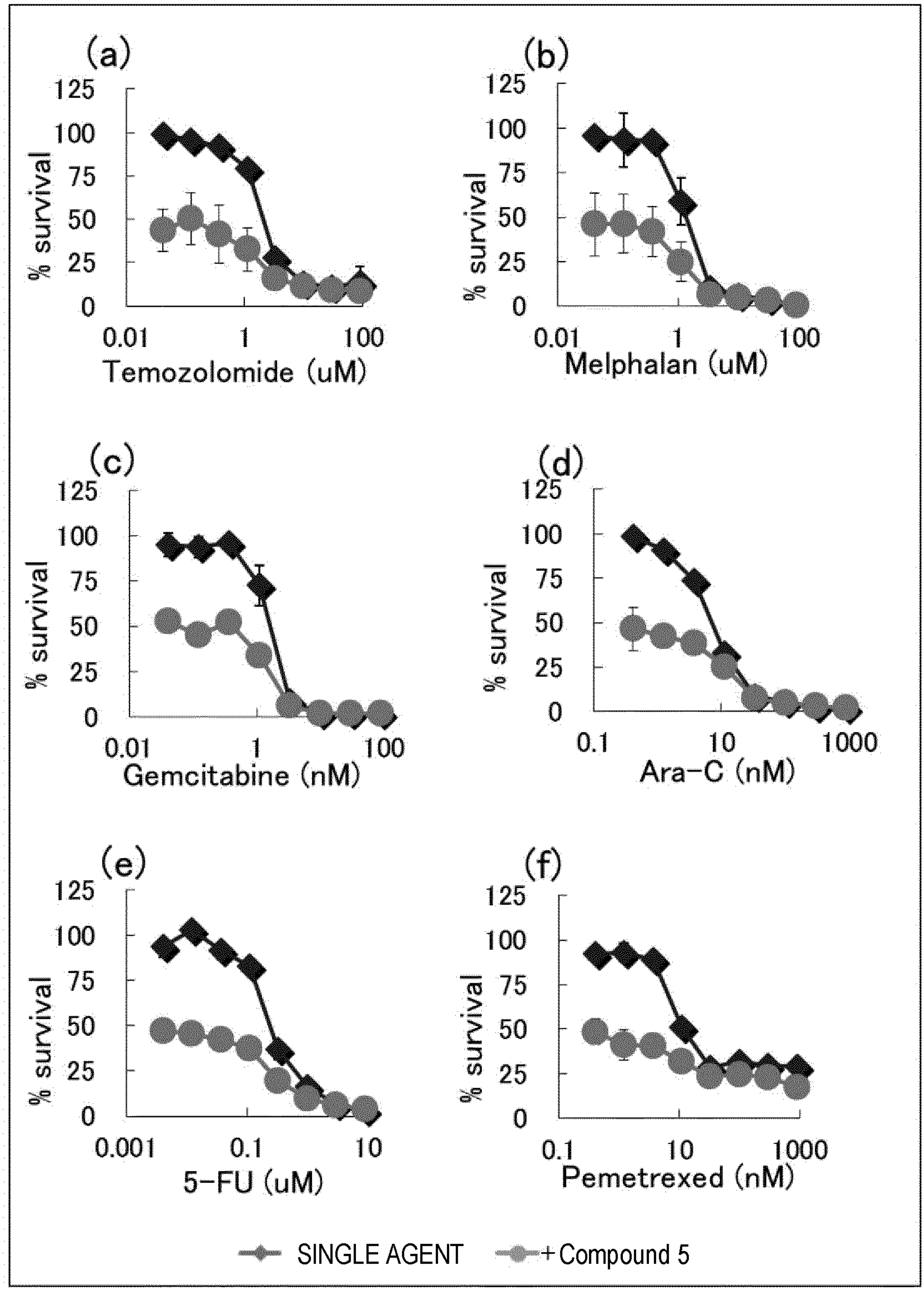
(a)
% survival
Temozolomide (uM)
(b)
% survival
Melphalan (uM)
(c)
% survival
Gemcitabine (nM)
(d)
% survival
Ara-C (nM)
(e)
% survival
5-FU (uM)
(f)
% survival
Pemetrexed (nM)
SINGLE AGENT
+Compound 5

[FIG. 8B]

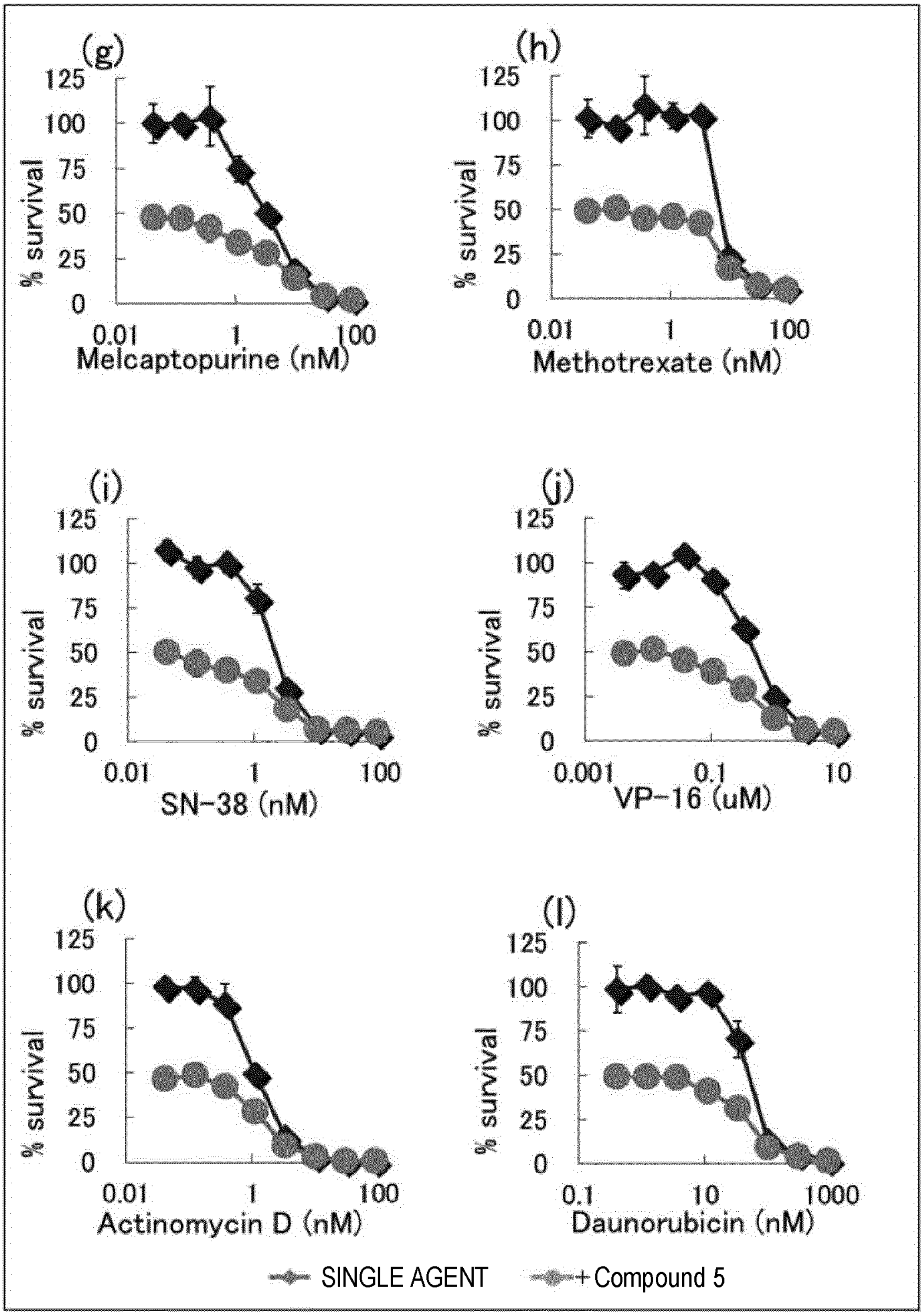
(g)
125
100
75
50
25
0
% survival
0.01
1
100
Melcaptopurine (nM)
(h)
Methotrexate (nM)
(i)
SN-38 (nM)
(j)
0.001
0.1
10
VP-16 (uM)
(k)
Actinomycin D (nM)
(l)
0.1
10
1000
Daunorubicin (nM)
SINGLE AGENT
+ Compound 5

[FIG. 8C]

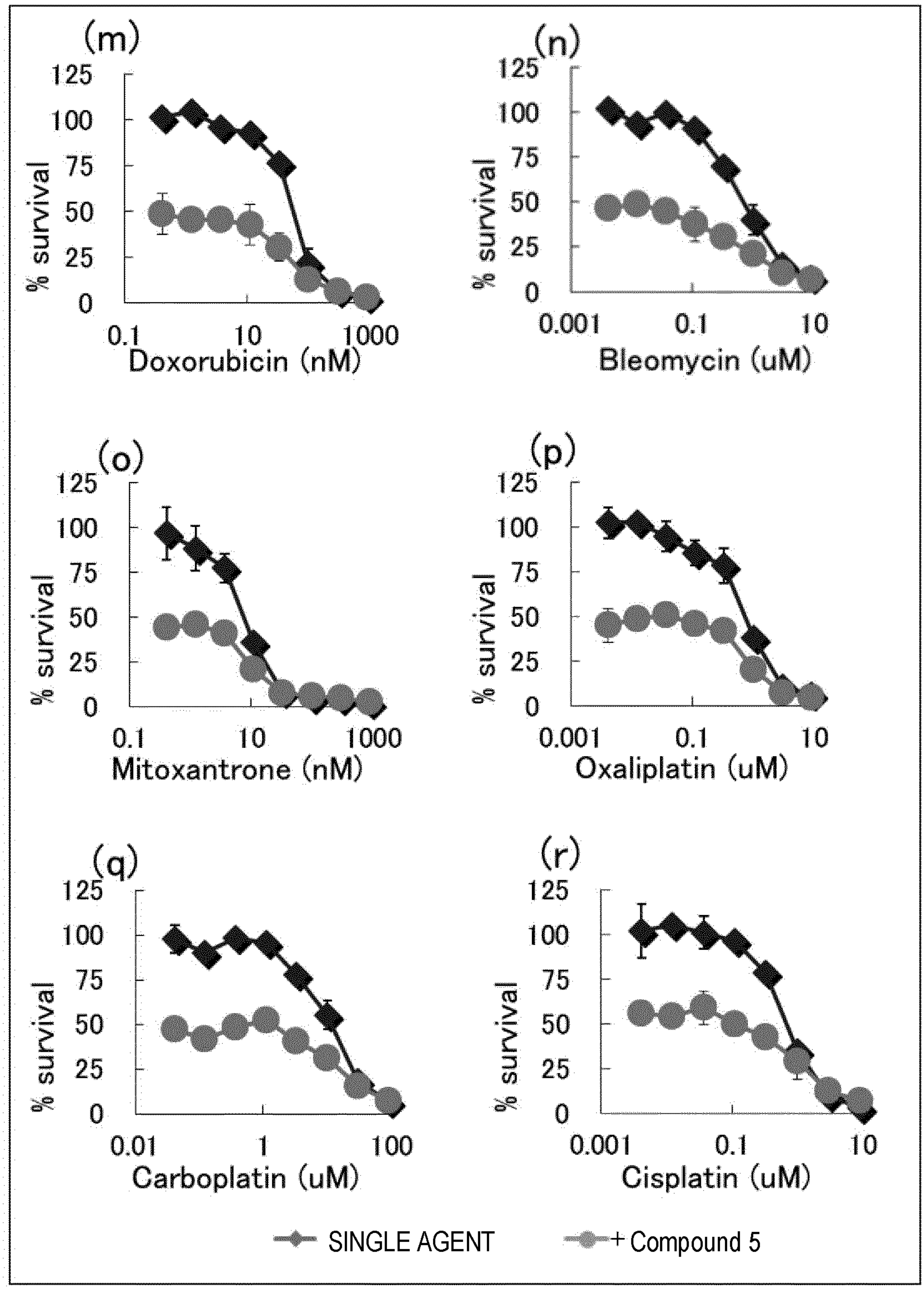
(m)
% survival
125
100
75
50
25
0
0.1
10
1000
Doxorubicin (nM)
(n)
% survival
125
100
75
50
25
0
0.001
0.1
10
Bleomycin (uM)
(o)
% survival
Mitoxantrone (nM)
(p)
% survival
Oxaliplatin (uM)
(q)
% survival
0.01
1
100
Carboplatin (uM)
(r)
% survival
Cisplatin (uM)
SINGLE AGENT
+ Compound 5

[FIG. 9]

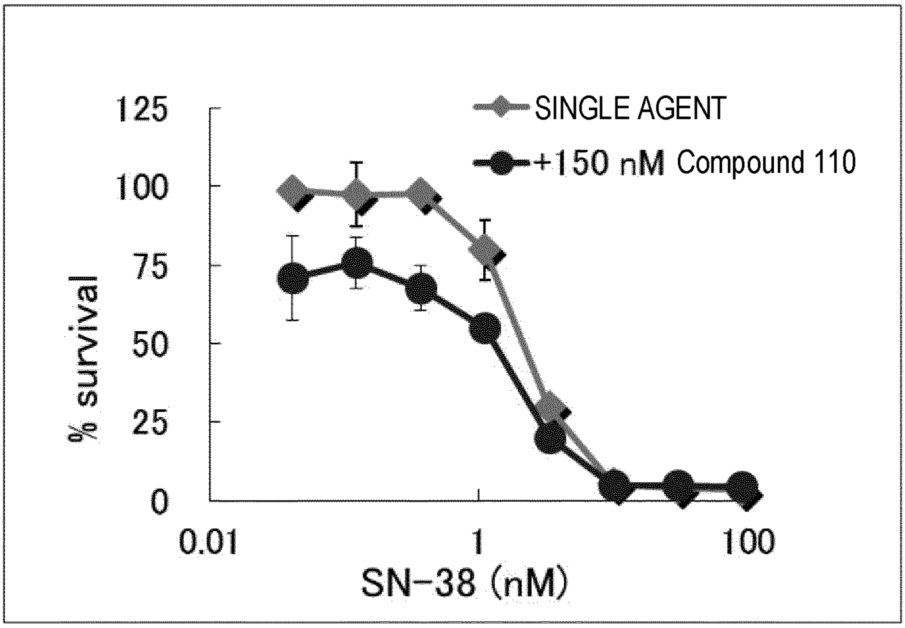
SINGLE AGENT
+150 nM Compound 110
125
100
75
50
25
0
% survival
0.01
1
100
SN-38 (nM)

[FIG. 10]

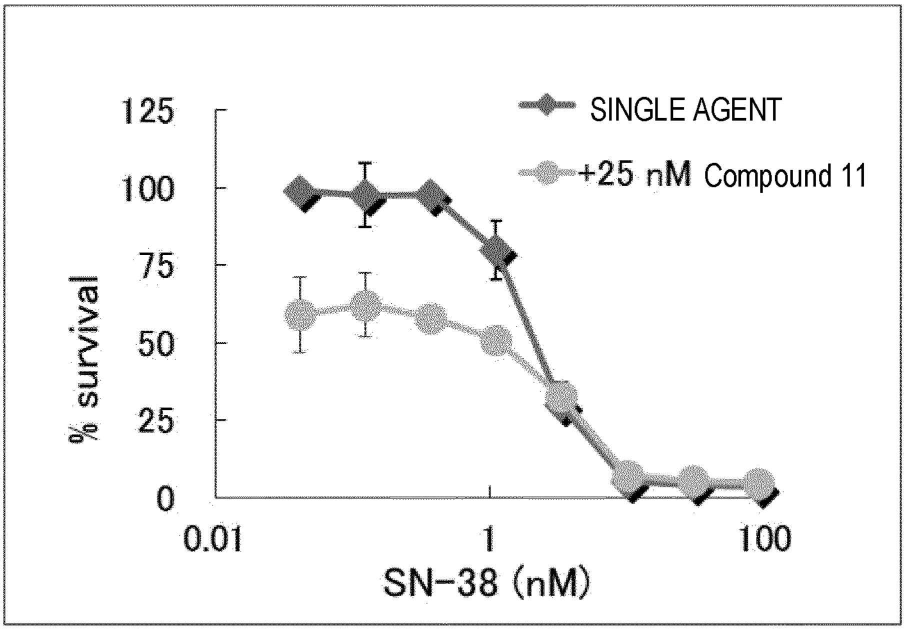
SINGLE AGENT
+25 nM Compound 11
125
100
75
50
25
0
% survival
0.01
1
100
SN-38 (nM)

[FIG. 11]

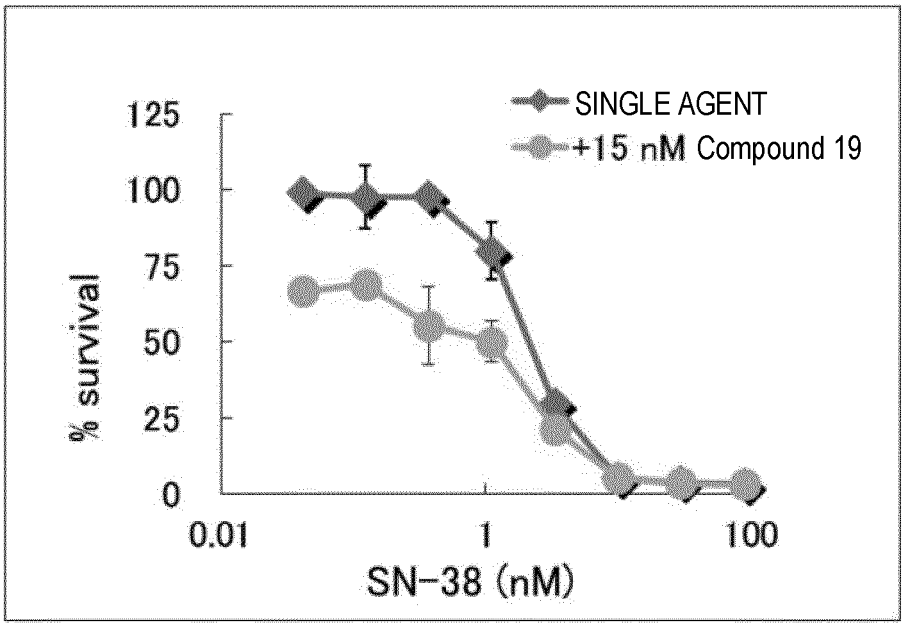
SINGLE AGENT
+15 nM Compound 19
125
100
75
50
25
0
% survival
0.01
1
100
SN-38 (nM)

[FIG. 12]

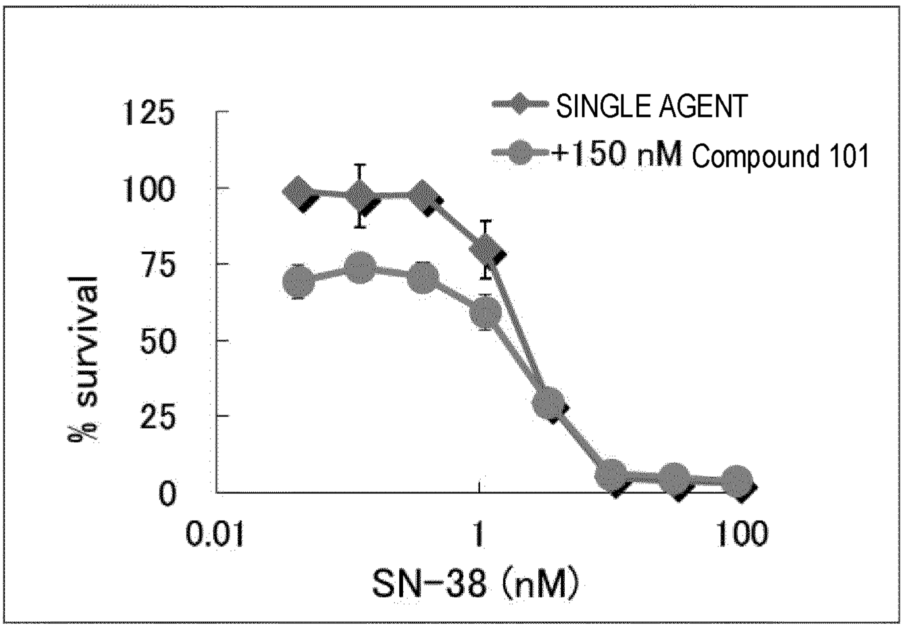
SINGLE AGENT
+150 nM Compound 101
125
100
75
50
25
0
% survival
0.01
1
100
SN-38 (nM)

[FIG. 13]

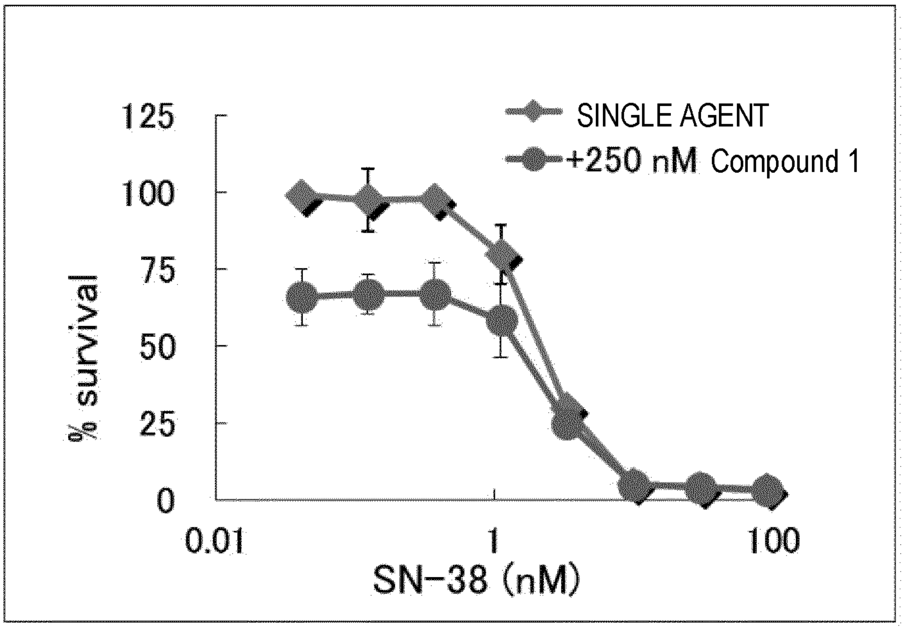
SINGLE AGENT
+250 nM Compound 1
125
100
75
50
25
0
% survival
0.01
1
100
SN-38 (nM)

[FIG. 14]

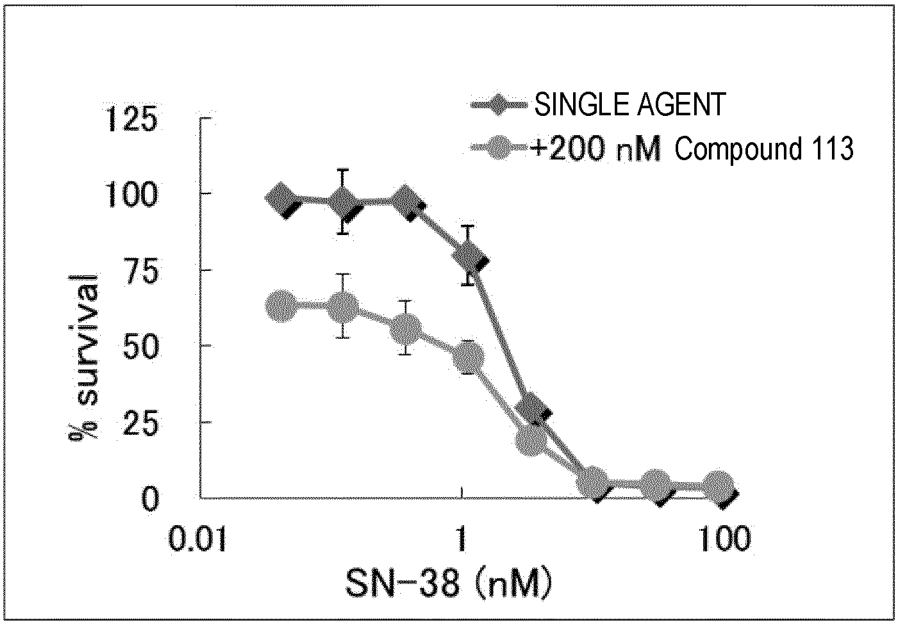
SINGLE AGENT
+200 nM Compound 113
125
100
75
50
25
0
% survival
0.01
1
100
SN-38 (nM)

## INTERNATIONAL SEARCH REPORT

International application No.
PCT/JP2018/047937

A. CLASSIFICATION OF SUBJECT MATTER
Int.Cl. A61K45/06(2006.01)i, A61K31/337(2006.01)i, A61K31/357(2006.01)i, A61K31/475(2006.01)i, A61K31/517(2006.01)i, A61P35/00(2006.01)i, A61P43/00(2006.01)i
According to International Patent Classification (IPC) or to both national classification and IPC

B. FIELDS SEARCHED
Minimum documentation searched (classification system followed by classification symbols)
Int.Cl. A61K45/06, A61K31/337, A61K31/357, A61K31/475, A61K31/517, A61P35/00, A61P43/00

Documentation searched other than minimum documentation to the extent that such documents are included in the fields searched

| | |
|---|---|
| Published examined utility model applications of Japan | 1922-1996 |
| Published unexamined utility model applications of Japan | 1971-2019 |
| Registered utility model specifications of Japan | 1996-2019 |
| Published registered utility model applications of Japan | 1994-2019 |

Electronic data base consulted during the international search (name of data base and, where practicable, search terms used)
CAplus/MEDLINE/EMBASE/BIOSIS/WPIDS (STN)
JSTPlus/JMEDPlus/JST7580 (JDreamIII)

C. DOCUMENTS CONSIDERED TO BE RELEVANT

| Category* | Citation of document, with indication, where appropriate, of the relevant passages | Relevant to claim No. |
|---|---|---|
| X<br>A | JP 2017-526676 A (ONCOMED PHARMACEUTICALS, INC.) 14 September 2017, claims 1, 19-21, paragraph [0262] & US 2017/0247465 A1 & WO 2016/033284 A1, claims 1, 46-50, paragraph [0269] & EP 3185884 A4 & AU 2015308854 A & CA 2959529 A & CN 106714822 A & MX 2017002364 A | 1-5, 7-9<br>6, 10 |
| X<br>A | JP 2015-535831 A (MERCK PATENT GMBH) 17 December 2015, claims 6, 9, paragraphs [0002], [0117], [0128] & US 2015/0239850 A1 & WO 2014/048532 A1, claims 6, 9, pp. 1, 32, 36 & EP 2900643 A1 & CN 104662006 A & KR 10-2015-0063474 A | 1-4, 7-9<br>5, 6, 10 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 13 March 2019 (13.03.2019) | 26 March 2019 (26.03.2019) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

International application No.
PCT/JP2018/047937

C (Continuation). DOCUMENTS CONSIDERED TO BE RELEVANT

| Category* | Citation of document, with indication, where appropriate, of the relevant passages | Relevant to claim No. |
|---|---|---|
| A | HUA, Z. et al., "Development of novel dual binders as potent, selective, and orally bioavailable tankyrase inhibitors", Journal of Medicinal Chemistry, 2013, vol. 56, pp. 10003-10015 | 1-10 |
| P, Y | WO 2018/003962 A1 (RIKEN, INSTITUTE OF PHYSICAL AND CHEMICAL RESEARCH) 04 January 2018, claim 1 (Family: none) | 1-10 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2013117288 A
- WO 2013182580 A
- WO 2012076898 A

### Non-patent literature cited in the description


- **HUANG SM. et al.** *Nature,* 2009, vol. 461, 614-620
- **MICHAEL D. SHULTZ et al.** *Journal of Medicinal Chemistry,* 2013, vol. 56, 6495-6511
- **ANDREW VORONKOV. et al.** *Journal of Medicinal Chemistry,* 2013, vol. 56, 3012-3023
- **ORIOL ARQUES et al.** *Clinical Cancer Research,* 2016, vol. 22 (3), 644-656
- **KEVIN S. QUACKENBUSH et al.** *Oncotarget,* 2016, vol. 7 (19), 28273-28285
- **HANNAH A. SCARBOROUGH et al.** *Clinical Cancer Research,* 2017, vol. 23 (6), 1531-1541
- **HA et al.** *Cell Death Differ.,* 2012, vol. 19, 321-332
- **DYNEK ; SMITH.** *Science,* 2004, vol. 304, 97-100
- **CHANG et al.** *Nat Cell Biol.,* 2005, vol. 7, 1133-1139
- **GREEN WUTS.** PROTECTIVE GROUPS in ORGANIC SYNTHESIS THIRD EDITION. John Wiley & Sons, Inc,
- **GREEN WUTS.** PROTECTIVE GROUPS in ORGANIC SYNTHESIS. John Wiley & Sons, Inc
- *Synthesis,* 1998, vol. 10, 1140
- *Tetrahedron Letters,* 2001, vol. 42, 9117
- *Bioorganic & Medicinal Chemistry Letters,* 2008, 184932